# EUROPÄISCHE PATENTSCHRIFT

(11) **EP 2 054 427 B1**
(45) Veröffentlichungstag und Bekanntmachung des Hinweises auf die Patenterteilung: **14.07.2010**
(21) Anmeldenummer: 07786613.5
(22) Anmeldetag: 08.08.2007
(51) Int. Cl.: C07H 17/08, C07H 13/02, A01N 43/90

(54) **AVERMECTINDERIVATE**
AVERMECTIN DERIVATIVES
DÉRIVÉS D'AVERMECTINE

(30) Priorität: 17.08.2006 DE 102006038632; 16.02.2007 DE 102007007750
(43) Veröffentlichungstag der Anmeldung: 06.05.2009
(73) Patentinhaber: Bayer CropScience AG, 40789 Monheim (DE)
(72) Erfinder: JESCHKE, Peter, 51467 Bergisch Gladbach (DE); KARIG, Gunter, 65719 Hofheim am Taunus (DE); VELTEN, Robert, 40764 Langenfeld (DE); FISCHER, Reiner, Monheim 40789 (DE); MALSAM, Olga, 51503 Rösrath (DE); GÖRGENS, Ulrich, 40882 Ratingen (DE); ARNOLD, Christian, Langenfeld 40764 (DE); SANWALD, Erich, Kiel 24159 (DE); HARDER, Achim, 51109 Köln (DE); TURBERG, Andreas, 42781 Haan (DE)
(86) Internationale Anmeldenummer: PCT/EP2007/006991
(87) Internationale Veröffentlichungsnummer: WO 2008/019784

(56) Entgegenhaltungen:
- EP-A- 0 235 085
- US-A- 4 201 861

## Beschreibung

Die vorliegende Anmeldung betrifft neue Avermectinderivate, Verfahren zu ihrer Herstellung und ihre Verwendung zur Bekämpfung von tierischen Schädlingen, vor allem von Arthropoden, insbesondere Insekten, in Veterinärmedizin, Hygiene, Landwirtschaft, Forsten und Materialschutz sowie Schädlingsbekämpfungsmittel, die Avermectinderivate enthalten. Ferner betrifft die vorliegende Anmeldung die Verwendung neuer Avermectinderivate als Parasitizide gegen Helminthen, Nematoden und Trematoden in Tieren, sowie Endo- und Ectoparasitizide, die Avermectinderivate enthalten.

Bestimmte Avermectin A1a Derivate sind als Insektizide oder Parasitizide bereits bekannt, beispielsweise das 4'-O-(4-amino-1,4-dioxobutyl)-4'-O-de(2,6-dideoxy-3-O-methyl-α-L-arabino-hexopyranosyl)-5-O-demethyl-22,23-dihydro-avermectin Ala (R. A. Dybas, A. S. J. Green, British Crop Protection Conference-Pests and Dis., Proc. (1984), 3, 947-54) als Insektizid, das 4'-O-Acetyl-4'-O-de(2,6-dideoxy-3-O-methyl-a-L-arabino-hexopyranosyl)-5-O-demethyl- und das 4'-O-(4-chloro-benzoyl)-4'-O-de(2,6-dideoxy-3-O-methyl-a-L-arabino-hexopyranosyl)-5-O-demethyl-avermectin Ala (US 4,201,861, JP 54-06197) als Insektizide und Parasitizide und das 4'-O-Acetyl-4'-O-de(2,6-dideoxy-3-0-methyl-a-L-arabino-hexopyranosyl)-5-0-demethyl-25-de(1-methyl-propyl)-22,23-dihydro-25-(1-methyl-ethyl)-avermectin Ala (EP 2 350 85 A1) sowie das 4'-O-acetyl-25-cyclohexyl-4'-O-de(2,6-dideoxy-3-O-methyl-α-L-arabino-hexo-pyranosyl)-5-O-demethyl-25-de(1 -methylpropyl)-22,23-dihydro-avermectin Ala (US 5,981,500 A, WO 94/15944 A1) als Parasitizide, insbesondere Endo- und Ectoparasitizide.

Die Wirkung dieser vorbekannten Verbindungen ist jedoch nicht in jeder Hinsicht völlig zufrieden stellend. Insbesondere bei niedrigen Aufwandmengen und -konzentrationen besteht in allen Anwendungsgebieten das Bedürfnis nach effektiv wirkenden Verbindungen. Aufgabe der vorliegenden Erfindung ist deshalb, Verbindungen zur Verfügung zu stellen, die eine überzeugende Wirkung gegen tierische Schädlinge und Parasiten zeigen.

Es wurden nun neue Avermectinderivate der Formel (I) gefunden, 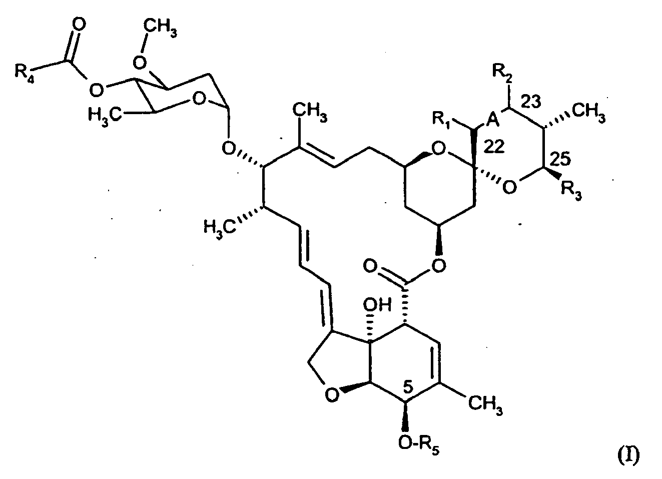 in welcher
die Gruppierung -C₂₂R¹-A-C₂₃R²- für -HC=CH-, -H₂C-CH(OH)- oder -H₂C-CH₂- steht,
- R³: für sec-Butyl, iso-Propyl oder Cyclohexyl,
- R⁵: für Wasserstoff, Methyl oder C₁₋₄-Alkylcarbonyl, und
- R⁴: für gegebenenfalls substituiertes C₂₋₆-Alkyl, C₂₋₆-Alkenyl, C₂₋₆-Alkinyl, C₁₋₆-Alkoxy-C₁₋₄-alkyl, C₁₋₄-Alkoxy-C₁₋₄-alkoxy-C₁₋₄-alkyl, Cycloalkyl, Cycloalkenyl, Cycloalkyl-C₁₋₄-alkyl, Aryl, Aryl-C₁₋₄alkyl, Hetaryl, Hetaryl-C₁₋₄-alkyl, Heterocyclyl oder Heterocyclyl-C₁₋₄-alkyl,
oder für einen Rest steht, ausgewählt aus den Resten (G⁷) bis (G¹⁴) 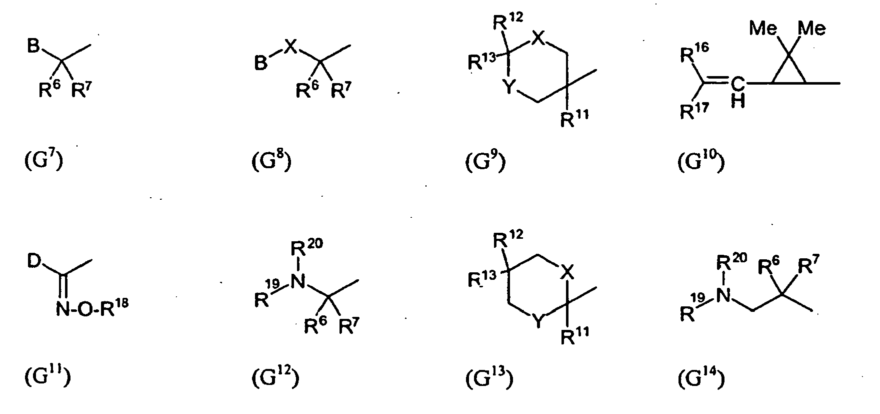 worin
B für gegebenenfalls mit R⁸, R⁹ und R¹⁰ substituiertes Aryl, Cycloalkyl, Heterocyclyl, Hetaryl, oder NR¹⁹R²⁰ steht,
D für gegebenenfalls mit R⁸, R⁹ und R¹⁰ substituiertes Aryl, Cycloalkyl, Heterocyclyl, Hetaryl oder NR¹⁹R²⁰ steht, wobei R⁴ jedoch nicht für 4-Chlorphenyl steht (bekannt aus US 4,201,861 und JP 54-06197),
R⁶ für Wasserstoff, Halogen, insbesondere Fluor, Cyan, gegebenenfalls substituiertes Alkyl, Alkenyl, Alkinyl, Cycloalkyl oder Heterocyclyl steht,
R⁷ für Wasserstoff, Halogen, insbesondere Fluor, Cyan, gegebenenfalls substituiertes Alkyl, Alkenyl, Alkinyl steht oder
R⁶ und R⁷ oder gemeinsam mit dem Atom, an das sie gebunden sind, für einen 3-, 4-, 5-, 6- 7-gliedrigen Ring stehen, der gegebenenfalls substituiert ist und/oder gegebenenfalls durch Sauerstoff, Schwefel, Stickstoff, Sulfinyl oder Sulfonyl unterbrochen ist, oder
R⁶ und R⁷ gemeinsam mit dem Atom, an das sie gebunden sind, für eine gegebenenfalls substituierte *exo*-Methylenbindung stehen,
R⁸ für Wasserstoff, gegebenenfalls substituiertes C₁₋₄-Alkyl, C₃₋₆-Cycloalkyl, C₁₋₄- Halogenalkyl, C₁₋₄-Alkoxy, C₁₋₄-Alkoxy-C₁₋₄-alkoxy, C₁₋₄-Halogenalkoxy, C₁₋₄-Alkylthio, C₁₋₄- Halogenalkylthio, C₁₋₄-Alkylsulfinyl, C₁₋₄-Halogenalkylsulfinyl, C₁₋₄-Alkylsulfonyl, C₁₋₄- Halogenalkylsulfonyl, Hetaryl, wie Pyridyl oder Thienyl, Halogen, Nitro, Cyan, Amino, C₁₋₄- Alkylamino, Di-(C₁₋₄-alkyl)-amino, steht, oder für einen Rest ausgewählt unter. CO-OH, COO⁽⁻⁾, COO-C₁₋₆-alkyl, NH-CHO, NH-CO-C₁₋₄-alkoxy, N(C ₁₋₄-alkyl)-CO-C₁₋₄-alkoxy, P(O)(OH)₂, P(O)O⁽⁻⁾₂, CO-NH₂, CS-NH₂, C(=NH)-NH₂, C(=N-OH)-NH₂, CO-NH-C₁₋₄-alkyl, CO-N-(C₁₋₄- alkyl)₂, CO-NH-C₁₋₄-alkoxy, CO-NH-CO-C₁₋₄-alkyl, CO-NH-CO-C₁₋₄-halogenalkyl, CO-NH- CO-C₃₋₇cycloalkyl, CO-NH-CO-C₁₋₄-alkoxy, CO-NH-CO-(aryl-C₁₋₂-alkyloxy), SO₂-OH, SO₂-O⁽⁻⁾, SO₂-NH₂, SO₂-NH-C₁₋₄-alkyl, SO₂-N-(C₁₋₄-alkyl)₂, CO-NH-SO₂-NH-C₁₋₄-alkyl, CO-NH- SO₂-N[di(C₁₋₄-alkyl), CO-O-C₁₋₆-alkyl, steht,
R⁹ für Wasserstoff oder gegebenenfalls substituiertes C₁₋₄₋Alkyl, C₁₋₄-Halogenalkyl, C₁₋₄- Alkoxy, C₁₋₄-Halogenalkoxy, C₁₋₄-Alkylthio, C₁₋₄-Alkylsulfinyl, C₁₋₄-Alkylsulfonyl, Halogen, Nitro, Cyan, Formyl, C₁₋₄-Alkylcarbonyl, Amino, C₁₋₄-Alkylamino, Di-(C₁₋₄-alkyl)-amino, gegebenenfalls substituiertes Aryl, gegebenenfalls substituiertes Hetaryl oder gegebenenfalls substituiertes Heterocyclyl steht,
R¹⁰ für Wasserstoff oder gegebenenfalls substituiertes C₁₋₄-Alkyl, C₂₋₄-Alkenyl, C₂₋₄- Alkinyl, C₁₋₄-Halogenalkyl, C₁₋₄-Alkylcarbonyl, C₁₋₄-Alkoxycarbonyl steht,
R¹¹ für Wasserstoff, Cyan oder gegebenenfalls substituiertes C₁₋₆-Alkyl steht,
R¹² und R¹³ unabhängig voneinander für Wasserstoff, Hydroxy, oder gegebenenfalls substituiertes C₁₋₄-Alkyl, C₂₋₄-Alkenyl, C₂₋₄-Alkinyl, C₁₋₆-Alkylcarbonyl, C₁₋₆- Alkylamino, Di-(C₁₋₆-alkyl)-amino, C₁₋₆-Alkylamino-C₁₋₄-alkyl, Di-(C₁₋₆-alkyl)-amino- C₁₋₄-alkyl, C₁₋₆-Alkoxy-C₁₋₄-alkyl, Amino-C₁₋₄-alkyl, C₃₋₆-Cycloalkyl, Aryl, Aryl-C₁₋₄- alkyl, Hetaryl-C₁₋₄-alkyl, Heterocyclyl, Heterocyclyl-C₁₋₄-alkyl oder Hetaryl steht, oder
R¹² und R¹³ gemeinsam mit dem Atom, an das sie gebunden sind, für einen gegebenenfalls substituierten 3-, 4-, 5-, 6- oder 7-gliedrigen Ring stehen, der gegebenenfalls durch Sauerstoff, Schwefel, Stickstoff, Sulfinyl oder Sulfonyl unterbrochen sein kann, oder
R¹² und R¹³ gemeinsam mit dem Atom, an das sie gebunden sind, für 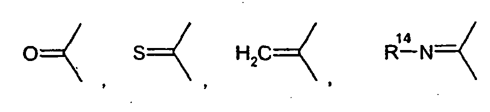 stehen,
X für Sauerstoff, Schwefel, Sulfinyl, Sulfonyl oder N-R¹⁴ steht, worin R¹⁴ für Wasserstoff oder gegebenenfalls substituiertes C₁₋₄-Alkyl steht,
Y für Sauerstoff, Schwefel, Sulfinyl, Sulfonyl oder N-R¹⁵ steht, worin R¹⁵ für Wasserstoff, gegebenenfalls substituiertes C₁₋₄-Alkyl steht,
R¹⁶ für Methyl, Chlor, Brom oder Trifluormethyl steht,
R¹⁷ für Methyl, Chlor oder Brom steht,
R¹⁸ für Wasserstoff oder gegebenenfalls substituiertes C₁₋₄-Alkyl, Aryl-C₁₋₄-alkyl oder Hetaryl-C₁₋₄-alkyl steht,
R¹⁹ und R²⁰ unabhängig voneinander für Wasserstoff oder gegebenenfalls substituiertes C₁₋₄-Alkyl, C₁₋₄-Halogenalkyl, C₁₋₄-Alkoxycarbonyl, C₁₋₄-Alkylcarbonyl, C₁₋₆- Alkoxy-C₁₋₄-alkyl, C₁₋₆-Alkylamino-C₁₋₄-alkyl, Di-(C₁₋₆-alkyl)-amino-C₁₋₄-alkyl, C₁₋₄- Alkoxycarbamoyl, C₂₋₆-Alkenyl, C₂₋₆-Alkinyl, Aryl-C₁₋₄-alkyl, Hetaryl-C₁₋₄-alkyl steht, oder
R¹⁹ und R²⁰ gemeinsam mit dem Stickstoffatom, an das sie gebunden sind, für eine cyclische Aminogruppe stehen, oder für einen 3-, 4-, 5-, 6- oder 7-gliedrigen Ring stehen, der gegebenenfalls durch Sauerstoff, Schwefel, Stickstoff, Sulfinyl oder Sulfonyl unterbrochen ist und/oder der gegebenenfalls mit mindestens einem, insbesondere 1, 2, 3 oder 4, Rest wie in R⁸, R⁹ und M¹⁰ definiert, substituiert ist.

Weiter wurde das nachfolgende beschriebene Herstellungsverfahren gefunden, durch das man erfindungsgemäße Avermectinderivate der Formel (I) erhält, indem man Verbindungen der Formel (II) 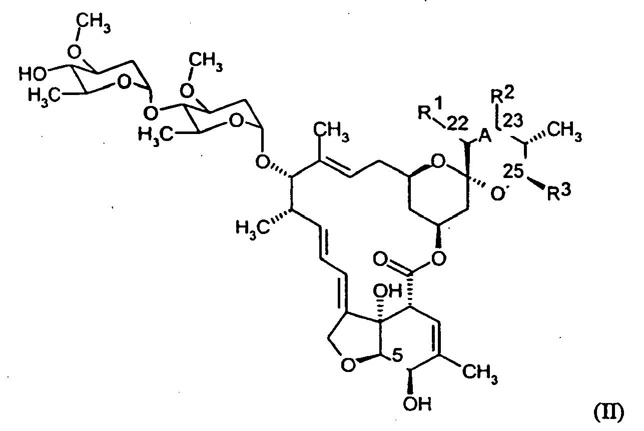 in welcher die Gruppierung -C₂₂R¹-A-C₂₃R²- und R³ die oben genannte Bedeutung haben,
in einem ersten Reaktionsschritt in Gegenwart eines Verdünnungsmittels und in Gegenwart eines sauren Reaktionshilfsmittels zu den entsprechenden Verbindungen der Formel (III) umsetzt 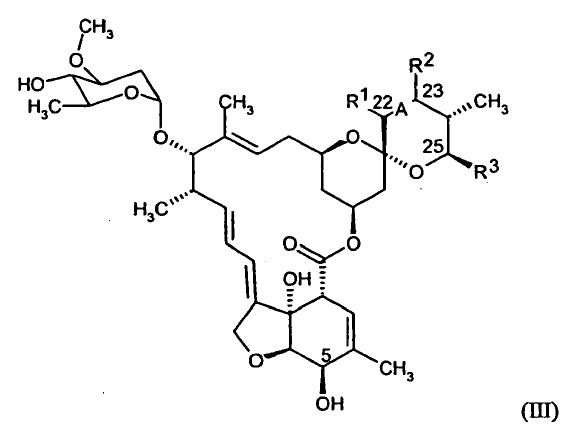 in welcher
die Gruppierung -C₂₂R¹-A-C₂₃R²- und R³ die oben genannte Bedeutung haben,
und diese dann in einem zweiten Reaktionsschritt in Gegenwart eines Verdünnungsmittels und gegebenenfalls in Gegenwart eines basischen Reaktionshilfsmittels mit geeigneten Schutzgruppen in makrocyclische Lactone der Formel (IV) überführt 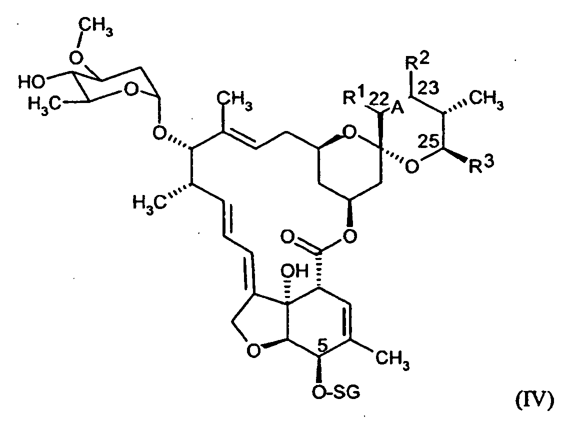 in welcher
die Gruppierung -C₂₂R¹-A-C₂₃R²- und R³ die oben genannte Bedeutung haben, und
- SG: für einen geeigneten Schutzgruppenrest steht,
und diese dann in einem dritten Reaktionsschritt gegebenenfalls in Gegenwart eines Verdünnungsmittels und gegebenenfalls in Gegenwart eines basischen Reaktionshilfsmittels mit Verbindungen der Formel (V)

R⁴-C(=O)-LG (V)

in welcher
- R⁴: die oben genannte Bedeutung hat, und
- LG: für eine gegebenenfalls *in-situ* erzeugte nucleofuge Abgangsgruppe (Leaving Group) steht,
zu Verbindungen der Formel (VI) umsetzt, 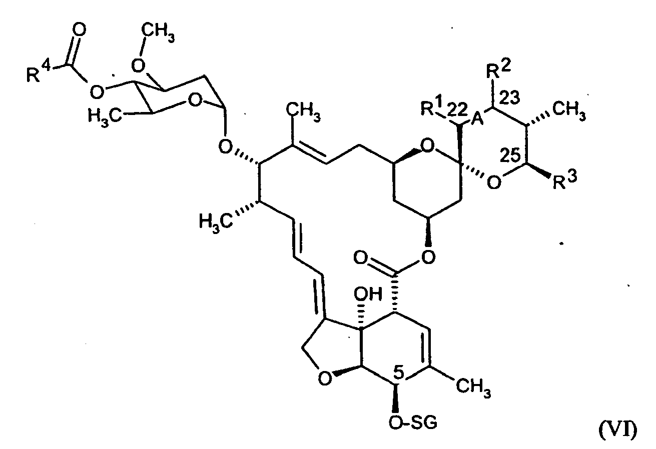 in welcher die Gruppierung -C₂₂R¹-A-C₂₃R²- und R³ die oben genannte Bedeutung haben, und SG für einen geeigneten Schutzgruppenrest steht,
und diese dann in einem vierten Reaktionsschritt unter den Reaktionsbedingungen einer Schutzgruppendeblockierung gegebenenfalls in Gegenwart eines Verdünnungsmittels und gegebenenfalls in Gegenwart eines geeigneten sauren oder basischen Reaktionshilfsmittels umsetzt.

Erfindungsgemäß wird unter Alkyl ein geradkettiges oder verzweigtes Alkyl mit 1 bis 6, insbesondere 1 bis 4, vorzugsweise 2 bis 6 Kohlenstoffatomen, wie beispielsweise Methyl, Ethyl, n-Propyl, Isopropyl, n-Butyl, Isobutyl, sec-Butyl, tert-Butyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,2-Dimethylpropyl, 1,3-Dimethylbutyl, 1,4-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl und 1-Ethylbutyl und 2-Ethylbutyl verstanden. Unter den Alkylen sind besonders Methyl, Ethyl, n-Propyl, Isopropyl, n-Butyl, Isobutyl, sec-Butyl und tert-Butyl bevorzugt.

Erfindungsgemäß wird unter Alkenyl eine geradkettiges oder verzweigtes Alkenyl mit vorzugsweise 2 bis 6, insbesondere 2 bis 4 Kohlenstoffatomen, wie beispielsweise Vinyl, 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-dimethyl-3-butenyl, 1,2-Dimethyl-2-butenyl, 1,2-Dimethyl-3-butenyl, 1,3-Dimethyl-2-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl verstanden. Unter den Alkenylen sind 2-Propenyl, 2-Butenyl oder 1-Methyl-2-propenyl bevorzugt.

Erfindungsgemäß wird unter Alkinyl ein geradkettiges oder verzweigtes Alkinyl mit vorzugsweise 2 bis 6, insbesondere 3 bis 4 Kohlenstoffatomen, wie beispielsweise 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-3-butinyl, 1,2-Dimethyl-3-butinyl, 2,2-Dimethyl-3-butinyl, 1-Ethyl-3-butinyl, 2-Ethyl-3-butinyl und 1-Ethyl-1-methyl-2-propinyl verstanden. Unter den Alkinylen sind besonders Ethinyl, 2-Propinyl oder 2-Butinyl bevorzugt.

Erfindungsgemäß wird unter Cycloalkyl mono-, bi- und tricyclisches Cycloalkyl, vorzugsweise mit 3 bis 10 Kohlenstoffatomen, insbesondere 3, 5 oder 7 Kohlenstoffatomen, wie beispielsweise Cyclopropyl, Cyclobutyl, Cyclopentyl, Cyclohexyl, Cycloheptyl, Cyclooctyl, Bicyclo[2.2.1]heptyl, Bycyclo[2.2.2]octyl und Adamantyl verstanden. Unter den Cycloalkylen sind besonders Cyclopropyl und Cyclobutyl genannt.

Erfindungsgemäß wird unter Halogencycloalkyl mono-, bi- und tricyclisches Halogencycloallcyl, vorzugsweise mit 3 bis 10 Kohlenstoffatomen, insbesondere 3, 5 oder 7 Kohlenstoffatomen, wie beispielsweise 1-Fluor-cyclopropyl, 2-Fluor-cyclopropyl oder 1-Fluor-cyclobutyl verstanden. Erfindungsgemäß enthält ein Halogenalkyl 1 bis 4 Kohlenstoffatome, insbesondere 1 bis 2 Kohlenstoffatome mit vorzugsweise 1 bis 9, insbesondere 1 bis 5 gleichen oder verschiedenen Halogenatomen. Bevorzugt sind Fluor, Chlor oder Brom, insbesondere Fluor oder Chlor. Beispielhaft seien Trifluormethyl, Trichlormethyl, Chlordifluormethyl, Dichlorfluormethyl, Chlormethyl, Brommethyl, 1-Fluorethyl, 2-Fluorethyl, 2,2-Difluorethyl, 2,2,2-Trifluorethyl, 2,2,2-Trichlorethyl, 2-Chlor-2,2₋difluorethyl, Pentafluorethyl und Pentafluor-tert-butyl genannt. Unter den Halogenalkylen sind Difluormethyl, Trifluorethyl oder 2,2-Difluorethyl bevorzugt.

Erfindungsgemäß wird unter Alkoxy ein geradkettiges oder verzweigtes Alkoxy mit vorzugsweise 1 bis 6 Kohlenstoffatomen, insbesondere 1 bis 4 Kohlenstoffatomen, wie beispielsweise Methoxy, Ethoxy, n-Propoxy, Isopropoxy, n-Butoxy, Isobutoxy, sec-Butoxy und tert-Butoxy verstanden.

Erfindungsgemäß wird unter Halogenalkoxy ein geradkettiges oder verzweigtes Halogenalkoxy mit vorzugsweise 1 bis 6 Kohlenstoffatomen, insbesondere 1 bis 4 Kohlenstoffatomen, wie beispielsweise Difluormethoxy, Trifluormethoxy, 2,2-Difluorethoxy, 1,1,2,2-Tetrafluorethoxy, 2,2,2-Trifluorethoxy und 2-Chlor-1,1,2-trifluorethoxy verstanden.

Erfindungsgemäß wird unter Alkoxyalkoxy ein geradkettiges oder verzweigtes Alkoxyalkoxy mit vorzugsweise 2 bis 6 Kohlenstoffatomen, insbesondere 2 bis 4 Kohlenstoffatomen, wie beispielsweise Methoxymethoxy, Methoxyethoxy, Methoxy-n-propoxy und Ethoxyisopropoxy verstanden.

Erfindungsgemäß wird unter Halogenalkoxy ein geradkettiges oder verzweigtes Halogenalkoxy mit vorzugsweise 1 bis 6 Kohlenstoffatomen, insbesondere 1 bis 4 Kohlenstoffatomen, wie beispielsweise. Difluormethoxy, Trifluormethoxy, Trichlormethoxy, Chlordifluormethoxy, 1-Fluorethoxy, 2-Fluorethoxy, 2,2-Difluorethoxy, 1,1,2,2-Tetrafluorethoxy, 2,2,2-Trifluorethoxy und 2-Chlor-1,1,2-trifluorethoxy verstanden.

Erfindungsgemäß wird unter Alkoxyalkoxyallcoxy ein geradkettiges oder verzweigtes Alkoxyalkoxyalkoxy mit vorzugsweise 3 bis 6 Kohlenstoffatomen, insbesondere 3 bis 4 Kohlenstoffatomen wie beispielsweise Methoxymethoxyethoxy, Methoxyethoxyethoxy und Methoxyethoxy-n-propoxy verstanden.

Erfindungsgemäß wird unter Alkylthio ein geradkettiges oder verzweigtes Alkylthio mit vorzugsweise 1 bis 6 Kohlenstoffatomen, insbesondere 1 bis 4 Kohlenstoffatomen, wie beispielsweise Methylthio, Ethylthio, n-Propylthio, Isopropylthio, n-Butylthio, Isobutylthio, sec-Butylthio und tert-Butylthio verstanden.

Erfindungsgemäß wird unter Alkylsulfinyl ein geradkettiges oder verzweigtes Alkylsulfinyl mit vorzugsweise 1 bis 6 Kohlenstoffatomen, insbesondere 1 bis 4 Kohlenstoffatomen, wie beispielsweise. Methylsulfinyl, Ethylsulfinyl, n-Propylsulfinyl, Isopropylsulfinyl, n-Butylsulfinyl, Isobutylsulfinyl, sec-Butylsulfinyl und tert-Butylsulfinyl verstanden.

Erfindungsgemäß wird unter Alkylsulfonyl ein geradkettiges oder verzweigtes Alkylsulfonyl mit vorzugsweise 1 bis 6 Kohlenstoffatomen, insbesondere 1 bis 4 Kohlenstoffatomen, wie beispielsweise Methylsulfonyl, Ethylsulfonyl, n-Propylsulfonyl, Isopropylsulfonyl, n-Butylsulfonyl, Isobutylsulfonyl, sec-Butylsulfonyl und tert-Butylsulfonyl verstanden.

Erfindungsgemäß wird unter Halogenalkylthio ein geradkettiges oder verzweigtes Halogenalkylthio mit vorzugsweise 1 bis 6 Kohlenstoffatomen, insbesondere 1 bis 4 Kohlenstoffatomen, wie beispielsweise Difluormethylthio, Trifluormethylthio, Trichlormethylthio, Chlordifluormethylthio, 1-Fluorethylthio, 2-Fluorethylthio, 2,2-Difluorethylthio, 1,1,2,2-Tetrafluorethylthio, 2,2,2-Trifluorethylthio und 2-Chlor-1,1,2-trifluorethylthio verstanden.

Erfindungsgemäß wird unter Halogenalkylsulfinyl ein geradkettiges oder verzweigtes Halogenalkylsulfinyl mit vorzugsweise 1 bis 6 Kohlenstoffatomen, insbesondere 1 bis 4 Kohlenstoffatomen, wie beispielsweise Difluormethylsulfinyl, Trifluormethylsulfinyl, Trichlormethylsulfinyl, Chlordifluormethylsulfmyl, 1-Fluorethylsulfinyl, 2-Fluorethylsulfinyl, 2,2-Difluorethylsulfinyl, 1,1,2,2-Tetrafluorethylsulfinyl, 2,2,2-Trifluorethylsulfinyl und 2-Chlor-1,1,2-trifluorethylsulfinyl verstanden.

Erfindungsgemäß wird unter Halogenalkylsulfonyl ein geradkettiges oder verzweigtes Halogenalkylsulfonyl mit vorzugsweise 1 bis 6 Kohlenstoffatomen, insbesondere 1 bis 4 Kohlenstoffatomen wie beispielsweise Difluormethylsulfonyl; Trifluormethylsulfonyl, Trichlormethylsulfonyl, Chlordifluormethylsulfonyl, 1-Fluorethylsulfonyl, 2-Fluorethylsulfonyl, 2,2-Difluorethylsulfonyl, 1,1,2,2-Tetrafluorethylsulfonyl, 2,2,2-Trifluorethylsulfonyl und 2-Chlor-1,1,2-trifluorethylsulfonyl verstanden.

Erfindungsgemäß wird unter Alkylcarbonyl geradkettiges oder verzweigtes Alkylcarbonyl mit vorzugsweise 1 bis 6 Kohlenstoffatomen, insbesondere 1 bis 4 Kohlenstoffatomen, wie beispielsweise Methylcarbonyl, Ethylcarbonyl, n-Propylcarbonyl, Isopropylcarbonyl, sec-Butylcarbonyl und tert-Butylcarbonyl verstanden.

Erfindungsgemäß wird unter gegebenenfalls substituiertes Cycloalkylcarbonyl ein mono-, bi- und tricyclisches Cycloalkylcarbonyl, vorzugsweise mit 3 bis 10 Kohlenstoffatomen, insbesondere 3, 5 oder 7 Kohlenstoffatomen im Cycloalkylteil verstanden. Beispielhaft seien Cyclopropylcarbonyl, Cyclobutylcarbonyl, Cyclopentylcarbonyl, Cyclohexyl-carbonyl, Cycloheptylcarbonyl, Cyclooctylcarbonyl, Bicyclo[2.2.1]heptylcarbonyl, Bycyclo[2.2.2]octylcarbonyl und Adamantylcarbonyl genannt.

Erfindungsgemäß wird unter Alkoxycarbonyl ein geradkettiges oder verzweigtes Alkoxycarbonyl mit vorzugsweise 1 bis 6 Kohlenstoffatomen, insbesondere 1 bis 4 Kohlenstoffatomen im Alkoxyteil verstanden. Beispielsweise seien Methoxycarbonyl, Ethoxycarbonyl, n-Propoxycarbonyl, Isopropoxycarbonyl, sec-Butoxycarbonyl und tert-Butoxycarbonyl genannt.

Aryl steht erfindungsgemäß beispielsweise für einen ein-, zwei- oder mehrkernigen aromatischen Rest wie Phenyl, Naphthyl, Tetrahydronaphthyl, Indanyl und Fluorenyl. Unter den Arylen ist Phenyl oder Naphthyl, insbesondere Phenyl bevorzugt.

Erfindungsgemäß bedeutet gebenenfalls substituiertes Arylalkyl, ein gegebenenfalls am Aryl und/oder' Alkyl substituiertes Arylalkyl, wobei der Arylteil vorzugsweise 6 oder 10 Kohlenstoffatome aufweist, wie Phenyl oder Naphthyl, insbesondere 6 Kohlenstoffatome , wie Phenyl, und der Alkylteil vorzugsweise 1 bis 4, insbesondere 1 oder 2 Kohlenstoffatome aufweist und geradkettig oder verzweigt sein kann. Unter den Arylalkylen sind Benzyl und 1-Phenylethyl bevorzugt.

Erfindungsgemäß wird unter Hetaryl 5- bis 7-gliedrige Ringe mit vorzugsweise 1 bis 3, insbesondere 1 oder 2 gleichen oder verschiedenen Heteroatomen, nämlich Sauerstoff, Schwefel oder Stickstoff oder anderen geeigneten Atomen verstanden. Bevorzugte Hetaryle sind Furyl, Thienyl, Pyrazolyl, Imidazolyl, 1,2,3- und 1,2,4-Triazolyl, Isoxazolyl, Thiazolyl, Isothiazolyl, 1,2,3-, 1,3,4-, 1,2,4- und 1,2,5-Oxadiazolyl, Azepinyl, Pyrrolyl, Pyridyl, Pyridazinyl, Pyrimidinyl, Pyrazinyl, 1,3,5-, 1,2,4- und 1,2,3-Triazinyl, 1,2,4-, 1,3,2-, 1,3,6- und 1,2,6-Oxazinyl, Oxepinyl, Thiepinyl, 1,2,4-Triazolonyl und 1,2,4-Diazepinyl.

Erfindungsgemäß wird unter Hetarylalkyl 5- bis 7-gliedrige Ringe mit vorzugsweise 1 bis 3, insbesondere 1 oder 2 gleichen oder verschiedenen Heteroatomen verstanden. Bevorzugte Hetarylalkyle sind Furylmethyl, Thienylmethyl, Pyrazolylmethyl, Imidazolylmethyl, 1,2,3- und 1,2,4-Triazolylmethyl, Isoxazolylmethyl, Thiazolylmethyl, Isothiazolylmethyl, 1,2,3-, 1,3,4-, 1,2,4- und 1,2,5-Oxadiazolylmethyl, Azepinylmethyl, Pyrrolylmethyl, Pyridylmethyl, Pyridazinylmethyl, Pyrimidinylmethyl, Pyrazinylmethyl, 1,3,5-, 1,2,4- und 1,2,3-Triazinylmethyl, 1,2,4-, 1,3,2-, 1,3,6- und 1,2,6-Oxazinylmethyl, Oxepinylmethyl, Thiepinylmethyl und 1,2,4-Diazepinylmethyl.

Erfindungsgemäß wird unter Heterocyclyl gesättigte oder teilweise ungesättigte 3- bis 7-gliedrige monocyclische Ringe, mit vorzugsweise 1 bis 3, insbesondere 1 oder 2 gleichen oder verschiedenen Heteroatomen nämlich Sauerstoff, Schwefel oder Stickstoff oder andere geeignete Atome oder gesättigte oder teilweise ungesättigte bicyclische Ringsysteme, bestehend aus 8 bis 14 Atomen, welche entweder in einem Ring oder beiden Ringen unabhängig voneinander 1 bis 5 gleiche oder verschiedene Heteroatome ausgewählt aus Sauerstoff, Schwefel oder Stickstoff enthalten verstanden. Bevorzugte Heterocyclyle sind Piperidinyl, Piperazinyl, Morpholinyl, Thiomorpholinyl, Dihydropyranyl, Tetrahydropyranyl, Dioxanyl, Pyrrolinyl, Pyrrolidinyl, Imidazolinyl, Imidazolidinyl, Thiazolidinyl, Oxazolidinyl, Dioxolanyl, Dioxolyl, Pyrazolidinyl, Tetrahydrofuranyl, Dihydrofuranyl, Oxetanyl, Oxiranyl, Azetidinyl, Aziridinyl, Oxazetidinyl, Oxaziridinyl, Oxazepanyl, Oxazinanyl, Azepanyl, Pyrrolidinonyl, Pyrrolidindionyl, Morpholinonyl, Piperazinonyl und Oxepanyl.

Die vorgenannten erfindungsgemäßen Gruppen und die Reste in den allgemeinen Formeln sind gegebenenfalls substituiert, wobei sie dann mindestens einen, bevorzugt 1 bis 3, besonders bevorzugt 1 bis 2 gleiche oder verschiedene Substituenten aufweisen. Das Vorhandensein von zwei gleichen oder verschiedenen Substituenten am gleichen Atom ist denkbar.

Als Substituenten seien beispielhaft und gleichzeitig vorzugsweise aufgeführt:
Alkyl mit vorzugsweise 1 bis 4 Kohlenstoffatomen, wie Methyl, Ethyl, n-Propyl, Isopropyl, n-Butyl, Isobutyl, sec-Butyl und tert-Butyl, insbesondere bevorzugt mit 1 bis 2 Kohlenstoffatomen; Alkoxy mit vorzugsweise 1 bis 4 Kohlenstoffatomen, wie Methoxy, Ethoxy, n-Propoxy, Isopropoxy, n-Butoxy, Isobutoxy, sec-Butoxy und tert-Butoxy, insbesondere bevorzugt mit 1 bis 2 Kohlenstoffatomen; Alkylthio mit vorzugsweise 1 bis 4 Kohlenstoffatomen, wie Methylthio, Ethylthio, n-Propylthio, Isopropylthio, n-Butylthio, Isobutylthio, sec-Butylthio und tert-Butylthio, insbesondere bevorzugt mit 1 bis 2 Kohlenstoffatomen; Halogenalkyl mit vorzugsweise 1 bis 5 Halogenatome, insbesondere 1 bis 3 Halogenatomen, wobei die Halogenatome gleich oder verschieden sind und als Halogenatome, vorzugsweise Fluor, Chlor oder Brom, insbesondere Fluor oder Chlor sehen, wie Difluormethyl, Trifluormethyl, Trichlormethyl; Hydroxy; Halogen, vorzugsweise Fluor, Chlor, Brom und Iod, insbesondere Fluor und Chlor; Cyan; Nitro; Amino; Monoalkyl- und Dialkylamino, mit vorzugsweise 1 bis 4 Kohlenstoffatomen je Allylgruppe, wie Methylamino, Mcthylethylamino, Dimethylamino, n-Propylamino, Isopropylamino, Methyl-n-butylamino insbesondere bevorzugt mit 1 oder 2 Kohlenstoffatomen; Alkylcarbonylreste wie Methylcarbonyl; Alkoxycarbonyl mit vorzugsweise 2 bis 4 Kohlenstoffatomen, insbesondere 2 bis 3 Kohlenstoffatomen wie Methoxycarbonyl und Ethoxycarbonyl; Alkylsulfinyl mit 1 bis 4, insbesondere 1 bis 2 Kohlenstoffatomen; Halogenallcylsulfinyl mit 1 bis 4, insbesondere 1 bis 2 Kohlenstoffatomen und 1 bis 5 Halogenatomen wie Trifluormethylsulfinyl; Halogenalkylsulfonyl mit 1 bis 4, insbesondere 1 bis 2 Kohlenstoffatomen und 1 bis 5 Halogenatomen wie Trifluormethylsulfonyl, Perfluor-n-butylsulfonyl, Perfluorisobutylsulfonyl; Arylsulfonyl mit vorzugsweise 6 oder 10 Arylkohlenstoffatomen wie Phenylsulfonyl; Acyl, Aryl, Aryloxy, die ihrerseits einen der oben genannten Substituenten tragen können sowie den Formiminorest (-HC=N-O-Alkyl).

Erfndungsgemäß weisen Mono- oder Dialkylaminogruppen ein geradkettiges oder verzweigtes Alkyl mit vorzugsweise 1 bis 6, insbesondere 1 bis 4 Kohlenstoffatomen auf und sind gegebenenfalls substituiert. Beispielhaft für substituierte Mono- oder Dialkylaminogruppen seien Methylamino, Ethylamino, Dimethylamino, Diethylamino, Di-n-propylamino, Diisopropylamino oder Dibutylamino genannt.

Erfindungsgemäß weist die Alkoxyalkylgruppe in erfindungsgemäßen Mono- oder Dialkoxyalkylaminogruppen ein geradkettiges oder verzweigtes Alkyl mit vorzugsweise 2 bis 6, insbesondere 2 bis 4 Kohlenstoffatomen auf. Mono- oder Dialkoxyalkylaminogruppen sind beispielsweise Methoxymethylamino, Methoxyethylamino, Di-(methoxymethyl)-amino oder Di-(methoxyethyl)-amino.

Als erfindungsgemäß geeignete cyclische Aminogruppen kommen gesättigte und/oder ungesättigte, aromatische oder aliphatische Ringsysteme mit mindestens einem Stickstoffatom als Heteroatom, die ein Ringsystem oder mehrere kondensierte Ringsysteme sein können, und gegebenenfalls weitere Heteroatome wie beispielsweise ein oder zwei Stickstoff-, Sauerstoff- und/oder Schwefelatome enthalten in Frage. Die erfindungsgemäßen cyclischen Aminogruppen können auch mindestens einen Spiroring oder verbrücktes Ringsystem aufweisen bzw. bedeuten. Die Anzahl der Atome, die cyclische Aminogruppen bilden, ist nicht begrenzt, beispielsweise bestehen sie im Falle eines Einringsystems aus 3 bis 8 Atomen, und im Falle eines Zweiringsystems aus 7 bis 11 Atomen.

Erfindungsgemäße cyclische Aminogruppen mit gesättigten und ungesättigten monocyclischen Gruppen mit einem Stickstoffatom als Heteroatom sind beispielsweise 1-Azetidinyl, Pyrrolidino, 2-Pyrrolidin-1-yl, 1-Pyrrolyl, Piperidino, 1,4-Dihydropyrazin-1-yl, 1,2,5,6-Tetrahydropyrazin-1-yl, 1,4-Dihydropyridin-1-yl, 1,2,5,6-Tetrahydropyridin-1-yl, Homopiperidinyl; erfindungsgemäße cyclische Aminogruppen mit gesättigten und ungesättigten monocyclischen Gruppen mit mindestens zwei Stickstoffatomen als Heteroatome sind beispielsweise 1-Imidazolidinyl, 1-Imidazolyl, 1-Pyrazolyl, 1-Triazolyl, 1-Tetrazolyl, 1-Piperazinyl, 1-Homopiperazinyl, 1,2-Dihydro-piperazin-1-yl, 1,2-Dihydropyrimidin-1-yl, Perhydropyrimidin-1-yl, 1,4-Diazacycloheptan-1-yl; erfindungsgemäße cyclische Aminogruppen mit gesättigten und ungesättigten monocyclischen Gruppen mit einem oder zwei Sauerstoffatomen und einem bis drei Stickstoffatomen als Heteroatome sind beispielsweise Oxazolidin-3-yl, 2,3-Dihydroisoxazol-2-yl, Isoxazol-2-yl, 1,2,3-Oxadiazin-2-yl, Morpholino; erfindungsgemäße cyclische Aminogruppen mit gesättigten und ungesättigten monocyclischen Gruppen mit einem bis drei Stickstoffatomen und einem bis zwei Schwefelatomen als Heteroatome sind beispielsweise Thiazolidin-3-yl, Isothiazolin-2-yl, Thiomorpholino, oder Dioxothiomorpholino; erfindungsgemäße cyclische Aminogruppen mit gesättigten und ungesättigten kondensierten cyclischen Gruppen sind beispielsweise Indol-1-yl, 1,2-Dibydrobenzinüdazol-1-yl, Perhydropyrrolo[1,2-a]pyrazin-2-yl; erfindungsgemäße cyclische Aminogruppen mit spirocyclischen Gruppen ist beispielsweise 2-Azaspiro[4,5]decan-2-yl; erfindungsgemäße cyclische Aminogruppen mit verbrückten heterocyclischen Gruppen ist beispielsweise 2-Azabicyclo[2,2,1]heptan-7-yl.

Ferner wurde gefunden, dass die erfindungsgemäßen Verbindungen der Formel (I) stark ausgeprägte biologische Eigenschaften besitzen und vor allem zur Bekämpfung von tierischen Schädlingen insbesondere von Insekten, Spinnentieren und Nematoden, die in der Landwirtschaft, in den Forsten, im Vorrats- und Materialschutz sowie auf dem Hygienesektor vorkommen, geeignet sind. Darüber hinaus sind die erfindungsgemäßen Verbindungen der Formel (I) ebenso zur Bekämpfung von Endo- und Ectoparasiten in der Veterinärmedizin geeignet.

Die Verbindungen der Formel (I) können gegebenenfalls in Abhängigkeit von der Art der Substituenten als geometrische und/oder als optisch aktive Isomere oder entsprechende Isomerengemische in unterschiedlicher Zusammensetzung vorliegen. Die Erfindung betrifft deshalb sowohl die reinen Isomere als auch die Isomerengemische.

Die erfindungsgemäßen Verbindungen sind durch die Formel (I) allgemein definiert.

Bevorzugte Gruppen, Gruppierungen, Substituenten und Bereiche der in den oben und nachstehend erwähnten Formeln aufgeführten Reste werden im Folgenden erläutert.

Bevorzugt steht die Gruppierung -C₂₂R¹-A-C₂₃R²- für -HC=CH- oder -H₂C-CH₂-,
- R³: für sec-Butyl oder iso-Propyl, R⁵ für Wasserstoff und
- R⁴: für gegebenenfalls substituiertes C₂₋₆-kyl, C₁₋₆-Halogenalkyl, insbesondere 2,2,2- Trifluoroethyl oder 1-Fluoro-ethyl; C₃₋₆-Cycloalkyl, insbesondere Cyclopropyl, 1-Cyan-cyclopropyl, 1-Methyl-cyclopropyl, Cyclobutyl oder Cyclopentyl; C₃₋₆-Cycloalkenyl, insbesondere Cyclopentenyl; C₃₋₆-Halogencycloalkyl, insbesondere 2,2-Dichlor-cyclopropyl oder 1-Fluor-cyclopropyl; Cycloalkyl- C₁₋₄-alkyl; Amino-C₁₋₄-alkyl, insbesondere Aminomethyl, Aminoethyl, Aminopropyl oder Aminobutyl; C₁₋₆-Alkylamino-C₁₋₄-alkyl, insbesondere N-Methylamino-methyl, N-Methylamino-ethyl, N-Methylamino-propyl, N-Methylamino-butyl; Di-(C₁₋₆-alkyl)-amino-C₁₋₄-alkyl, insbesondere N,N- Dimethylamino-methyl, N,N-Dimethylamino-ethyl, N,N-Dimethylamino-propyl, N,N- Dimethylamino-butyl, N-Ethyl-N-propylamino-methyl, N-Ethyl-N-propylamino-ethyl; C₁₋₆-Alkoxy- C₁₋₄-alkyl, insbesondere Methoxy-ethyl, Ethoxymethyl, Methoxyethyl, Ethoxyethyl, Methoxypropyl, Ethoxypropyl, Methoxybutyl, Ethoxybutyl; Aryl, insbesondere Phenyl, wobei 4-Chlorphenyl ausgeschlossen ist (bekannt aus US 4,201,861, JP 54-06197); Aryl-C₁₋₄-alkyl, insbesondere Benzyl oder Phenethyl; Heterocyclyl, insbesondere 2-Oxo-pyrrolidinyl oder Pyrrolidinyl; Heterocyclylmethyl, insbesondere N-(2,5-Dioxo-pyrrolidinyl)-methyl, 2-Oxo-piperazinylmethyl, Piperidinylmethyl, N-(3- Oxo-morpholinyl)-methyl; Hetaryl, insbesondere Pyridyl, Pyrimidyl, Pyrazinyl, Pyrazolyl, Thiazolyl, Thienyl, Furyl; Hetaryl-C₁₋₄-alkyl, insbesondere Pyridylmethyl, Pyridylethyl, Pyrazinylmethyl, Pyrimidylmethyl, Thiazolylmethyl, Triazolylmethyl, Pyrazolylmethyl, Pyrrolylmethyl, Furylmethyl, Thienylmethyl, Triazolinonmethyl, Oxadiazolylmethyl, Imidazolylmethyl, Isoxazolylmethyl; wobei die Substituenten ausgewählt sind aus Fluor, Chlor, Brom oder Iod, C₁₋₄-Alkyl, insbesondere Methyl, Ethyl, Isopropyl, C₃₋₆-Cycloalkyl, insbesondere Cyclopropyl, Cyclobutyl, Cyclopentyl oder Cyclohexyl, C₃₋₆-Cycloalkoxy, insbesondere Cyclopropoxy, Cyclobutoxy, Cyclopentoxy oder Cyclohexoxy, C₃₋₆-Cycloalkyl-C₁₋₂-alkoxy, insbesondere Cyclopropylmethoxy oder Cyclopropylethoxy C₁₋₄-Halogenalkyl, insbesondere Trifluormethyl, Amino, Hydroxy, Nitro, Cyan, SO₂OH, COOH, Formyl, C₁₋₄-Alkoxy, insbesondere Methoxy, Ethoxy, Isopropoxy, C₁₋₂- Alkylendioxy, insbesondere Methylendioxy oder Ethylendioxy, Alkylenoxy, insbesondere -H₂C=C(CH₃)-O-, Halogenalkylendioxy, C₁₋₄-Halogenalkoxy, insbesondere Trifluormethoxy, Difluormethoxy, Tetrafluorethoxy, C₁₋₄-Alkylthio, insbesondere Methylthio, C₁₋₄-Alkylsulfinyl, insbesondere Methylsulfinyl, C₁₋₄-Alkylsulfonyl, insbesondere Methylsulfonyl, C₁₋₄-Halogenalkylthio, insbesondere Trifluormethylthio, C₁₋₄-Halogenalkylsulfoxyl, insbesondere Trifluormethylsulfoxyl, C₁₋₄- Halogenalkylsulfonyl, insbesondere Trifluormethylsulfonyl, C₁₋₄-Alkylamino, insbesondere Methylamino, Di-(C₁₋₄-alkyl)-amino, insbesondere *N,N*-Dimethylamino, *N*,*N*-Diethylamino, C₁₋₄- Alkylcarbonyl, insbesondere Methylcarbonyl, Ethylcarbonyl, C₃₋₆-Cycloalkylcarbonyl, insbesondere Cyclopropylcarbonyl, Phenylcarbonyl, C₁₋₄-Alkoxycarbonyl, insbesondere Methoxycarbonyl, Ethoxycarbonyl, C₁₋₄-Alkoxy-C₁₋₄-alkyl, insbesondere Methoxymethyl, Ethoxymethyl, C₁₋₄-Alkoxy- C₁₋₄-alkoxy-C₁₋₄-alkyl, insbesondere Methoxy-ethoxy-methyl, Ethoxy-ethoxy-methyl steht oder für einen Rest R⁴ steht, der ausgewählt ist unter den Resten (G⁷-1) bis (G⁷-45) 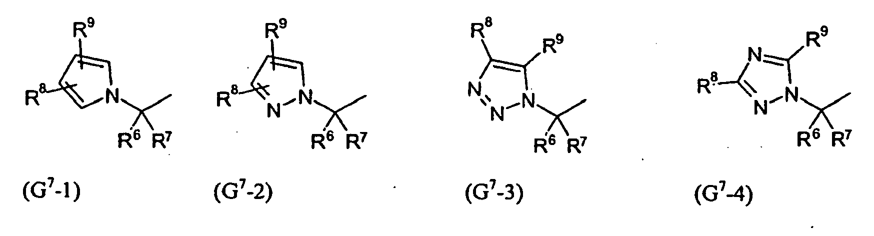 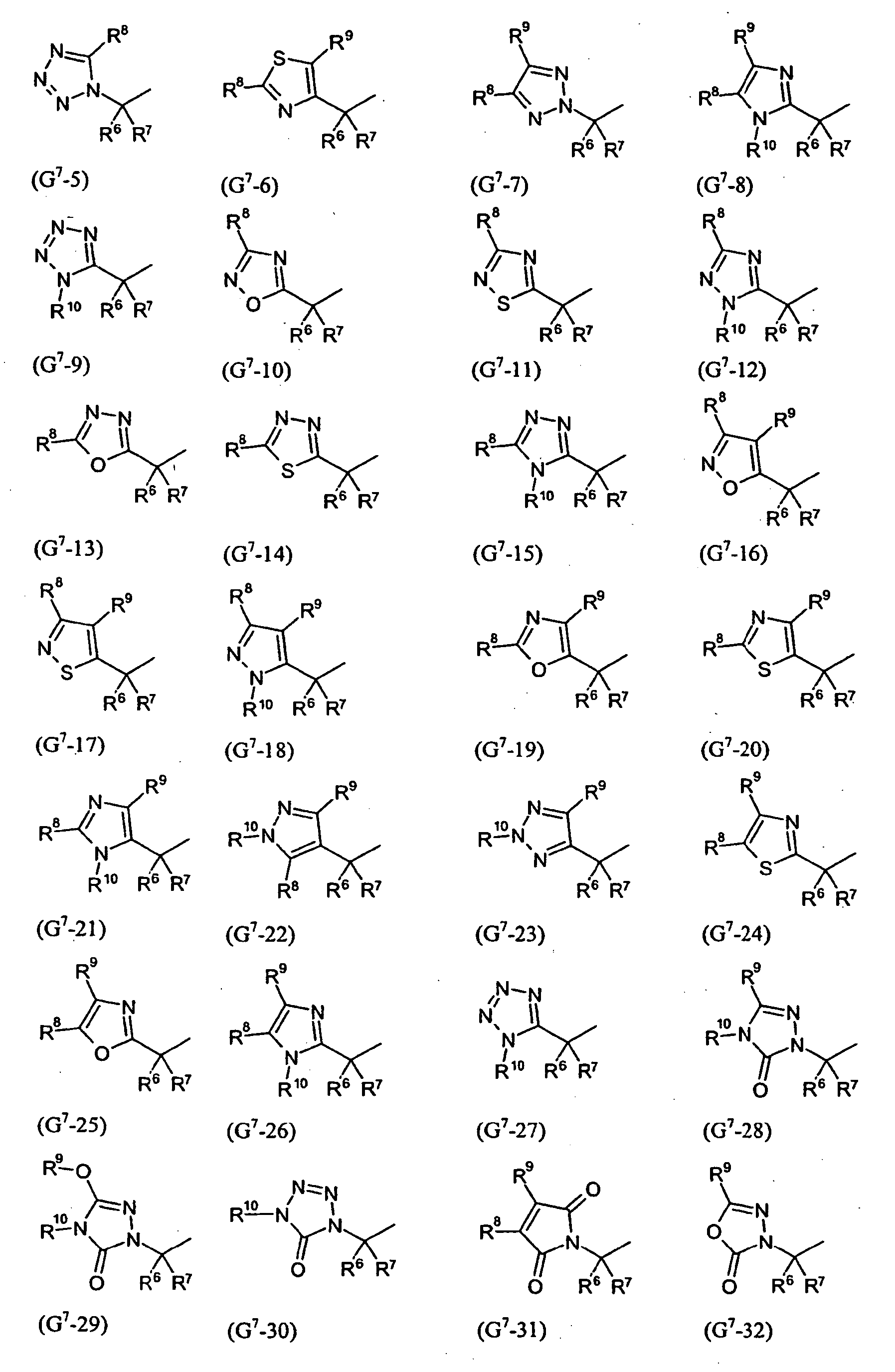 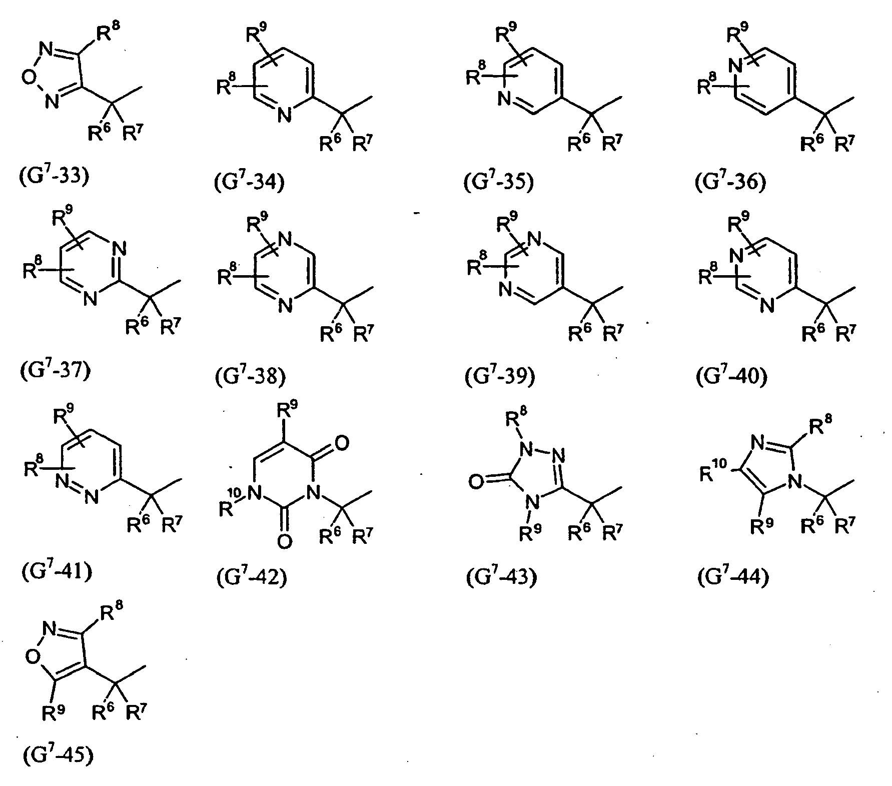
oder für einen Rest steht, der ausgewählt ist unter den Resten (G⁸-1) bis (G⁸-6) 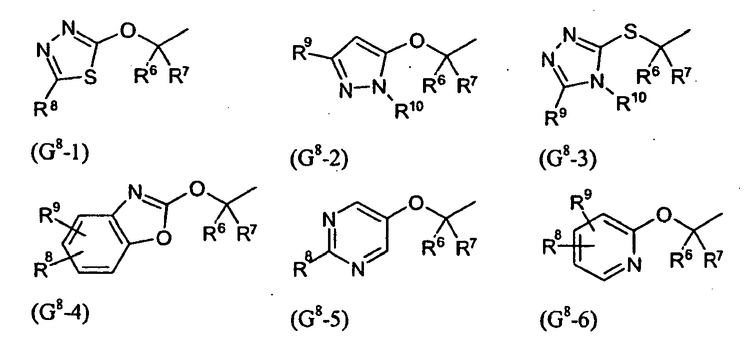 worin R⁶ und R⁷ gemeinsam mit dem Kohlenstoff an das sie gebunden sind unter den Gruppierungen (B-1) bis (B-11) ausgewählt ist 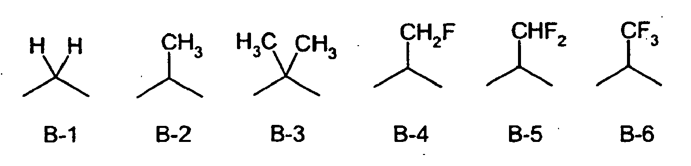 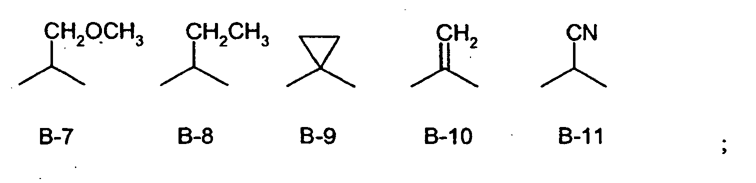 und
- R⁸: bevorzugt für Wasserstoff; C₁₋₄-Alkyl, insbesondere Methyl, Ethyl oder Propyl; C₁₋₄- Halogenalkyl, insbesondere Trifluormethyl oder Difluormethyl; C₁₋₄-Alkoxy, insbesondere Methoxy, Ethoxy; C₃₋₆-Cycloalkyl, insbesondere Cyclopropyl; C₁₋₄-Alkoxy-C₁₋₄-alkoxy, insbesondere Methoxyethoxy, Ethoxyethoxy; C₁₋₄-Halogenalkoxy, insbesondere Trifluormethoxy oder Difluormethoxy; C₁₋₄-Alkylthio, insbesondere Methylthio; C₁₋₄-Alkylsulfinyl, insbesondere Methylsulfinyl; C₁₋₄-Alkylsulfonyl, insbesondere Methylsulfonyl; C₁₋₄-Halogenalkylthio, insbesondere Trifluormethylthio; C₁₋₄-Halogenalkylsulfinyl, insbesondere Trifluormethylsulfinyl; C₁₋₄- Halogenalkylsulfonyl, insbesondere Trifluormethylsulfonyl; Hetaryl, insbesondere Pyridyl; Halogen, insbesondere Fluor, Chlor, Brom oder Iod; Nitro; Cyan; Amino,; C₁₋₄-Alkylamino, insbesondere Methylamino, Ethylamino, Di-(C₁₋₄-alkyl)-amino, insbesondere Dimethylamino, Diethylamino,
oder
bevorzugt für einen Rest aus der Reihe CO-OH; COO⁽⁻⁾; COO-C₁₋₆-alkyl; P(O)(OH)₂; P(O)O⁽⁻⁾₂; CO-NH₂; CS-NH₂; C(=NH)-NH₂; C(=N-OH)-NH₂; CO-NH-C₁₋₄-alkyl, insbesondere CO-NHCH₃; CO-N-(C₁₋₄-alkyl)₂, insbesondere CO-N(CH₃)₂; CO-NH-C₁₋₄-alkoxy, insbesondere CO-NHOCH₃; CO-NH-CO-C₁₋₄-alkyl, insbesondere CO-NH-COCH₃; CO-NH-CO-C₁₋₄-halogenalkyl, insbesondere CO-NH-COCF₃; CO-NH-CO-C₃₋₇-cycloalkyl, insbesondere CO-NH-CO-cyclopropyl; CO-NH-CO-C₁₋₄-alkoxy, insbesondere CO-NH-COOCH₃; CO-NH-CO-(aryl-C₁₋₂-alkyloxy), insbesondere CO-NH-CO-O-benzyl; SO₂-OH, SO₂-O⁽⁻⁾; SO₂-NH₂, SO₂-NH-C₁₋₄-alkyl, insbesondere SO₂-NHCH₃, SO₂-N-(C₁₋₄-alkyl)₂; insbesondere SO₂-N(CH₃)₂; CO-NH-SO₂-NH-C₁₋₄-alkyl, insbesondere CO-NH-SO₂-NHCH₃; CO-NH-SO₂-N[di(C₁₋₄-alkyl), insbesondere CO-NH-SO₂-N(CH₃)₂, steht;
und
- R⁹: bevorzugt für Wasserstoff; C₁₋₄₋Alkyl, insbesondere Methyl, Ethyl, n-Propyl, Isopropyl, Butyl; C₁₋₄-Halogenalkyl insbesondere Trifluormethyl oder Difluormethyl; C₁₋₄-Mkoxy, insbesondere Methoxy, Ethoxy; C₁₋₄-Halogenalkoxy, insbesondere Trifluormethoxy oder Difluormethoxy; C₁₋₄- Alkylthio, insbesondere Methylthio; C₁₋₄₋Alkylsulfmyl, insbesondere Methylsulfinyl; C₁₋₄- Alkylsulfonyl, insbesondere Methylsulfonyl; Halogen, insbesondere Fluor, Chlor, Brom oder Iod; Nitro; Cyan; Formyl; C₁₋₄-Alkylcarbonyl, insbesondere Acetyl, Amino; C₁₋₄-Mkylamino, insbesondere Methylamino, Ethylamino; Di-(C₁₋₄-alkyl)-amino, insbesondere Dimethylamino, Diethylamino; gegebenenfalls substituiertes Aryl, insbesondere Phenyl, 2-, 3- oder 4-Chlorphenyl; gegebenenfalls substitutiertes Pyridyl, insbesondere 3-Chlor-pyrid-2-yl, 2-Brom-pyrid-2-yl; gegebenenfalls substituiertes Heterocyclyl, insbesondere Piperidinyl, Piperazinyl oder Morpholinyl steht;
und
- R¹⁰: bevorzugt für C₁₋₄₋Alkyl, insbesondere Methyl, Ethyl, n-Propyl, Isopropyl, Butyl; C₁₋₄-Halogenalkyl, insbesondere Trifluormethyl oder Difluormethyl; C₁₋₄-Alkyl-carbonyl, insbesondere Acetyl; C₁₋₄-Alkoxycarbonyl, insbesondere Methoxycarbonyl, Ethoxycarbonyl, tert- Butoxycarbonyl, steht,
oder R⁴ für einen Rest steht, der ausgewählt ist unter den Resten (G⁹-1) bis (G⁹-5) 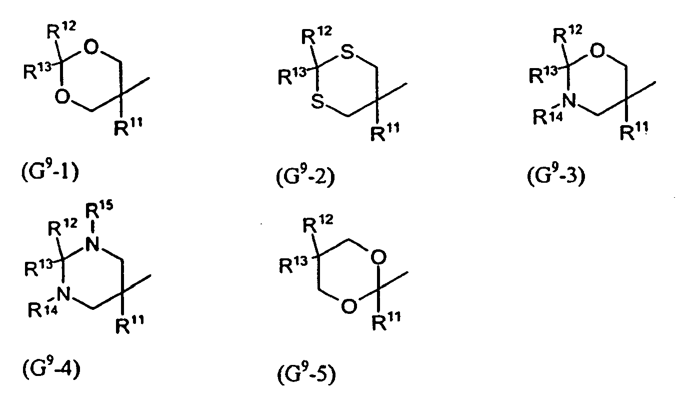 worin
R¹¹ für Wasserstoff, Cyan, C₁₋₄-Alkyl, insbesondere Methyl, Ethyl, n-Propyl, n-Butyl, Isopropyl, iso-Butyl oder sec-Butyl, steht, und
- R¹² und R¹³: unabhängig voneinander bevorzugt für Wasserstoff, C₁₋₄Alkyl, insbesondere Methyl, Ethyl, n-Propyl, n-Butyl, Isopropyl, iso-Butyl oder sec-Butyl; C₂₋₄-Alkenyl, insbesondere 1-Propenyl; C₂₋₄₋Alkinyl, insbesondere 1-Propinyl; C₁₋₆-Alkoxy-C₁₋₄-alkyl, insbesondere Methoxyethyl oder Ethoxyethyl; Amino-C₁₋₄-alkyl, insbesondere Aminomethyl, Aminoethyl, Aminopropyl oder Aminobutyl; C₁₋₆-Alkylamino-C₁₋₄-alkyl, insbesondere Methylaminomethyl; Di-(C₁₋₆-alkyl)-amino- C₁₋₄-alkyl, insbesondere N,N-Dimethylaminomethyl, Hydroxymethyl; Aryloxy-C₁₋₄-alkyl, insbesondere Benzyloxymethyl; C₁₋₆-Alkylcarbonyl, insbesondere Acetyl, gegebenenfalls substituiertes Aryl, insbesondere gegebenenfalls substituiertes Phenyl; Aryl-C₁₋₄-alkyl, insbesondere Benzyl oder Phenethyl; Hetaryl, insbesondere Pyridyl, Pyrimidyl, Pyrazinyl, Pyrazolyl, Thiazolyl, Thienyl, Furyl; Hetarylk-C₁₋₄-alkyl, insbesondere Pyridylmethyl, Pyridylethyl, Pyrazinylmethyl, Pyrimidylmethyl, Thiazolylmethyl stehen können, die gegebenenfalls mit mindestestens einem Substituenten ausgewählt aus Fluor, Chlor, Brom oder Iod, C₁₋₄-Alkyl, insbesondere Methyl, Ethyl, Isopropyl, C₃₋₆-Cycloalkyl, insbesondere Cyclopropyl, Cyclobutyl, Cyclopentyl oder Cyclohexyl, C₃₋₆-Cycloalkoxy, insbesondere Cyclopropoxy, Cyclobutoxy, Cyclopentoxy oder Cyclohexoxy, C₃₋₆- Cycloalkyl-C₁₋₂-alkoxy, insbesondere Cyclopropylmethoxy oder Cyclopropylethoxy; C₁₋₄- Halogenalkyl, insbesondere Trifluormethyl, Amino, Hydroxy, Nitro, Cyan, SO₂OH, COOH, Formyl, C₁₋₄-Alkoxy, insbesondere Methoxy, Ethoxy, Isopropoxy, C₁₋₂-Alkylendioxy, insbesondere Methylendioxy oder Ethylendioxy, Alkylenoxy, insbesondere -H₂C=C(CH₃)-O-, Halogenalkylendioxy, C₁₋₄-Halogenalkoxy, insbesondere Trifluormethoxy, Difluormethoxy, Tetrafluorethoxy, C₁₋₄-Alkylthio, insbesondere Methylthio, C₁₋₄-Alkylsulfinyl, insbesondere Methylsulfinyl, C₁₋₄-Alkylsulfonyl, insbesondere Methylsulfonyl, C₁₋₄-Halogenalkylthio, insbesondere Trifluormethylthio, C₁₋₄-Halogenalkylsulfoxyl, insbesondere Trifluormethylsulfoxyl, C₁₋₄-Halogenalkylsulfonyl, insbesondere Trifluormethylsulfonyl, C₁₋₄-Alkylamino, insbesondere Methylamino, Di-(C₁₋₄-alkyl)-amino, insbesondere *N*,*N*-Dimethylamino, *N,N*-Diethylamino, C₁₋₄- Alkylcarbonyl, insbesondere Methylcarbonyl, Ethylcarbonyl, C₃₋₆-Cycloalkylcarbonyl, insbesondere Cyclopropylcarbonyl, Phenylcarbonyl, C₁₋₄-Alkoxycarbonyl, insbesondere Methoxycarbonyl, Ethoxycarbonyl, substituiert sein können; und Ethyl,
- R¹³: bevorzugt für Wasserstoff, C₁₋₄-Alkyl, insbesondere Methyl, Ethyl, n-Propyl, n-Butyl, Isopropyl, iso-Butyl oder sec-Butyl, C₂₋₄-Alkenyl, insbesondere 1-Propenyl, C₂₋₄-Alkinyl, insbesondere 1-Propinyl, C₁₋₆-Alkoxy-C₁₋₄-alkyl, insbesondere Methoxyethyl oder Ethoxyethyl, C₁₋₆- Alkylamino-C₁₋₄-alkyl, insbesondere Methylaminomethyl, Di-(C₁₋₆-alkyl)-amino-C₁₋₄-alkyl, insbesondere N,N-Dimethylaminomethyl, Hydroxymethyl, Aryloxy-C₁₋₄-alkyl, insbesondere Benzyloxymethyl, C₁₋₆-Alkylcarbonyl, insbesondere Acetyl, gegebenenfalls substituiertes Aryl, insbesondere gegebenenfalls substituiertes Phenyl, gegebenenfalls substituiertes Aryl-C₁₋₄-alkyl, insbesondere gegebenenfalls substituiertes Benzyl, gegebenenfalls substituiertes Hetaryl, insbesondere Pyridyl, steht; und
- R¹⁴: bevorzugt für Wasserstoff; C₁₋₄-Alkyl, insbesondere Methyl, Ethyl, n-Propyl, n-Butyl, Isopropyl, iso-Butyl oder sec-Butyl, steht;
- R¹⁵: bevorzugt für Wasserstoff, C₁₋₄-Alkyl, insbesondere Methyl, Ethyl, n-Propyl, n-Butyl, Isopropyl, iso-Butyl oder sec-Butyl, steht;
oder R⁴ für einen Rest steht, der ausgewählt ist unter den Resten (G¹⁰-1) bis (G¹⁰-3) 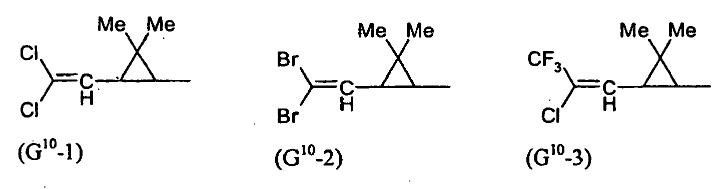 oder R⁴ für einen Rest (G¹¹-1) steht 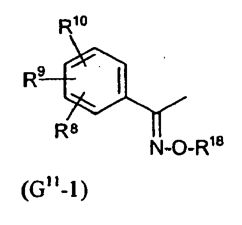 in dem die Reste R⁸, R⁹, R¹⁰ die oben genannte Bedeutung haben; und
- R¹⁸: bevorzugt für gegebenenfalls substituiertes C₁₋₄-Alkyl, insbesondere Methyl, Ethyl steht;
oder R⁴ für einen Rest (G¹²-1) oder (G¹⁴-1) steht 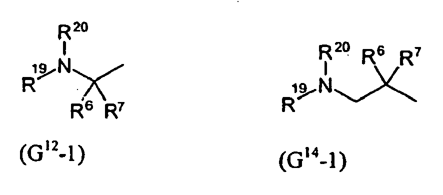 in denen R⁶ und R⁷ die oben genante Bedeutung haben; und
- R¹⁹ und R²⁰: unabhängig voneinander bevorzugt für Wasserstoff oder gegebenenfalls substituiertes C₁₋₄-Alkyl, insbesondere Methyl, Ethyl, C₁₋₄-Alkoxycarbonyl, insbesondere tert-Butyloxycarbonyl, C₁₋₄-Alkylcarbonyl, insbesondere Acetyl, steht, oder R¹⁹ und R²⁰ gemeinsam mit dem Stickstoffatom, an das sie gebunden sind, bevorzugt für gegebenenfalls substituiertes Pyrrolidin, Morpholin, Thiomorpholin, 2,6-Dimethyl-morpholin, 3-Oxo-morpholin, gegebenenfalls substituiertes Piperidin, insbesondere für tert-Butyloxycarbonyl-amino-substituiertes oder Amino-substituiertes Piperidin, gegebenenfalls substituiertes Piperazin, insbesondere für tert-Butyloxycarbonyl-substituiertes Piperazin, N-Benzyl-piperazin, Methyl-substituiertes Piperazin, oder 2,5-Diketo-morpholin steht.

Besonders bevorzugte Gruppen, Gruppierungen, Substituenten und Bereiche der in den oben und nachstehend erwähnten Formeln aufgeführten Reste werden im Folgenden erläutert.

Besonders bevorzugt steht die Gruppierung -C₂₂R¹-A-C₂₃R²- für -HC=CH- oder -H₂C-CH₂-,
- R³: für sec-Butyl oder isö-Propyl, R⁵ für Wasserstoff und
- R⁴: für einen Rest ausgewählt aus Ethyl, n-Propyl, Isopropyl, n-Butyl, Isobutyl, sec-Butyl, tert- Butyl, Pentyl, 1-Methylbutyl, 2-Methylbutyl, 3-Methylbutyl, 1,2-Dimethylpropyl, 1,1- Dimethylpropyl, 2,2-Dimethylpropyl, 1-Ethylpropyl, 2-Ethylpropyl, Hexyl, 1-Methylpentyl, 2- Methylpentyl, 3-Methylpentyl, 4-Methylpentyl, 1,2-Dimethylpropyl, 1,3-Dimethylbutyl, 1,4- Dimethylbutyl, 2,3-Dimethylbutyl, 1,1-Dimethylbutyl, 2,2-Dimethylbutyl, 3,3-Dimethylbutyl, 1,1,2- Trimethylpropyl, 1,2,2-Trimethylpropyl und 1-Ethylbutyl und 2-Ethylbutyl; Cyclopropyl, 1-Methyl- cyclobutyl, 1-Cyan-cyclopropyl, 1-Fluor-cyclopropyl, Cyclopentyl, Cyclopentenyl, Cyclohexyl, Methoxymethyl, Methoxyethyl, Ethoxymethyl, Ethoxyethyl, Methoxypropyl, Ethoxypropyl, Methoxybutyl, Ethoxybutyl, 2-Fluorethyl, 3,3,3-Trifluorethyl, 2,2-Dichlor-cyclopropyl, Aminomethyl, Aminoethyl, Aminopropyl, Aminobutyl, N-Methylamino-methyl, N-Methylamino-ethyl, N- Methylamino-propyl, N-Methylamino-butyl, N,N-Dimethylamino-methyl, N,N- Dirnethylamino-ethyl, N,N-Dimethylamino-propyl, N,N-Dimethylamino-butyl, N-Ethyl,N-propylanino-methyl, N-Ethyl,N- propylamino-ethyl, Phenyl, Benzyl, Phenethyl, Pyridyl, Pyrimidyl, Pyrazinyl, Pyrazolyl, Thiazolyl, Thienyl, Furyl, Pyridylmethyl, Pyridylethyl, Pyrazinylmethyl, Pyrimidylmethyl, Thiazolylmethyl, 1,2,3-Triazolyl-1-ylmethyl, 1,2,3-Triazolyl-1-yl-2-ethyl, N-Pyrazolylmethyl, N-Pyrrolylmethyl, N- Methyl-pyrrol-2-ylmethyl, Furylmethyl, Thien-3-ylmethyl, Pyrid-2-ylmethyl, Pyrid-3-ylmethyl, 1,2,3,4-Tetrazol-1-ylmethyl steht, die gegebenenfalls mit mindestens einem Subsituenten ausgewählt aus Fluor, Chlor, Brom, Iod, Methyl, Ethyl, Isopropyl, Cyclopropyl, Cyclopropoxy, Cyclopropylmethoxy, Trifluormethyl, Amino, Hydroxy, Nitro, Cyan, SO₂OH, COOH, Formyl, Methoxy, Ethoxy, Isopropoxy, Methylendioxy, Ethylendioxy, Difluormethoxy, Tetrafluorethoxy, Trifluormethoxy, Methylthio, Methylsulfonyl, Trifluormethylthio, Trifluormethylsulfoxyl, Methylamino, *N*,*N*-Dimethylamino, Methylcarbonyl, Cyclopropylcarbonyl, Methoxycarbonyl, Methoxyethoxymethyl substituiert sein kann, unter der Voraussetzung, dass R⁴ nicht 4-Chlorphenyl ist oder R⁴ für einen Rest steht, der ausgewählt ist aus den nachfolgenden Resten 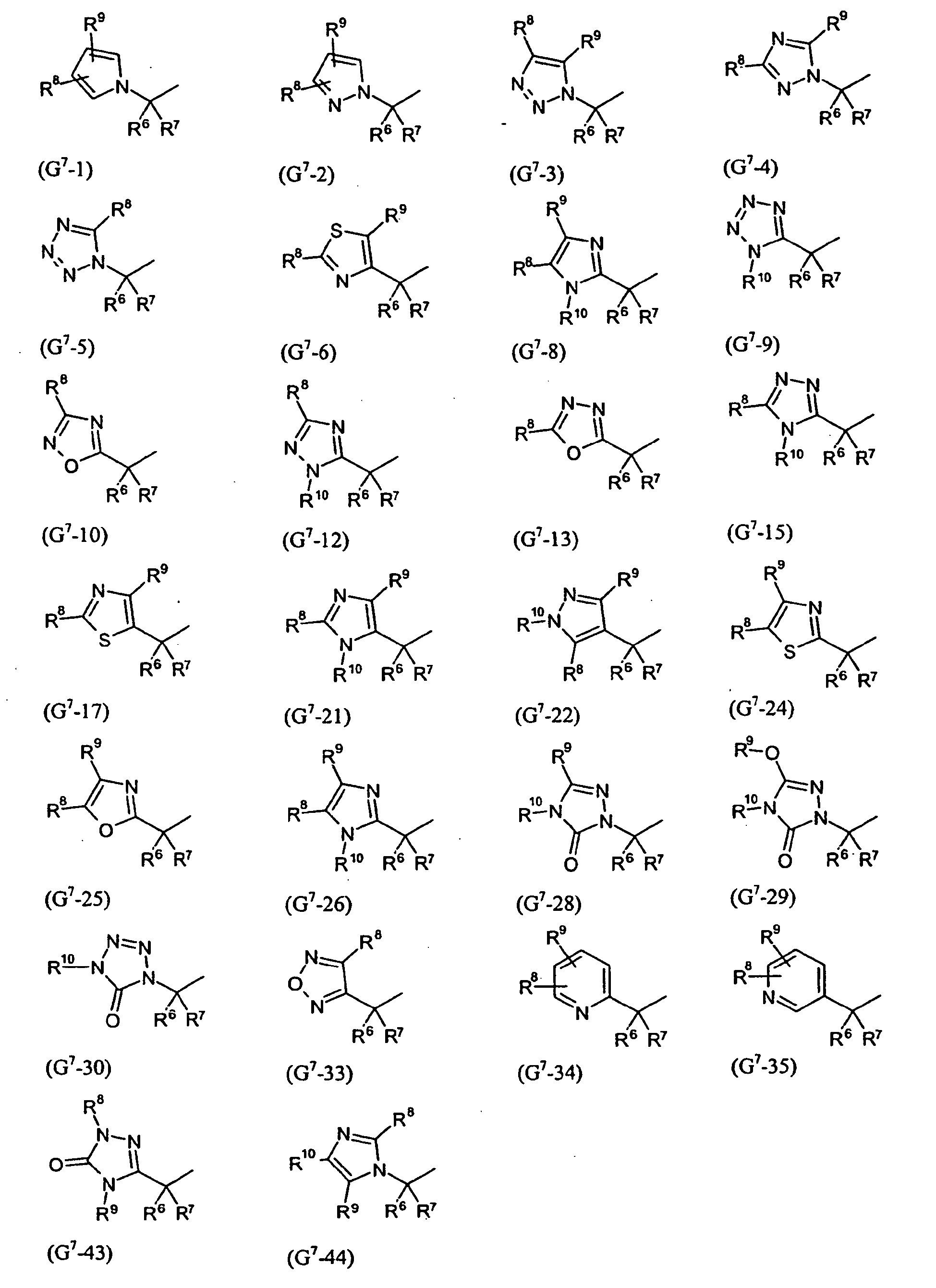
oder für einen Rest (G¹²-1) oder (G¹⁴-1) steht 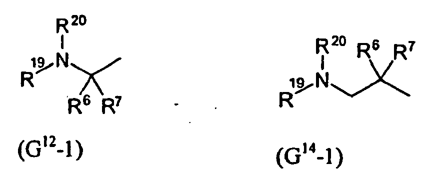 worin
R⁶ und R⁷ gemeinsam mit dem Kohlenstoff an das sie gebunden sind besonders bevorzugt unter den Gruppierungen (B-1), (B-2), (B-3), (B-9) oder (B-10) ausgewählt ist 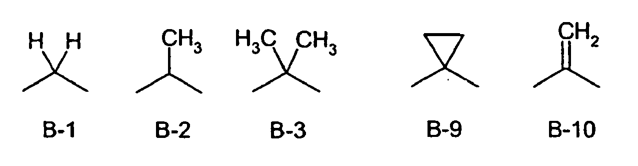
- R⁸: für Wasserstoff, Methyl, Trifluormethyl, Difluormethyl, Methoxy, Methoxyethoxy, Trifluormethoxy, Difluormethoxy, Methylthio, Methylsulfinyl, Methylsulfonyl, Trifluormethylthio, Trifluormethylsulfinyl, Trifluormethylsulfonyl, Cyclopropyl, Pyridyl, Thienyl, Fluor, Chlor, Brom, Iod, Nitro, Cyan, Amino, Methylamino, Dimethylamino, Diethylamino steht oder ausgewählt ist aus CO-OH, COO⁽⁻⁾, COO-C₁₋₆-alkyl, CO-NH₂, CS-NH₂, C(=NH)-NH₂, C(=N-OH)-NH₂, CO-NHCH₃, CO-N(CH₃)₂, CO-NHOCH₃, CO-NH-COCH₃, CO-NH-COOCH₃, CO-NH-CO-O-benzyl, SO₂-OH, SO₂-O⁽⁻⁾, SO₂-NH₂, SO₂-NHCH₃, SO₂-N(CH₃)₂, CO-NH-SO₂-NHCH₃, CO-NH-SO₂-N(CH₃)₂;
- R⁹: für Wasserstoff, Methyl, Trifluormethyl, Methoxy, Trifluormethoxy, Methylthio, Methylsulfinyl, Methylsulfonyl, Fluor, Chlor, Brom, Iod, Nitro, Cyan, Formyl, Acetyl, Amino, Methylamino, Dimethylamino, Diethylamino, Phenyl, 2-, 3- oder 4-Chlorphenyl, 3-Chlor-pyrid-2-yl, Pyrid-4-yl, oder 2-Brom-pyrid-2-yl, steht; und
- R¹⁰: für Wasserstoff, Methyl, Ethyl, n-Propyl, Isopropyl, Butyl, Trifluormethyl, Difluormethyl, Acetyl, Methoxycarbonyl, Ethoxycarbonyl, oder tert-Butoxycarbonyl steht,
oder R⁴ für einen Rest (G⁹-1) steht 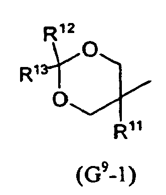 worin R¹¹ Methyl ist, und
- R¹² und R¹³: unabhängig voneinander für Wasserstoff, Methyl, Ethyl, n-Propyl, n-Butyl, Isopropyl, 1-Propenyl, Methoxyethyl, Ethoxyethyl, Methylaminomethyl, N,N-Dimethylaminomethyl, Hydroxymethyl, Benzyloxymethyl, Acetyl, Phenyl, Benzyl, Phenethyl, Pyridyl, Pyrimidyl, Pyrazinyl, Pyrazolyl, Thiazolyl, Thienyl, Furyl, Pyridylmethyl, Pyridylethyl, Pyrazinylmethyl, Pyrimidylmethyl, Thiazolylmethyl stehen, die gegebenenfalls mit mindestens einem Substituenten ausgewählt aus Fluor, Chlor, Brom, Iod, Methyl, Ethyl, Isopropyl, Cyclopropyl, Cyclopropoxy, Cyclopropylmethoxy, Trifluormethyl, Amino, Hydroxy, Nitro, Cyan, SO₂OH, COOH, Formyl, Methoxy, Ethoxy, Isopropoxy, Methylendioxy, Ethylendioxy, Difluormethoxy, Tetrafluorethoxy, Trifluormethoxy, Methylthio, Methylsulfonyl, Trifluormethylthio, Trifluormethylsulfoxyl, Methylamino, *N,N-*Dimethylamino, Methylcarbonyl, Cyclopropylcarbonyl, Methoxycarbonyl substituiert sein können, und
- R¹⁹ und R²⁰: unabhängig voneinander für Wasserstoff oder gegebenenfalls substituiertes C₁₋₄-Alkyl, insbesondere Methyl; C₁₋₄-Alkoxycarbonyl, insbesondere tert-Butyloxycarbonyl; C₁₋₄- Alkylcarbonyl, insbesondere Acetyl steht, oder
- R¹⁹ und R²⁰: gemeinsam mit dem Stickstoffatom, an das sie gebunden sind, für gegebenenfalls substituiertes Pyrrolidin, Morpholin, 2,6-Dimethyl-morpholin, 3-Oxo-morpholin; gegebenenfalls substituiertes Piperidin, insbesondere tert-Butyloxycarbonylamino-substituiertes Piperidin oder Amino-substituiertes Piperidin; gegebenenfalls substituiertes Piperazin, insbesondere tert- Butyloxycarbonyl-substituiertes Piperazin oder Methyl-substituiertes Piperazin steht.

Ganz besonders bevorzugte Gruppen, Gruppierungen, Substituenten und Bereiche der in den oben und nachstehend erwähnten Formeln aufgeführten Reste werden im Folgenden erläutert.

Ganz besonders bevorzugt steht die Gruppierung -C₂₂R¹-A-C₂₃R²- für -HC=CH- oder -H₂C-CH₂-, R³ für sec-Butyl, R⁵ für Wasserstoff und

R⁴ für einen Rest ausgewählt aus Ethyl, n-Propyl, Isopropyl, n-Butyl, Isobutyl, sec-Butyl, tert-Butyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, 2-Ethylpropyl, Hexyl, 1-Methylpentyl, 2-Methylpentyl, 3-Methylpentyl, 4-Methylpentyl, 1,2-Dimethylpropyl, 1,3-Dimethylbutyl, 1,4-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl und 1-Ethylbutyl und 2-Ethylbutyl; Cyclopropyl, 1-Methyl-cyclobutyl, Cyan-cyclopropyl, 1-Fluor-cyclopropyl, Aminomethyl, Aminoethyl, N-Methylamino-methyl, N-Methylamino-ethyl, N,N-Dimethylamino-methyl, N,N-Dimethylamino-ethyl, Cyclohexyl, Methoxymethyl, Methoyethyl, 2-Fluorethyl, 3,3,3-Trifluorethyl, 2,2-Dichlor-cyclopropyl, Phenethyl, Pyridylmethyl, Pyridylethyl, Pyrazinylmethyl, Pyrimidylmethyl, 1,2,3-Triazol-1-ylmethyl, N-Pyrazolylmethyl, N-Pyrrolylmethyl, N-Methyl-pyrrol-2-ylmethyl, 1,2,3,4-Tetrazol-1-ylmethyl, die gegebenenfalls mit mindestens einem Substituenten ausgewählt aus Fluor, Chlor, Brom, Iod, Methyl, Trifluormethyl, Amino, Hydroxy, SO₂OH, COOH, Formyl, Methoxy, Trifluormethoxy, Difluormethoxy, Methylamino, *N,N*-Dimethylamino, Methylcarbonyl, Cyclopropylcarbonyl, Methoxycarbonyl substituiert sein können, oder für einen Rest steht, der ausgewählt ist aus den nachfolgenden Resten 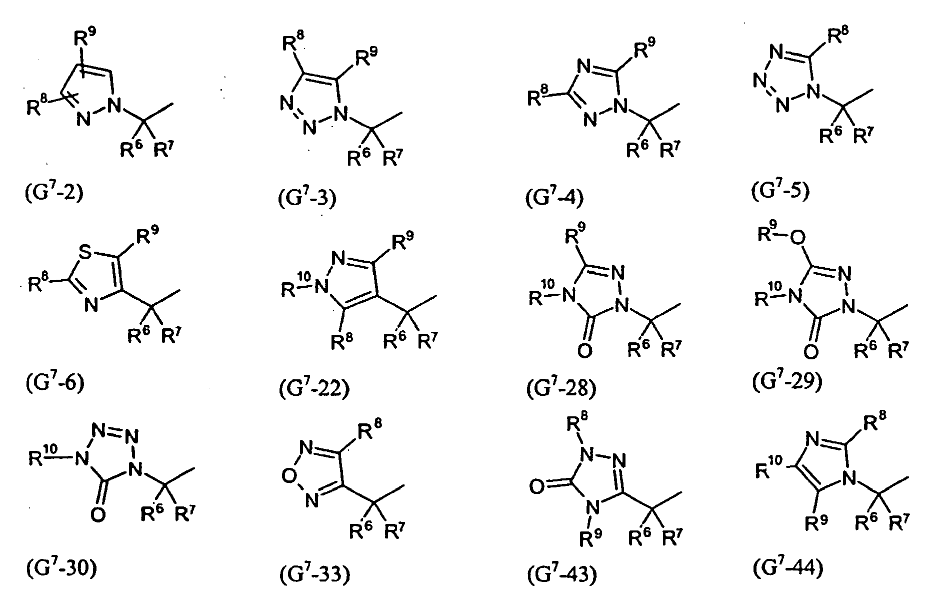 oder für einen Rest (G¹²-1) oder (G¹⁴-1) steht 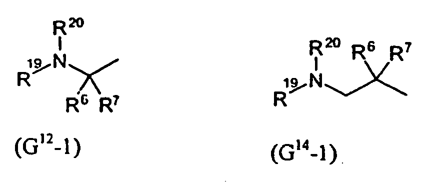 worin
R⁶ und R⁷ gemeinsam mit dem Kohlenstoff an das sie gebunden sind ganz besonders bevorzugt unter den Gruppierungen (B-1), (B-2) (B-3) oder (B-9) ausgewählt ist 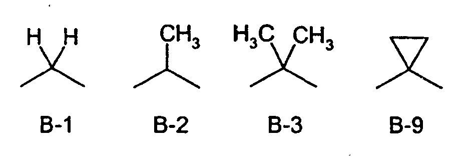 R⁸ für Wasserstoff, Methyl, Trifluormethyl, Methoxy, Trifluormethoxy, Fluor, Chlor, Brom, Iod, Methylamino oder Dimethylamino, steht oder ausgewählt ist unter CO-NH₂, CO-NHCH₃, CO-N(CH₃)₂, CO-NHOCH₃, CO-NH-COCH₃, CO-NH-COOCH₃, CO-NH-CO-O-benzyl, SO₂-NH₂, SO₂-NHCH₃, SO₂-N(CH₃)₂, CO-NH-SO₂-NHCH₃, CO-NH-SO₂-N(CH₃)₂;
R⁹ für Wasserstoff, Methyl, Trifluormethyl, Methoxy, Trifluormethoxy, Fluor, Chlor, Brom, Iod, Acetyl, Methylamino, Dimethylamino, Diethylamino, Phenyl, 4-Chlorphenyl, Pyrid-4-yl steht,
R¹⁰ für Methyl, Acetyl, Methoxycarbonyl, tert-Butoxycarbonyl, steht,
oder ganz besonders bevorzugt für einen Rest aus den nachfolgenden Gruppen (G³) und (G⁹) steht 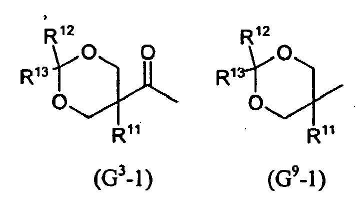 worin
R¹¹ für Methyl steht, und
R¹² und R¹³ unabhängig voneinander für Methyl, Methoxyethyl, Methylaminomethyl stehen, oder R¹² für Wasserstoff, und R¹³ für Methyl, Ethyl, n-Propyl, 1-Propenyl, Methoxyethyl, Ethoxyethyl, Methylaminomethyl, N,N-Dimethylaminomethyl, Phenyl, Benzyl, Phenethyl, Pyridyl, Pyrimidyl, Pyrazinyl, Pyrazolyl, Thiazolyl, Thienyl, Furyl, Pyridylmethyl, Pyridylethyl, Pyrazinylmethyl, Pyrimidylmethyl, Thiazolylmethyl steht, die gegebenenfalls mit mindestens einem Substituent ausgewählt aus Fluor, Chlor, Brom, Iod, Methyl, Ethyl, Isopropyl, Cyclopropyl, Cyclopropoxy, Cyclopropylmethoxy, Trifluormethyl, Amino, Hydroxy, Nitro, Cyan, SO₂OH, COOH, Formyl, Methoxy, Ethoxy, Isopropoxy, Methylendioxy, Ethylendioxy, Difluormethoxy, Tetrafluorethoxy, Trifluormethoxy, Difluormethoxy, Methylthio, Methylsulfonyl, Trifluormethylthio, Trifluormethylsulfoxyl, Methylamino, *N,N*-Dimethylamino, Methylcarbonyl, Cyclopropylcarbonyl, Methoxycarbonyl substituiert sein können.

R¹⁹ und R²⁰ unabhängig voneinander für Wasserstoff, Methyl, Ethyl, tert-Butyloxycarbonyl, Acetyl, stehen, oder

R¹⁹ und R²⁰ gemeinsam mit dem Stickstoffatom, an das sie gebunden sind für Pyrrolidin, Morpholin, 2,6-Dimethyl-morpholin, 3-Oxo-morpholin, Piperidin, tert-Butyloxycarbonyl-amino-substituiertes Piperidin oder 4-Amino-substituiertes Piperidin, Piperazin, tert-Butyloxycarbonyl-substituiertes Piperazin oder 4-Methyl-substituiertes Piperazin steht.

Die oben aufgeführten allgemeinen oder in Vorzugsbereichen aufgeführten Reste-definitionen bzw. Erläuterungen gelten für erfindungsgemäße Endprodukte, Ausgangsprodukte und Zwischenprodukte gleichermaßen. Diese Restedefinitionen können untereinander, als auch zwischen den jeweiligen Vorzugsbereichen, beliebig kombiniert werden.

Erfindungsgemäß bevorzugt werden Verbindungen der Formel (I), in welchen eine Kombination der vorstehend als bevorzugt aufgeführten Bedeutungen vorliegt.

Erfindungsgemäß besonders bevorzugt werden Verbindungen der Formel (I), in welchen eine Kombination der vorstehend als besonders bevorzugt aufgeführten Bedeutungen vorliegt.

Erfindungsgemäß ganz besonders bevorzugt werden Verbindungen der Formel (I), in welchen eine Kombination der vorstehend als ganz besonders bevorzugt aufgeführten Bedeutungen vorliegt.

Die Verbindungen der Formel (I) können gegebenenfalls auch in Abhängigkeit von der Art der Substituenten als Stereoisomere, d.h. als geometrische und/oder als optische Isomere oder Isomerengemische in unterschiedlichen Zusammensetzungen vorliegen. Erfindungsgemäß fallen demnach unter die Formel (I) sowohl die reinen Stereoisomeren als auch beliebige Gemische dieser Isomeren und sind damit auch Gegenstand dieser Erfindung.

Die Erfindung betrifft auch solche Verbindungen der Formel (I), die in Form eines Säweadditionssalzes vorliegen können. Säuren die zur Bildung eines solchen Salzes herangezogen werden sind im Allgemeinen bekannt und sind beispielsweise anorganische Säuren, wie Salzsäure, Bromwasserstoffsäure, Salpetersäure, Schwefelsäure, Phosphorsäure oder organische Säuren wie Ameisensäure, Essigsäure, Propionsäure, Malonsäure, Oxalsäure, Fumarsäure, Adipinsäure, Stearinsäure, Weinsäure, Ölsäure, Methansulfonsäure, Benzolsulfonsäure, Benzolcarbonsäure oder Toluolsulfonsäure.

Als geeignete Salze der Verbindungen der Formel (I) können übliche Salze, d.h. Salze mit verschiedenen Basen und Salze mit zugesetzten Säuren genannt werden, die nicht toxisch sind. Bevorzugt werden Salze mit anorganischen Basen wie Alkalimetallsalze, beispielsweise Natrium-, Kalium- oder Cäsiumsalze, Erdalkalimetallsalze, beispielsweise Kalzium- oder Magnesiumsalze, Ammoniumsalze, Salze mit organischen Basen sowie mit organischen Aminen, beispielsweise Triethylammonium-, Pyridinium-, Picolinium-, Ethanolammonium-, Triethanolammonium-, Dicyclohexylammonium- oder N,N'-Dibenzylethylen-diammoniumsalze, Salze mit anorganischen Säuren, beispielsweise Hydrochloride, Hydrobromide, Dihydrosulfate oder Trihydrophosphate, Salze mit organischen Carbonsäuren oder organischen Sulfosäuren, beispielsweise Formiate, Acetate, Trifluoracetate, Maleate, Tartrate, Methansulfonate, Benzolsulfonate, Benzoate oder para-Toluolsulfonate, Salze mit basischen Aminosäuren oder sauren Aminosäuren, beispielsweise Arginate, Aspartate oder Glutamate.

Setzt man zur Herstellung der neuen erfindungsgemäßen Avermectinderivate als Verbindungen der Formel (II) beispielsweise Abamectin und als Verbindungen der Formel (V) beispielsweise Cyclopropylcarbonsäure in Gegenwart eines basischen Reaktionshilfsmittels ein, so lässt sich das über vier Reaktionsstufen verlaufende oben genannte Herstellungsverfahren durch das folgende Reaktionsschema wiedergeben:

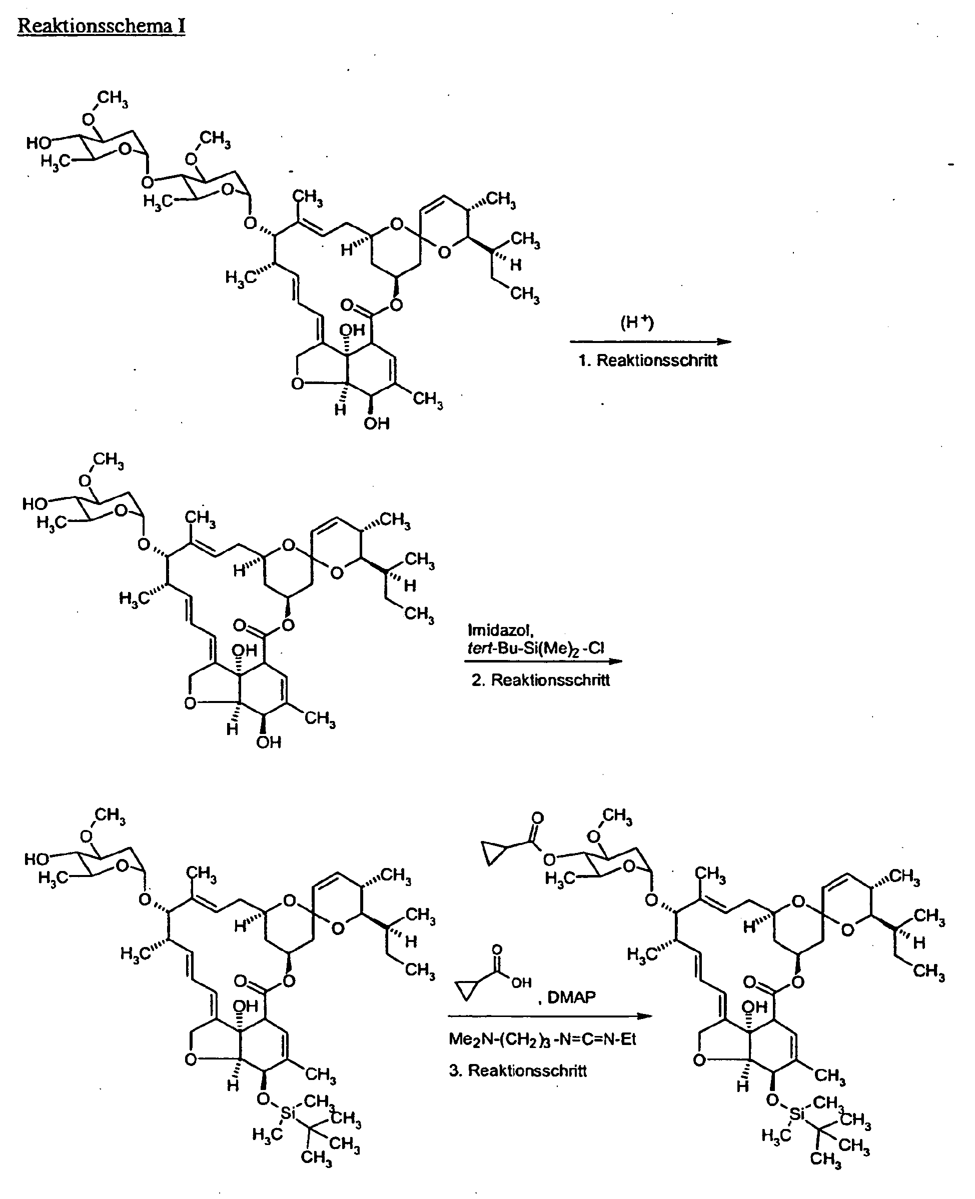

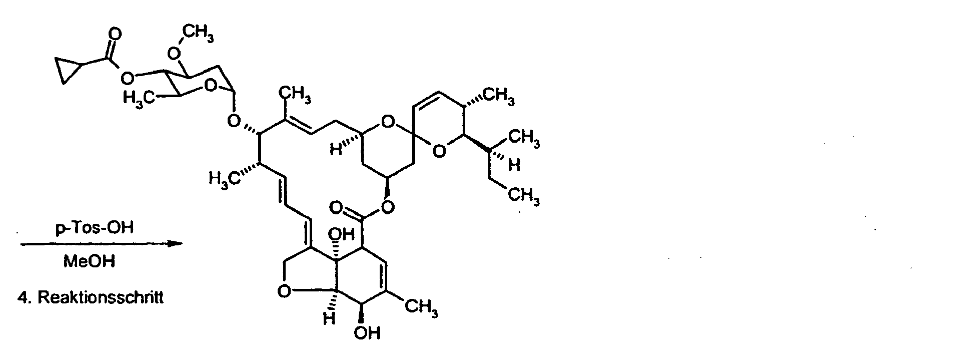

Als geeignete Ausgangsverbindungen der Formel (II) werden makrocyclische Lactone, insbesondere Avermectine und deren Derivate verwendet. Avermectine lassen sich aus dem Mikroorganismus *Strepomyces avermitilis* als mikrobieller Metabolit isolieren (vgl. US 4,310,519) und können im wesentlichen als Gemisch, bestehend aus acht Komponenten A₁ₐ, A_{1b}, A₂ₐ, A_{2b}, B₁ₐ, B_{1b}, B₂ₐ und B_{2b}, auftreten (I. Putter et al., Experentia (1981) 37, S. 963, Birkhäuser Verlag, Schweiz). Als Ausgangsverbindungen eignen sich auch als mikrobieller Metabolit isolierte Avermectine sowie synthetische Derivate der makrocyclischen Lactone, wie insbesondere das 22,23-Dihydroavermectin B₁ (Ivermectin B₁). Bei den erfmdungsgemäß insbesondere geeigneten Ausgangsverbindungen handelt es sich um ein Stoffgemisch makrocyclischer Lactone der Formel (II). 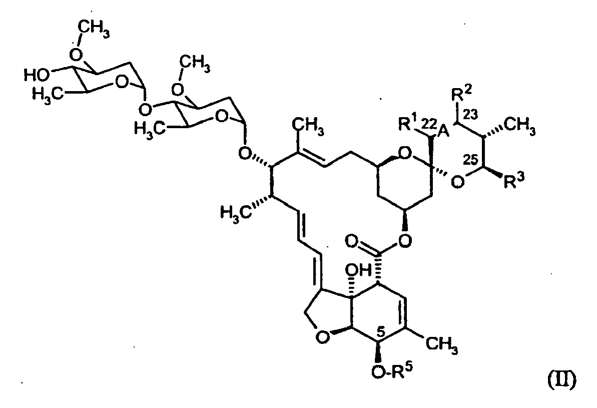 in welcher die Reste R¹, R², R³ und R⁵ die in der nachfolgenden Tabelle (Tabelle 1) angegebenen Bedeutungen haben.

**Tabelle 1**

| **Makrocyclisches Lacton der Formel (II)** | **-C₂₂R¹-A-C₂₃R²-** | **R³** | **R⁵** |
|---|---|---|---|
| Avermectin A₁ₐ | -HC=CH- | *sec*-Butyl | Methyl |
| Avermectin A_{1b} | -HC=CH- | *iso*-Propyl | Methyl |
| Avermectin A₂ₐ | -H₂C-CH(OH)- | *sec*- Butyl | Methyl |
| Avermectin A_{2b} | -H₂C-CH(OH)- | *iso-* Propyl | Methyl |
| Avermectin B₁ₐ | -HC=CH- | *sec-* Butyl | H |
| Avermectin B_{1b} | -HC=CH- | *iso*- Propyl | H |
| Avermectin B₂ₐ | -H₂C-CH(OH)- | *sec*- Butyl | H |
| Avermectin B_{2b} | -H₂C-CH(OH)- | *iso*- Propyl | H |
| 22,23-Dihydroavermectin B₁ₐ (=Ivermectin B₁) | -H₂C-CH₂- | *sec-* Butyl | H |
| 22,23-Dihydroavermectin B_{1b} (=Ivermectin B₁) | -H₂C-CH₂- | *iso-* Propyl | H |
| Doramectin | -HC=CH- | Cyclohexyl | H |

Die Avermectine und das 22, 23-Dihydroavermectin B₁ (Ivermectin B₁) der Formel (II) werden der Regel als Gemische eingesetzt. Zu nennen ist hier insbesondere Abamectin, welches im Wesentlichen die Avermectine B₁ und deren Hydrierungsprodukte, die 22, 23-Dihydroavermectine B₁ (Ivermectin B₁) enthält.

Die makrocyclischen Lactone, die in der C₂₅-Position einen *iso*-Propylrest besitzen und die in der obigen Tabelle mit dem kleinen Buchstaben "b" indiziert sind, müssen nicht notwendiger Weise von den Lactonen getrennt werden, die eine *sec*-Butylgruppe in der C₂₅-Position besitzen und die in obiger Tabelle mit dem kleinen Buchstaben "a" indiziert sind. Im Allgemeinen wird ein Gemisch beider Lactone, das aus > 80% *sec*-Butylderivate (B₁ₐ) und < 20% *iso*-Propylderivat (B_{1b}) besteht isoliert, das sich als erfindungsgemäße Ausgangsverbindung eignet. Als Ausgangverbindung eignet sich auch ein Gemisch bestehend aus > 80% *sec*-Butylderivate (B₂ₐ) und < 20% *iso*-Propylderivat (B_{2b}) oder > 80% *sec*-Butylderivate (A₁ₐ) und < 20% *iso*-Propylderivat (A_{1b}).

Zudem können bei Stereoisomeren die Substituenten in der C₁₃- und C₂₃-Position sowohl α- als auch β-ständig am Ringsystem angeordnet sein, d. h. sich oberhalb oder unterhalb der Molekülebene befinden. In jedem Fall werden alle Stereoisomere erfindungsgemäß berücksichtigt.

Aus der Klasse der macrocyclischen Lactone ist die Verwendung von Abamectin oder 22,23 Dihydroavermectin B₁ (Ivermectin B₁) als Pesticide und Endoparasitizide bekannt und Gegenstand zahlreicher Übersichtsartikel geworden, z B. D. J. Wright "Avermectins: action on target pest species", Biochem. Soc. Trans. (1987) 15, 65-67; L. Strong, T. A. Brown, "Avermectins in insect control and biology: a review", Bull. Entomol. Res. (1987), 77, 357-389; J. A. Lasota, R. A. Dybas, "Abamectin as a pesticide for agricultural use", Acta Leidensia (1990), 59, 217-225; J. A. Lasota, R. A. Dybas, "Avermectins, a novel class of compounds: implications for use in arthropod pest control", Ann. Rev. Entomol. (1991) 36, 91-117; L. Strong "Overview: the impact of avermectins on pastureland ecology", Vet. Parasitol. (1993), 48, 3-17; W. "Ivermectin and Abamectin" (Ed. C. Campbell), Springer-Verlag, New York, N. Y. 1989; I. H. Sutherland, "Veterinary use of ivermectin", Acta Leidensia (1990) 59, 211-216; A. Datry, M. Thellier "Ivermectin, a broad spectrum antiparasitic drug", Presse medicale (Paris, France: 1983) (2002) 31, 607-611; R. O. Drummond "Effectiveness of ivermectin for control of arthropod pests of livestock", (1985) 7, 34-42; W. C. Campbell "Iverectin, an antiparasitic agent" Med. Res. Rev. (1993), 13, 61-79; G. A. Conder "Chemistry, pharmacology and safety: doramectin and selamectin" Macrocyclic Lactones in Antiparasitic Therapy (2002), 30-50; A. C. Goudie et al., "Doramectin - a potent novel endectozide", Vet. Parasitol. 1993, 49, 5-15).

Manche Verbindungen der Formel (III) sind bekannt und können entsprechend der hier beschriebenen oder literaturbekannten Methoden hergestellt werden.

Bekannt sind beispielsweise Verbindungen der Formel (III) in denen
a) -C₂₂R¹-A-C₂₃R²- für -HC=CH- und R³ für sec-Butyl steht (JP 54-061198, EP 0 004 812, J. C. Chabala et al. J. Med. Chem. (1980), 23, 1134-1136, US 4,201,861, H. Mrozik et al., J. Org. Chem. (1982), 47, 489-492, EP 0 411 897, WO 2002/012248, Q. Wu et al., Nongyao (2004), 43, 28-29, CN 1502239) bzw. R³ für iso-Propyl steht (WO 93/018779, J. C. Chabala et al. J. Med. Chem. (1980), 23, 1134-1136) oder R³ für Cyclohexyl steht (US 5,981,500, WO 94/015944); oder
b) -C₂₂R¹-A-C₂₃R²- für -H₂C-CH₂- und R³ für sec-Butyl steht (JP 54-061198, US 4,199,569, EP 0 004 812, J. C. Chabala et al. J. Med. Chem. (1980), 23, 1134-1136, WO 93/018779, WO 94/015944, US 5,981,500, WO 2002/012248) bzw. R³ für iso-Propyl steht (EP 0 411 897, J. C. Chabala et al. J. Med. Chem. (1980), 23, 1134-1136, WO 93/018779) oder R³ für Cyclohexyl steht (US 5,981,500, WO 94/015944, WO 94/029328, WO 95/003317); oder
c) -C₂₂R¹-A-C₂₃R²- für -H₂C-CH(OH)- und R³ für sec-Butyl steht (JP 54-061198, US 4,199,569, J. C. Chabala et al. J. Med. Chem. (1980), 23, 1134-1136, US 4,201,861, EP 0 004 812, US 4,206,205, H. Mrozik et al., J. Org. Chem. (1982), 47, 489-492) bzw. R³ für iso-Propyl steht (J. C. Chabala et al. J. Med. Chcm. (1980), 23, 1134-1136) oder R³ für Cyclohexyl steht (WO 94/029328).

Als saure Reaktionshilfsmittel kommen alle Mineralsäuren, organische Säuren oder Lewis Säuren in Frage. Bevorzugte Mineralsäuren sind Halogenwasserstoffsäuren wie Fluorwasserstoffsäure, Chlorwasserstoffsäure, Bromwasserstoffsäure oder Iodwasserstoffsäure, Schwefelsäure, Phosphorsäure, Phosphorige Säure, Salpetersäure. Bevorzugte organischen Säuren sind Ameisensäure, Essigsäure, Propionsäure, Malonsäure, Milchsäure, Oxalsäure, Fumarsäure, Adipinsäure, Stearinsäure, Weinsäure, Ölsäure, Methansulfonsäure, Benzoesäure, Benzolsulfonsäure oder para-Toluolsulfonsäure. Bevorzugte Lewis Säuren sind Aluminium(III)-chlorid, Bortrifluorid oder sein Etherat, Titan(V)chlorid, Zinn(V)-chlorid.

Bevorzugt wird der erste Reaktionsschritt in Gegenwart von Mineralsäuren, insbesondere Schwefelsäure durchgeführt.

Zur Herstellung von Verbindungen der Formel (IV), in welcher R⁴ beispielsweise für Wasserstoff steht, können beispielsweise als geeignete Schutzgruppen für Hydroxylgruppen substituierte Methylether und Ether, substituierte Ethylether, substituierte Benzylether, Silylether, Ester, Carbonate oder Sulfonate verwendet werden (vgl. Greene T. W., Wuts P. G. W. in Protective Groups in Organic Synthesis; John Wiley & Sons, Inc. 1999, "Protection for the hydroxyl group including 1,2- and 1,3-diols") verwendet werden.

Substituierte Methylether-Schutzgruppen sind beispielsweise Methoxymethylether (MOM), Methylthiomethylether (MTM), (Phenyl-dimethylsilyl)methoxymethylether (SNOM-OR), Benzyloxymethylether (BOM-OR), para-Methoxybenzyloxymethylether (PMBM-OR), para-Nitrobenzyloxymethyl-ether, ortho-Nitrobenzyloxymethylether (NBOM-OR), (4-Methoxyphenoxy)-methylether (p-AOM-OR), Guaiacolmethylether (GUM-OR), tert-Butoxymethylether, 4-Pentyloxymethylether (POM-OR), Silyloxymethylether, 2-Methoxy-ethoxy-methylether (MEM-OR), 2,2,2-Trichlorethoxymethylether, Bis(2-chloretuoxy)-methylether, 2-(Trimethyl-silyl)ethoxymethylether (SEM-OR) oder Methoxymethylether (MM-OR).

Ether-Schutzgruppen sind beispielsweise Tetrahydropyranylether (THP-OR), 3-Bromtetrahydropyranylether (3-BrTHP-OR), Tetrahydrothiopyranylether, 1-Methoxy-cyclohexylether, 2-und 4-Picolylether, 3-Methyl-2-picolyl-N-oxido-ether, 2-Quinolinylmethylether (Qm-OR), 1-Pyrenylmethylether, Diphenylmethylether (DPM-OR), para, para'-Dinitrobenzhydrylether (DNB-OR), 5-Dibenzosuberylether, Triphenylmethylether (Tr-OR), alpha-Naphthyldiphenylmethylether, para-Methoxy-phenyldiphenylmethylether (MMTrOR), Di(para-methoxy-phenyl)phenylmethylether (DMTr-OR), Tri(para-methoxy-phenyl)phenylinethylether (TMTr-OR), 4-(4'-Brom-phenacyloxy) phenyldiphenylmethylether, 4,4',4"-Tris(4,5-dichlorphthalimido-phenyl)methylether (CPTr-OR), 4,4',4"-Tris(benzoyloxyphenyl)-methylether (TBTr-OR), 4,4'-Dimethoxy-3"-[N-(imidazolylmethyl)]-tritylether (IDTr-OR), 4,4'-Dimethoxy-3"-[N-(imidazolylethyl)carbamoyl]tritylether (IETr-OR), 1,1-Bis(4-methoxy-phenyl)-1'-pyrenyl-methylether (Bmpm-OR), 9-Anthrylether, 9-(9-Phenyl)xanthenylether (Pixyl-OR), 9-(9-Phenyl-10-oxo)anthrylether (Tritylon-Ether), 4-Methoxy-tetrahydropyranylether (MTHP-OR), 4-Methoxytetrahydrothiopyranylether, 4-Methoxy-tetrahydrothiopyranyl-S,S-dioxidether, 1-[(2-Chlor-4-methyl)phenyl]-4-methoxypiperidin-4-yl-ether (CTMP-OR), 1-(2-Fluorphenyl)-4-methoxy-piperidin-4-yl-ether (Fpmp-OR), 1,4-Dioxan-2-yl-ether, Tetrahydrofuranylether, Tetrahydrothiofuranylether, 2,3,3a,4,5,6,7,7a-Octahydro-7,8,8-trimethyl-4,7-methanbemofuran-2-yl-ether (MBF-OR), tert-Butylether, Allylether, Propargylether, para-Chlor-phenylether, para-Methoxy-phenylether, para-Nitro-phenylether, para-2,4-Dinitro-phenylether (DNP-OR), 2,3,5,6-Tetrafluor-4-(trifluorrnethyl)phenylether oder Benzylether (Bn-OR).

Substituierte Ethylether-Schutzgruppen sind beispielsweise 1-Ethoxyethylether (EE-OR), 1-(2-Chlorethoxy)ethylether (CEE-OR), 1-[2-(Trimethylsilyl)ethoxy]ethylether (SEE-OR), 1-Methyl-1-methoxyethylether (MIP-OR), 1-Methyl-1-benzyloxyethylether (MBE-OR), 1-Methyl-1-benzyloxy-2-fluor-ethylether (MIP-OR), 1-Methyl-1-phenoxyethylether, 2,2,2-Trichlorethylether, 1,1-Dianisyl-2,2,2-trichlorethylether (DATE-OR), 1,1,1,3,3,3-Hexafluor-2-phenylisopropylether (HIP-OR), 2-Trimethylsilylethylether, 2-(Benzylthio)ethylether oder 2-(Phenylselenyl)ethylether.

Substituierte Benzylether-Schutzgruppen sind beispielsweise para-Methoxy-benzylether (MPM-OR), 3,4-Dimethoxy-benzylether (DMPM-OR), ortho-Nitro-benzylether, para-Nitro-benzylether, para-Halo-benzylether, 2,6-Dichlor-benzylether, para-Aminoacyl-benzylether (PAB-OR), para-Azidobenzylether (Azb-OR), 4-Azido-3-chlor-benzylether, 2-Trifluormethyl-benzylether, oder para-(Methylsulfinyl)benzylether (Msib-OR).

Silylether-Schutzgruppen sind beispielsweise Trimethylsilylether (TMS-OR), Triethylsilylether (TES-OR), Trüsopropylsilylether (TIPS-OR), Dimethylisopropylsilylether (IPDMS-OR), Diethylisopropylsilylether (DEIPS-OR), Dimethylhexylsilylether (TDS-OR), tert-Butyldimethylsilylether (TBDMS-OR), tert-Butyldiphenylsilylether (TBDPS-OR), Tribenzylsilylether, Tri-paraxylylsilylether, Triphenylsilylether (TPS-OR), Diphenylmethylsilylether (DPMS-OR), Di-tert-butylmethylsilylether (DTBMS-OR), Tris(trimethylsilyl)silylether (Sisylether), (2-Hydroxystyryl)-dimethylsilylether (HSDMS-OR), (2-Hydroxystyryl)diisopropylsilylether (HSDIS-OR), tert-Butylmethoxyphenyl-silylether (TBMPS-OR) oder tert-Butoxydiphenylsilylether (DPTBOS-OR).

Ester-Schutzgruppen sind beispielsweise Formiatester, Benzoylformiatester, Acetatester (Ac-OR), Chloracetatester, Dichloracetatester, Trichloracetatester, Trifluoracetatester (TFA-OR), Methoxyacetatester, Triphenylmethoxyacetatester, Phenoxyacetatester, para-Chlorphenoxyacetatester, Phenylacetatester, Diphenylacetatester (DPA-OR), Nicotinatester, 3-Phenylpropionatester, 4-Pentoatester, 4-Oxo-pentoatester (Levulinate) (Lev-OR), 4,4-(Ethylendithio)-pentanoatester (LevS-OR), 5-[3-Bis(4-methoxyphenyl)hydroxy-methoxyphenoxy]-levulinatester, Pivaloatester (Pv-OR), 1-Adamantanoatester, Crotonatester, 4-Methoxy-crotonatester, Benzoatester (Bz-OR), para-Phenyl-benzoatester, 2,4,6-Trimethyl-benzoatester (Mesitoate), 4-(Methylthiomethoxy)-butyratester (MTMB-OR), oder 2-(Methylthiomethoxymethyl)-benzoatester (MTMT-OR).

Carbonat-Schutzgruppen sind beispielsweise Methylcarbonat, Methoxymethylcarbonat, 9-Fluorenylmethylcarbonat (Fmoc-OR), Ethylcarbonat, 2,2,2-Trichlorethylcarbonat (Troc-OR), 1,1-Dimethyl-2,2,2-trichlor-ethylcarbonat (TCBOC-OR), 2-(Trimethylsilyl)ethylcarbonat (TMSEC-OR), 2-(Phenylsulfonyl)-ethylearbonat (Psec-OR), 2-(Triphenylphosphonio)-ethylcarbonat (Peoc-OR), tert-Butylcarbonat (Boc-OR), Isobutylcarbonat, Vinylcarbonat, Allylcarbonat (Alloc-OR), para-Nitrophenylcarbonat, Benzylcarbonat (Z-OR), para-Methoxy-benzylcarbonat, 3,4-Dimethoxybenzylcarbonat, ortho-Nitro-benzylcarbonat, para-Nitro-benzylcarbonat, 2-Dansylethylcarbonat (Dnseoc-OR), 2-(4-Nitrophenyl)ethylca;bonat (Npeoc-OR), 2-(2,4-Dinitrophenyl)ethylcarbonat (Dnpeoc). Als Schutzgruppen vom Sulfat-Typ sind beispielsweise zu nennen: Allylsulfonat (Als-OR), Methansulfonat (Ms-OR), Benzylsulfonat oder Tosylat (Ts-OR), 2-[(4-Nitrophenyl)ethyl]sulfonat (Npes-OR).

Bevorzugte Schutzgruppen zur Durchführung des erfindungsgemäßen Herstellungsverfahrens sind solche, die einen Silylrest wie beispielsweise SiMe₂-*tert*-Bu Rest aufweisen.

Verbindungen der Formel (IV) sind teilweise bekannt und können entsprechend den vorbeschriebenen Methoden erhalten werden. Literaturbekannt sind beispielsweise folgenden Verbindungen in denen SG=SiMe₂-*tert*-Bu steht und in denen
a) -C₂₂R¹-A-C₂₃R²- für -HC=CH₂- und R³ für sec-Butyl (Ch. Bliard et al., J. Chem. Soc., Chem. Commun. (1987), 5, 368-370; US 5,229,416; Y. Tsukamoto et al, Bioorg. Med. Chem. Lett. (2000) 8, 19-26, WO 2002012248, WO 2005021569) bzw. R³ für iso-Propyl (EP 0 411 897 A2), WO 2005/021569) steht; und
b) -C₂₂R¹-A-C₂₃R²- für -H₂C-CH₂- und R³ für sec-Butyl (WO 2002/012 248, EP 0 411 997 A2) bzw. R³ für iso-Propyl (US 5,229,415) oder R³ für Cyclohexyl (US 5,981,500, WO 94/015944, B. J. Banks et al., Bioorg. Med. Chem. Lett. (2000), 8, 2017-2025) steht..

Geeignete Verbindungen der Formel (V) sind Carbonsäuren, welche kommerziell erhältlich oder nach literaturbekannten Methoden darstellbar sind. Allgemeine Wege zur Herstellung von Carbonsäuren der Formel (V) werden im nachfolgenden Reaktionsschema III beschrieben: 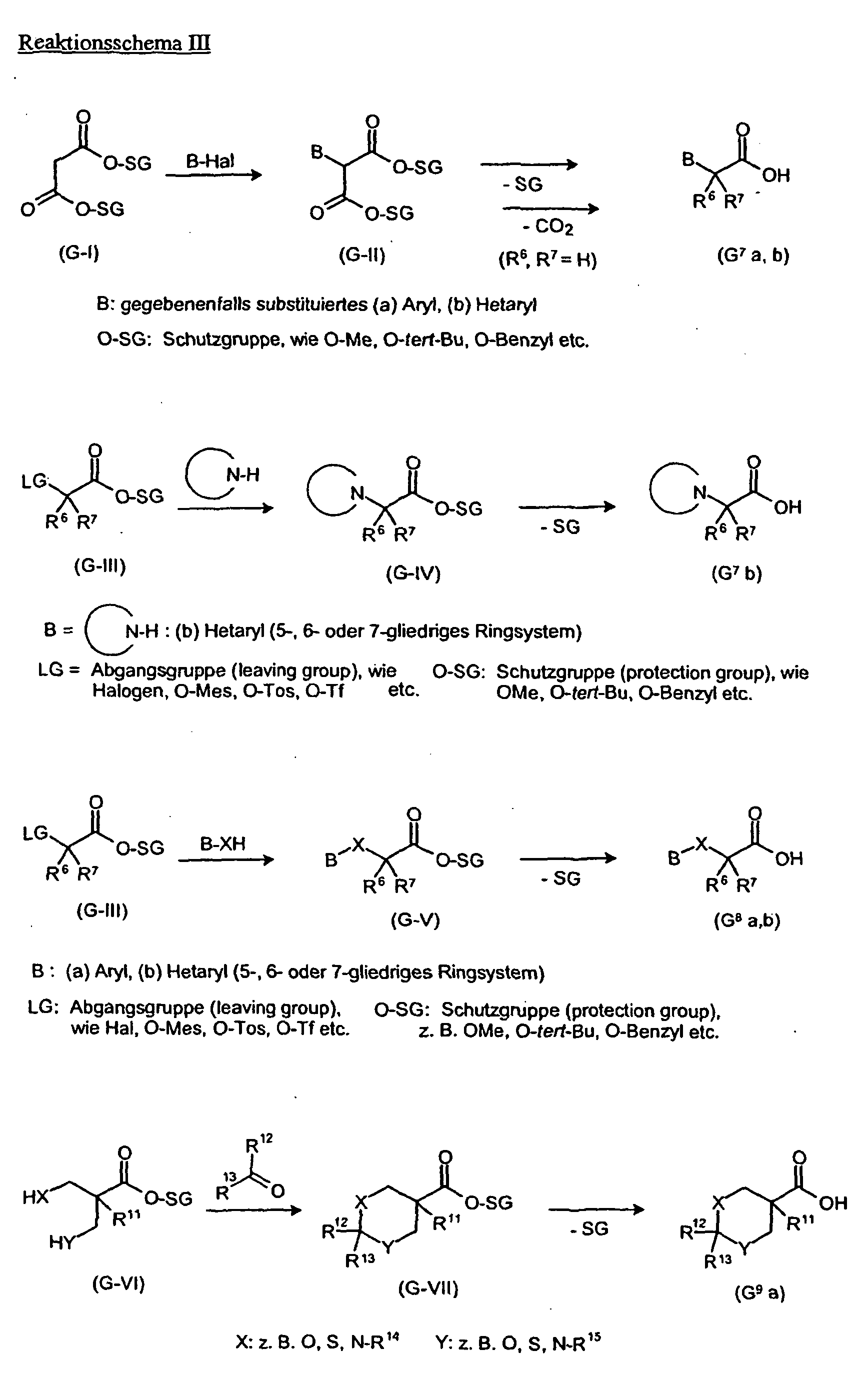 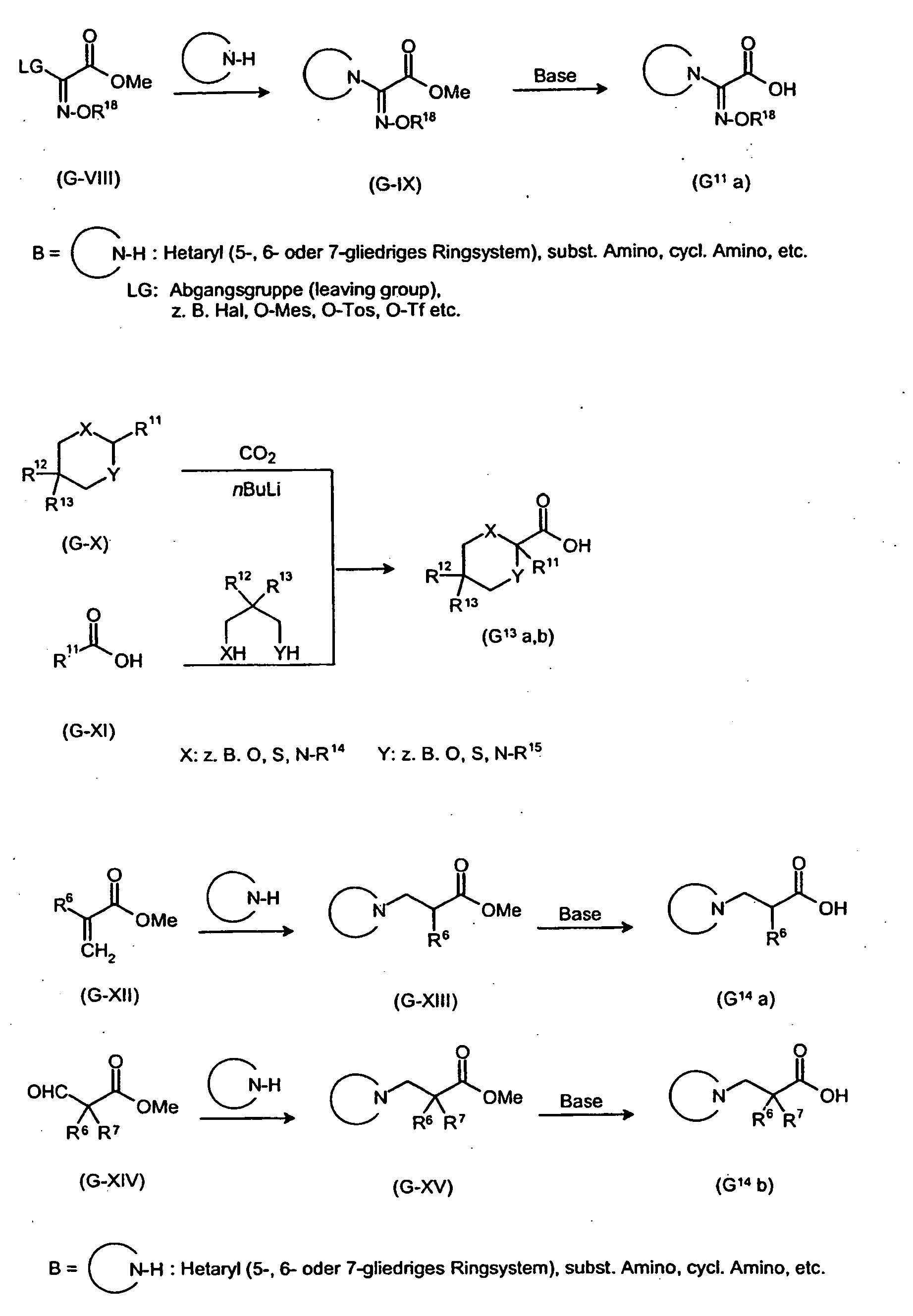 Ein allgemeiner Weg zur Herstellung von (Het)aryl-substituierten Carbonsäuren (G⁷a, b) besteht beispielsweise darin, dass gegebenenfalls geschützte Malonsäurederivate (G-I) (het)aryliert werden und die gebildeten (Het)aryl-malonsäuren (G-II) anschließend O-deblockiert und danach decarboxyliert werden (z. B. (G⁷a): R⁶, R⁷=H, B=Phenyl: Synth. Commun. (2000) 30, 2099-2104; (G⁷b): R⁶, R⁷=H, B=*N*-Pyrazolyl: DE 19503827 A1). Die *N*-hetaryl-substituierten Carbonsäuren (G⁷b) und (het)aryl-substituierten Carbonsäuren (G⁸a, b) können aus den Verbindungen (G-III), erhalten werden. Anschließend erfolgt die Abspaltung der Schutzgruppe in den Verbindungen (G-IV) oder (G-V) nach literaturbekannter Verfahrensweise (vgl. z. B. (G⁸a): R⁶=H, R⁷=Me, X=O, B=4-CF₃-Phenyl: D. Kato et al., J. Org. Chem. (2003), 68, 7234-7242; (G⁸b): R⁶=H, R⁷=Me, X=O, B=3-Cl-pyrid-2-yl: D. Heilmann, G. Kempter, Wiss. Zeitschrift Paedag. Hochschule Karl Liebknecht Potsdam (1981), 25, 35-8).

Zur Darstellung der Carbonsäuren (G⁹a) werden die Verbindungen (G-VI) mit geeigneten Carbonylverbindungen (z. B. Aldehyde, falls R¹² und/oder R¹³=H; Ketone, falls R¹² oder R¹³=aryl, hetaryl, alkyl etc.) zu den funktionalisierten Ringsystemen (G-VII) cyclisiert und anschließend O-deblockiert. Gegebenenfalls kann auch ohne Verwendung einer Schutzgruppe, beispielsweise wenn X, Y=O und SG=OH, gearbeitet werden (vgl. z. B. (G⁹a): R⁸=Me, R¹², R¹³=H: DE 1900202; R⁸=Me, R¹²=H, R¹³=Ph: T. Parkkari et al., Bioorg. Med. Chem. Lett. (2004), 14, 3231-3234).
Die Verwendung der Reste (G¹⁰) und deren Herstellung ist hinreichend bekannt (vgl. z. B. R¹⁶, R¹⁷=Me: S. Julia et al., Bull. Soc. Chim. Franc. (1966), 11, 3499-507; R¹⁶, R¹⁷=Cl: DE 2439177; R¹⁶=Cl, R¹⁷=CF₃: DE 2802962; R¹⁶, R¹⁷=Br: M. Elliott et al., Pest. Sci. (1975), 6, 537-42).
Die Verwendung der Reste (G¹¹) und deren Herstellung sind beispielsweise aus WO 2002/059078 A1 bekannt.
Die Verwendung von Chlormethoximinoessigsäuremethylester (G-VIII), worin LG=Cl und R¹⁸=Me (vgl. WO 98/12179, WO 99/67209), zur Darstellung der Reste (C¹¹) ist bekannt (vgl. z. B. die Reaktion von 4-Nitro-1*H*-imidazol mit Chlormethoximinoessigsäuremethylester und die anschließende Esterhydrolyse in G. Elitropi et al. J. Het. Chem. (1979), 16, 1545-1550).
Zur Darstellung der Reste G¹³ können Verbindungen der Formel (G-X), worin beispielsweise X, Y=S und R¹¹=Me bedeuten, nach Deprotonierung am Kohlenstoffatom mit Kohlendioxid umgesetzt werden (E. Capito et al., Tetrahedron: Asymmetry (2003), 16, 3232-3240). Alternativ können die Reste G¹³, worin beispielsweise X,Y=O und R¹¹=Me auch aus geeigneten alpha-Ketocarbonsäuren (G-XI) und Dialkoholen gebildet werden (vgl. D. J. Wardrop et al., Org. Lett. (2001), 3, 2261-2264; D. J. Wardrop et al., Tetrahedron: Asymmetry (2003),14,929-940).
Zur Darstellung der Reste (G¹⁴a,b) können beispielsweise geeignete alpha-Methylencarbonsäureester (G-XII), wie Methylmethacrylat (R⁶=Me) mit Aminoverbindungen gemäß einer aza-Michaeladdition zu Verbindungen der Formel (G-XIII) umgesetzt werden (vgl. B. C. Ranu et al., Tetrahedron Lett. (2006), 48, 141-143; K. R. Reddy et al., Synlett (2006), 2246-2250) oder 2-Formyl-carbonsäureester der Formel (G-XIV), beispielsweise 2-Formyl-2-methyl-propionsäwemethylester (R⁶,R⁷=Me; US 2005/239838) mittels reduktiver Aminierung in Verbindungen der Formel (G-XV) überführt werden. Anschließend erfolgt die Hydrolyse der Estergruppe nach literaturbekannter Verfahrensweise.
Gemäß Schema IV lassen sich substituierte 1*H*-1,2,4-Triazol-3-essigsäureester beispielsweise auch aus entsprechend substituierten Estern von (Triphenylphosphoranyliden)alkansäuren in Gegenwart von Alkyliso-cyanatocarbonaten und Arylhydrazinen herstellen (Capuano et al., Liebigs Annalen der Chemie (1985), 12, 2305-2312).

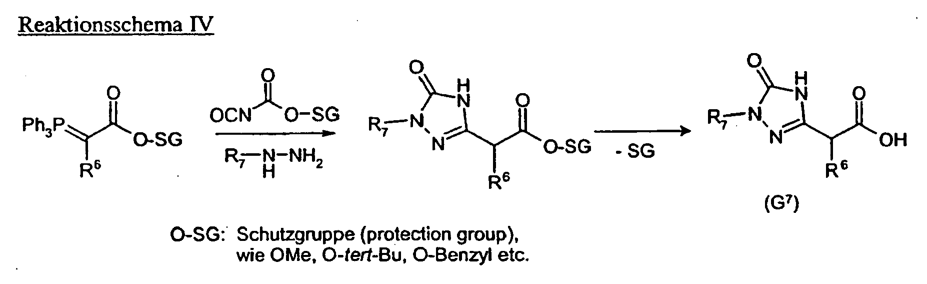

Weiterhin als Ausgangsstoffe verwendete Carbonsäuren der Formel (V), wenn B für gegebenenfalls substituiertes Amino steht, sind natürliche oder synthetische Aminosäuren. Diese können, falls chiral, in der *(S)*- oder *(R)*-Form (bzw. L- oder D-Form) vorliegen.

Beispielsweise seien die folgenden natürliche oder synthetische Aminosäuren genannt: Aad, Abu, j-Abu, Abz, 2Abz, ε-Aca, Acp, Adpd, Ahb, Aib, β-Aib, Ala, β-Ala, Δ-Ala, Alg, All, Ama, Amt, Ape, Apm, Apr, Arg, Asn, Asp, Asu, Aze, Azi, Bai, Bph, Can, Cit, Cys, (Cys)₂, Cyta, Daad, Dab, Dadd, Dap, Dapm, Dasu, Djen, Dpa, Dtc, Fel, Gln, Glu, Gly, Guv, hAla, hArg, hCys, hGln, hGlu, His, hIle, hLeu, hLys, hMet, hPhe, Pro, hSer, hThr, hTrp, hTyr, HyI, Hyp, 3Hyp, Ile, Ise, Iva, Kyn, Lant, Lcn, Leu, Lsg, Lys, β-Lys, Δ-Lys, Met, Mim, Min, nArg, Nle, Nva, Oly, Orn, Pan, Pec, Pen, Phe, Phg, Pic, Pro, Δ-Pro, Pse, Pya, Pyr, Pza, Qin, Ros, Sar, Sec, Sem, Ser, Thi, β-Thi, Thr, Thy, Thx, Tia, Tle, Tly, Trp, Trta, Tyr, Val, Nal, Tbg, Npg, Chg, Thia (vgl. z. B. Houben-Weyl, Methoden der Organischen Chemie, Band XV/1 und 2, Stuttgart, 1974).

Die natürlichen oder synthetischen Aminosäuren können z. T. kommerziell oder nach literaturbekannten Methoden erhalten werden (vgl. z. B. N-Methylaminosäuren: R. Bowmann et al., J. Chem. Soc. (1950), S. 1346; J. R. McDermott et al., Can. J. Chem. (1973) 51, S. 1915; H. Wurziger et al., Kontakte (Merck, Darmstadt) (1987) 3, S. 8).

Im Allgemeinen ist es vorteilhaft das erfindungsgemäße Herstellungsverfahren in Gegenwart von Verdünnungsmitteln durchzuführen.

Verdünnungsmittel werden vorteilhafterweise in einer solchen Menge eingesetzt, dass das Reaktionsgemisch während des ganzen Verfahrens gut rührbar bleibt. Geeignete Verdünnungsmittel sind dem Fachmann bekannt. Als Verdünnungsmittel zur Durchführung des erfindungsgemäßen Verfahrens kommen insbesondere inerte organische Lösungsmittel allein oder als Gemische in Frage.

Als Beispiele sind zu nennen: Halogenkohlenwasserstoffe, insbesondere Chlorkohlenwasserstoffe, wie Tetraethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol; Alkohole wie Methanol, Ethanol, Isopropanol, Butanol; Ether wie Ethylpropylether, Methyl-tert-butylether, n-Butylether, Anisol, Phenetol, Cyclohexylmethylether, Dimethylether, Diethylether, Dipropylether, Diisopropylether, Di-n-butylether, Diisobutylether, Diisoamylether, Ethylenglycoldimethylether, Tetrahydrofuran, Dioxan, Dichlordiethylether und Polyether des Ethylenoxids und/oder Propylenoxids; Amine wie Trimethyl-, Triethyl-, Tripropyl-, Tributylamin, N-Methylmorpholin, Pyridin und Tetramethylendiamin; Nitrokohlenwasserstoffe wie Nitromethan, Nitroethan, Nitropropan, Nitrobenzol, Chlornitrobenzol, o-Nitrotoluol; Nitrile wie Acetonitril, Propionitril, Butyronitril, Isobutyronitril, Benzonitril, m-Chlorbenzonitril sowie Verbindungen wie Tetrahydrothiophendioxid und Dimethylsulfoxid, Tetramethylensulfoxid, Dipropylsulfoxid, Benzylmethylsulfoxid, Diisobutylsulfoxid, Dibutylsulfoxid, Diisoamylsulfoxid; Sulfone wie dimethyl-, Diethyl-, Dipropyl-, Dibutyl-, Diphenyl-, Dihexyl-, Methylethyl-, Ethylpropyl-, Ethylisobutyl- und Pentamethylensulfon; aliphatische, cycloaliphatische oder aromatische Kohlenwasserstoffe wie Pentan, Hexan, Heptan, Oktan, Nonan und technische Kohlenwasserstoffe; beispielsweise sogenannte White Spirits mit Komponenten mit Siedepunkten im Bereich beispielsweise von 40°C bis 250 °C, Cymol, Benzinfraktionen innerhalb eines siedeintervalles von 70 °C bis 190 °C, Cyclohexan, Methylcyclohexan, Petrolether, Ligroin, Octan, Benzol, Toluol, Chlorbenzol, Brombenzol, Nitrobenzol, Xylol; Ester wie Methyl-, Ethyl-, Butyl-, Isobutylacetat, sowie Dimethyl-, Dibutyl-, Ethylencarbonat; Amide wie Hexamethylenphosphorsäuretriamid, Formamid, *N*-Methyl-formamid, N,N-Dimethyl-formamid, *N,N*-Dipropyl-formamid, *N,N*-Dibutylformamid, *N*-Methyl-pyrrolidin, *N*-Methyl-caprolactam, 1,3-Dimethyl-3,4,5,6-tetrahydro-2(1H)-pyrimidin, Octylpyrrolidon, Octylcaprolactany 1,3-Dimethyl-2-imidazolindion, N-Formyl-piperidin, *N,N*'-1,4-Diformyl-piperazin; Ketone wie Aceton, Acetophenon, Methylethylketon, Methylbutylketon.

Bevorzugte Verdünnungsmittel zur Durchführung des zweiten Reaktionsschrittes des erfindungsgemäßen Herstellungsverfahrens sind Methanol, Ethanol, Isopropanol oder Butanol, insbesondere Methanol.

Bevorzugte Verdünnungsmittel zur Durchführung des dritten Reaktionsschrittes des erfindungsgemäßen Herstellungsverfahrens sind Halogenkohlenwasserstoffe, insbesondere Chlorkohlenwasserstoffe, wie Tetraethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol oder Trichlorbenzol, besonders bevorzugt Dichlorpropan, Methylenchlorid, Dichlorbutan oder Chloroform.

Als basische Reaktionshilfsmittel im erfindungsgemäßen Herstellungsverfahren können alle geeigneten Säurebindemittel wie beispielsweise Amine, insbesondere tertiäre Amine sowie Alkalimetall- und Erdalkalimetallverbindungen eingesetzt werden.

Als Beispiele sind zu nennen die Hydroxide, Hydride, Oxide und Carbonate von Lithium, Natrium, Kalium, Magnesium, Calcium und/oder Barium, ferner weitere basische Verbindungen wie Amidinbasen oder Guanidinbasen wie 7-Methyl-1,5,7-triaza-bicyclo[4.4.0]dec-5-en (MTBD); Diazabicyclo[4.3.0]nonen (DBN), Diazabicyclo [2.2.2]octan (DABCO), 1,8-Diazabicyclo[5.4.0]undecen (DBU), Cyclohexyltetrabutyl-guanidin (CyTBG), Cyclohexyltetramethylguanidin (CyTMG), N,N,N,N-Tetramethyl-1,8-naphthalindiamin, Pentamethylpiperidin, tertiäre Amine wie Triethylamin, Trimethylamin, Tribenzylamin, Triisopropylamin, Tributylamin, Tricyclohexylamin, Triamylamin, Trihexylamin, N,N-Dimethylanilin, N,N-Dimethyl-toluidin, N,N-Dimethyl-p-aminopyridin, N-Methyl-pyrrolidin, N-Methyl-piperidin, N-Methyl-imidazol, N-Methyl-pyrazol, N-Methyl-morpholin, N-Methylhexamethylendiamin, Pyridin, 4-Pyrrolidinopyridin, 4-Dimethylamino-pyridin, Chinolin, α-Picolin, β-Picolin, Isochinolin, Pyrimidin, Acridin, N,N,N',N'-Tetramethylendiamin, N,N',N'-Tetraethylendiamin, Chinoxalin, N-Propyl-diisopropylamin, N-Ethyl-diisopropylamin, N,N'-Dimethyl-cyclohexylamin, 2,6-Lutidin, 2,4-Lutidin oder Triethyldiamin.

Vorzugsweise finden tertiäre Amine wie Triethylamin, Trimethylamin, Triisopropylamin, Tributylamin, Tricyclohexylamin, N,N-Dimethyl-p-aminopyridin, N-Methyl-pyrrolidin, N-Methylpiperidin oder N-Methyl-imidazol, besonders bevorzugt finden Triethylamin und N,N-Dimethyl-p-aminopyridin Verwendung.

Die erfindungsgemäße Umsetzung von Verbindungen der Formel (IV) wird durchgeführt, indem man in dem dritten Reaktionsschritt die in 5-Position geschützten Verbindungen der Formel (IV) in Gegenwart eines basischen Reaktionshilfsmittels, beispielsweise Triethylamin oder N,N-Dimethyl-p-aminopyridin, in einem der angegebenen Verdünnungsmittel mit einer aktivierten Carbonsäure als Verbindung der Formel (V) gegebenenfalls in Gegenwart eines Kupplungsreagens umsetzt.

Als Kupplungsagenzien zur Durchführung des Herstellungsverfahrens fmden alle, die zur Herstellung einer Ester- oder Amidbindung geeignet sind (vgl. z. B. Houben-Weyl, Methoden der Organischen Chemie, Band 15/2; Bodansky et al., Peptide Synthesis 2nd ed. (Wiley & Sons, New York 1976) oder Gross, Meienhofer, The Peptides: Analysis, Synthesis, Biology (Academic Press, New York 1979)) Verwendung. Vorzugsweise werden folgende Methoden herangezogen: Aktivestermethode mit Pentachlorphenol (Pcp) oder Pentafluorphenol (Pfp), N-Hydroxysuccinimid (HOSu), N-Hydroxy5-norbornen-2,3-dicarboxamid (HONB), 1-Hydroxy-benzotriazol (HOBt) oder 3-Hydroxy-4-oxo-3,4-dihydro-1,2,3-benzotriazin als Alkoholkomponente, Kupplung mit Carbodiimiden wie Dicyclohexylcarbodiimid (DCCI), nach dem DCC-Additiv-Verfahren, oder mit n-Propanphosphonsäureanhydrid (PPA) und Gemischt-Anhydrid-Methode mit Pivaloylchlorid, Ethyl-(EEDQ) und Isobutylchlorformiat) (IIDQ) oder Kupplung mit Phosphoniumreagenzien, wie Benzotriazol-1-yl-oxy-tris(dimethylamino-phos-phonium)-hexafluorophosphat (BOP), Bis(2-oxo-3-oxazolidinyl)-phosphonium-säurechlorid (BOP-Cl), Benzotriazol-1-yl-trispyrrolidino-phosphonium-hexafluoro-phosphat (PyBOP^{®}), Bromo-tris-pyrrolidino-phosphonium-hexafluoro-phosphat (PyBroP^{®}) oder mit Phosphonsäurereagenzien, wie Cyanphosphon-säurediethylester (DEPC) und Diphenylphosphorylazid (DPPA), Uroniumreagenzien, wie 2-(1H-Benzotriazol-1-yl)-1,1,3,3-tetramethyluronium-tetrafluoroborat (TNTU), 2-(2-Oxo-1(2H)-pyridyl)-1,1,3,3-bis-pentamethylentetramethyluronium-tetrafluoroborat (TOPPipU), O-(N-Succinimidyl-1,1,3,3-tetramethyluronium-tetrafluoroborat (TSTU) oder wie 2-(1H-Benzo-triazol-1-yl)-1,1,3,3-tetramethyluronium-hexafluorophosphat (HBTU) oder Reagenzien vom Onium Typ, wie beispielsweise 1-Ethyl-2-fluoropyridiniumtetrafluoroborat (FEP).

Ein bevorzugtes Aktivierungsmittel für die erfingdungsgemäß verwendbaren Carbonsäuren ist beispielsweise N-(3-Dimethylaminopropyl)-N'-ethylcarbodiimid.

Die Reaktionsdauer im erfindungsgemäßen Herstellungsverfahren kann je nach Reaktionsschritt von 5 Minuten bis 48 Stunden betragen. Im erfindungsgemäßen Herstellungsverfahren erfolgen die jeweiligen Umsetzungen bei Temperaturen zwischen -100 °C und +200 °C, bevorzugt zwischen - 50 °C und 150 °C, besonders bevorzugt bei Raumtemperatur. Die erfindungsgemäßen Reaktionsschritte des Herstellungsverfahrens können grundsätzlich unter Normaldruck, vorzugsweise bei Drücken bis zu 15 bar und gegebenenfalls unter Schutzgasatmosphäre (beispielsweise Stickstoff, Helium oder Argon) durchgeführt werden.

Zur Durchführung des dritten Reaktionsschrittes werden pro Mol Verbindung der Formel (IV) 0,5 bis 4,0 Mol, vorzugsweise 0,7 bis 3,0 Mol, besonders bevorzugt 2,0 bis 3,0 Mol an Verbindung der Formel (V), wie beispielsweise aktivierter Carbonsäure eingesetzt.

Nach vollendeter Umsetzung wird der gesamte Reaktionsansatz eingeengt. Die nach Aufarbeitung anfallenden Produkte der Formel (VI) lassen sich in üblicher Weise durch beispielsweise Umkristallisieren, Vakuumdestillation oder Säulenchromatographie reinigen, können aber auch ohne Reinigung weiter umgesetzt werden.

Der vierte Reaktionsschritt im erfindungsgemäßen Herstellungsverfahren wird bevorzugt in Gegenwart von Benzolsulfonsäure oder para-Toluolsulfonsäure. Die Durchführung in Gegenwart von para-Toluolsulfonsäure ist besonders bevorzugt.

Zur Durchführung des vierten Reaktionschrittes des erfindungsgemäßen Herstellungsverfahrens setzt man pro Mol Verbindung der Formel (VI) im allgemeinen 0,1 bis 4,0 Mol, vorzugsweise 0,1 bis 1,0 Mol ein. Besonders bevorzugt wird 0,1 bis 1,0 Mol an *para*-Toluolsulfonsäure eingesetzt.

Nach vollendeter Umsetzung wird der gesamte Reaktionsansatz eingeengt. Die nach Aufarbeitung anfallenden Produkte der Formel (I) lassen sich in üblicher Weise beispielsweise durch Umkristallisieren, Vakuumdestillation oder Säulenchromatographie, insbesondere präparativer HPLC reinigen.

Desweiteren wurde gefunden, dass die erfindungsgemäßen Avermectinderivate Formel (I), worin R⁴ für einen Rest aus der Gruppe (G⁷) oder (G⁸) steht, in welchem die Gruppierung B die weiter oben genannte Bedeutung hat und B die Gruppierung H-N entweder als Teil des Ringes oder als Teil eines Substituenten enthält, erhalten werden, indem Verbindungen der Formel (I), worin R⁴ für einen Rest aus der Gruppe (G⁷) oder (G⁸) steht und in dem B eine Stickstoff enthaltende Gruppierung SG-N, entweder als Teil eines Ringes oder Substituenten, enthält und wobei SG für eine geeignete Schutzgruppe steht, in einem fünften Reaktionsschritt unter den Reaktionsbedingungen einer Schutzgruppendeblockierung gegebenenfalls in Gegenwart eines Verdünnungsmittels und gegebenenfalls in Gegenwart eines geeigneten sauren oder basischen Reaktionshilfsmittels umgesetzt werden (Reaktionsschema V).

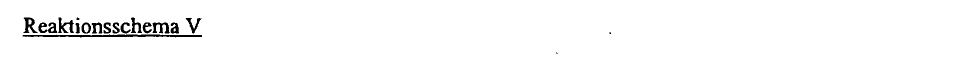

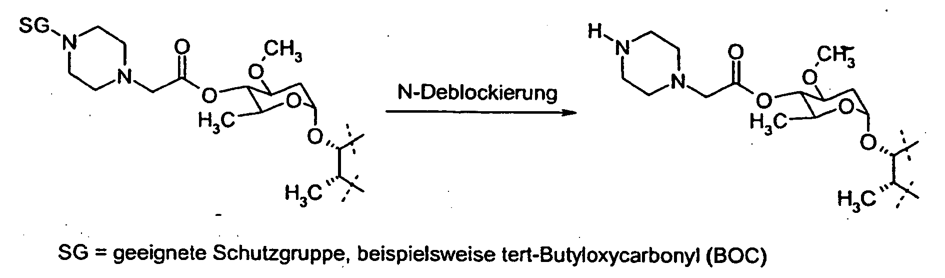

In diesem Fall können zur Herstellung von Verbindungen der Formel (I) beispielsweise als geeignete Schutzgruppen für Aminogruppen substituierte Carbamate, Amide, N-Alkylamine, N-Arylamine, Iminderivate, Enaminderivate, N-Sulfenylderivate, N-Sulfonylderivate oder N-Diarylphosphinylderivate verwendet werden (vgl. Greene T. W., Wuts P. G. W. in Protective Groups in Organic Synthesis; John Wiley & Sons, Inc. 1999, "Protection for the Amino Group").

Bevorzugt werden Schutzgruppen des Carbamat-Typs verwendet.

Zur Schutzgruppendeblockierung können geeignete saure oder basische Reaktionshilfsmittel nach literaturbekannter Verfahrensweise verwendet werden. Bei Verwendung von Schutzgruppen für Aminogruppen des Carbamat-Typs werden bevorzugt saure Reaktionshilfsmittel verwendet. Bei Verwendung der t-Butylcarbamat (BOC) -Schutzgruppe werden beispielsweise Mischungen von Mineralsäuren wie Salzsäure, Bromwasserstoffsäure, Salpetersäure, Schwefelsäure, Phosphorsäure oder organischen Säuren wie Benzoesäure, Ameisensäure, Essigsäure, Trifluoressigsäure, Methansulfonsäure, Benzolsulfonsäure oder Toluolsulfonsäure und einem geeigneten Verdünnungsmittel wie Wasser und/oder einem organischen Lösungsmittel wie Tetrahydrofuran, Dioxan, Dichlormethan, Chloroform, Essigester, Ethanol oder Methanol verwendet. Bevorzugt sind Mischungen von Salzsäure oder Essigsäure mit Wasser und/oder einem organischen Lösungsmittel wie Essigester.

Die erfindungsgemäßen Avermectinderivate können in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Die erfindungsgemäßen Avermectinderivate eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie werden vorzugsweise als Pflanzenschutzmittel eingesetzt. Sie sind gegen normal sensible und resistente Arten sowie gegen alle oder einzelne Entwicklungsstadien wirksam. Zu den oben erwähnten Schädlingen gehören unter anderem:

Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp..

Aus der Klasse der Gastropoda z.B. Arion spp., Biomphalaria spp., Bulinus spp., Deroceras spp., Galba spp., Lymnaea spp., Oncomelania spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Reticulitermes spp., Odontotermes spp..

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp..

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gegaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp..

Die erfindungsgemäßen Avermectinderivate können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im Allgemeinen zwischen 0,01 und 98 Gew.% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen. Besonders günstige Mischpartner sind z.B. die folgenden:
**Fungizide:**
   Inhibitoren der Nucleinsäure Synthese
      Benalaxyl, Benalaxyl-M, Bupirimat, Chiralaxyl, Clozylacon, Dimethirimol, Ethirimol, Furalaxyl, Hymexazol, Metalaxyl, Metalaxyl-M, Ofurace, Oxadixyl, Oxolinsäure
   Inhibitoren der Mitose und Zellteilung
      Benomyl, Carbendazim, Diethofencarb, Fuberidazole, Pencycuron, Thiabendazol, Thiophanatmethyl, Zoxamid
   Inhibitoren der Atmungskette Komplex I
      Diflumetorim
   Inhibitoren der Atmungskette Komplex II
      Boscalid, Carboxin, Fenfuram, Flutolanil, Furametpyr, Mepronil, Oxycarboxin, Penthiopyrad, Thifluzamid
   Inhibitoren der Atmungskette Komplex III
      Azoxystrobin, Cyazofamid, Dimoxystrobin, Enestrobin, Famoxadon, Fenamidon, Fluoxastrobin, Kresoximmethyl, Metominostrobin, Orysastrobin, Pyraclostrobin, Picoxystrobin, Trifloxystrobin
   Entkoppler
      Dinocap, Fluazinam
   Inhibitoren der ATP Produktion
      Fentinacetat, Fentinchlorid, Fentinhydroxid, Silthiofam
   Inhibitoren der Aminosäure- und Proteinbiosynthese
      Andoprim, Blasticidin-S, Cyprodinil, Kasugamycin, Kasugamycinhydrochlorid Hydrat, Mepanipyrim, Pyrimethanil
   Inhibitoren der Signal-Transduktion Fenpiclonil, Fludioxonil, Quinoxyfen
   Inhibitoren der Fett- und Membran Synthese
      Chlozolinat, Iprodion, Procymidon, Vinclozolin
      Ampropylfos, Kalium-Ampropylfos, Edifenphos, Iprobenfos (IBP), Isoprothiolan, Pyrazophos
      Tolclofos-methyl, Biphenyl
      lodocarb, Propamocarb, Propamocarb hydrochlorid
   Inhibitoren der Ergosterol Biosynthese
      Fenhexamid,
      Azaconazol, Bitertanol, Bromuronazol, Cyproconazol, Diclobutrazol, Difenoconazol, Diniconazol, Diniconazol-M, Epoxiconazol, Etaconazol, Fenbuconazol, Fluquinconazol, Flusilazol, Flutriafol, Furconazol, Furconazol-cis, Hexaconazol, Imibenconazol, Ipconazol, Metconazol, Myclobutanil, Paclobutrazol, Penconazol, Propiconazol, Prothioconazol, Simeconazol, Tebuconazol, Tetraconazol, Triadimefon, Triadimenol, Triticonazol, Uniconazol, Voriconazol, Imazalil, Imazalilsulfat, Oxpoconazol, Fenarimol, Flurprimidol, Nuarimol, Pyrifenox, Triforin, Pefurazoat, Prochloraz, Triflumizol, Viniconazol,
      Aldimorph, Dodemorph, Dodemorphacetat, Fenpropimorph, Tridemorph, Fenpropidin, Spiroxamin,
      Naftifin, Pyributicarb, Terbinafin
   Inhibitoren der Zellwand Synthese
      Benthiavalicarb, Bialaphos, Dimethomorph, Flumorph, Iprovalicarb, Polyoxins, Polyoxorim, Validamycin A
   Inhibitoren der Melanin Biosynthese
      Capropamid, Diclocymet, Fenoxanil, Phtalid, Pyroquilon, Tricyclazol
   Resistenzinduktion Aciöenzolar-S-methyl, Probenazol, Tiadinil
   Multisite
      Captafol, Captan, Chlorothalonil, Kupfersalze wie: Kupferhydroxid, Kupfemaphthenat, Kupferoxychlorid, Kupfersulfat, Kupferoxid, Oxin-Kupfer und Bordeaux Mischung, Dichlofluanid, Dithianon, Dodin, Dodin freie Base, Ferbam, Folpet, Fluorofolpet, Guazatin, Guazatinacetat, Iminoctadin, Iminoctadinalbesilat, Iminoctadintriacetat, Mankupfer, Mancozeb, Maneb, Metiram, Metiram Zink, Propineb, Schwefel und Schwefelpräparate enthaltend Calciumpolysulphid, Thiram, Tolylfluanid, Zineb, Ziram
   Unbekannter Mechanismus
      Amibromdol, Benthiazol, Bethoxazin, Capsimycin, Carvon, Chinomethionat, Chloropicrin, Cufraneb, Cyflufenamid, Cymoxanil, Dazomet, Debacarb, Diclomezine, Dichlorophen, Dicloran, Difenzoquat, Difenzoquat Methylsulphat, Diphenylamin, Ethaboxam, Ferimzon, flumetover, Flusulfamid, Fluopicolid, Fluoroimid, Hexachlorobenzol, 8-Hydroxychinolinsulfat, Irumamycin, Methasulphocarb, Metrafenon, Methyl Isothiocyanat, Mildiomycin, Natamycin, Nickel dimethyldithiocarbamat, Nitrothal-isopropyl, Octhilinon, Oxamocarb, Oxyfenthiin, Pentachlorophenol und Salze, 2-Phenylphenol und Salze, Piperalin, Propanosin Natrium, Proquinazid, Pyrrolnitrin, Quintozen, Tecloftalam, Tecnazen, Triazoxid, Trichlamid, Zarilamid und 2,3,5,6-Tetrachlor-4-(methylsulfonyl)-pyridin, N-(4-Chlor-2-nitrophenyl)-N-ethyl-4-methyl-benzenesutfonamid, 2-Amino-4-methyl-N-phenyl-5-thiazolecarboxamid, 2-Chlor-N-(2,3-dihydro-1, 1,3-trimethyl-1 H-inden-4-yl)-3-pyridincarboxamid, 3-[5-(4-Chlorphenyl)-2,3-dimethylisoxazolidin-3-yl]pyridin, cis-1-(4-Chlorphenyl)-2-(1H-1,2,4-triazol-1-yl)-cycloheptanol, 2,4-Dihydro-5-methoxy-2-methyl-4-[[[[1-[3-(trifluoromethyl)-phenyl]-ethylidcnl-amino]-oxy]-methyl]-phenyl]-314-1,2,3-triazol-3-on (185336-79-2), Methyl 1-(2,3-dihydm-2,2-dimethyl-1H-inden-1-yl)-1H-imidazole-5-arboxylat, 3,4,5-Trichlor-2,6-pyridindicarbonitril, Methyl 2-[[[cyclopropyl[(4-methoxyphenyl) imino]methyl]thio]methyl]-.alpha.-(methoxymethylen)- benzacetat, 4-Chlor-alpha-propinyloxy-N-[2-[3-methoxy-4-(2-propinyloxy)phenyl]ethyl]-benzacetamide, (2S)-N-[2-[4-[[3-(4-chlorophenyl)-2-propinyl]oxy]-3-methoxyphenyl]ethyl]- 3-methyl-2-[(methylsulfonyl)amino]-butanamid, 5-Chlor-7-(4-methylpiperidin-1-yl)-6-(2,4,6-trifluorophenyl)[1,2,4]triazolo[1,5-a]pyämidin, 5-Chlor-6-(2,4,6-trifluorophenyl)-N-[(IR)-1,2,2-trimethylpropyl][1,2,4]triazolo[1,5-a]pyrimidin-7-amin, 5-Chlor-N-[(1R)-1,2-dimethylpropyl]-6-(2,4,6-trifluorophenyl) [1,2,4]tciazolo[1,5-a]pyrimidin-7-amine, N-[1-(5-Brom-3-chloropyridin-2-yl)ethyl]-2,4-dichloronicotinamid, N-(5-Brom-3-chlorpyridin-2-yl)methyl-2,4-dicldornicotnamid, 2-Butoxy-6-iod-3-propyl-benzopyranon-4-on, N-{(Z)-[(cyclopropylmethoxy) imino][6-(difluormethoxy)-2,3-difluorphenyl]methyl}-2-benzacetamid, N-(3-Ethyl-3,5,5-trimethyl-cyclohexyl)-3-formylamino-2-hydroxy-benzamid, 2-[[[[1-[3-(1-Fluor-2-phenylethyl)oxy] phenyl] ethyliden]amino]oxy]methyl]-alpha-(methoxyimino)-N-methyl-alphaE-benzacetamid, N-{2-[3-Chlor-5-(trifluormethyl)pyridin-2-yl]ethyl}-2-(trifluoromethyl)benzamid, N-(3',4'-dichior-5-fluorbiphenyl-2-yl)-3-(difluormethyl)-1-methyl-1H-pyrazol-4-carboxamid, N-(6-Methoxy-3-pyridinyl)-cyclopropan carboxamid, 1-[(4-Methoxyphenoxy)methyl]-2,2-dimethylpropyl-1H-imidazol-1- carbonsäure, O-[1-[(4-Methoxyphenoxy)methyl]-2,2-dimethylpropyl]-1H-imidazol- 1- carbothioic acid, 2-(2-{[6-(3-Chlor-2-methylphenoxy)-5-fluorpyrimidin-4-yl]oxy}phenyl)-2-(methoxyimino)-N-methylacetamid
**Bakterizide:**
   Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Acetylcholinesterase (AChE) Inhibitoren
      Carbamate,
      zum Beispiel Alanycarb, Aldicarb, Aldoxycarb, Allyxycarb, Aminocarb, Bendiocarb, Benfuracarb, Bufencarb, Butacarb, Butocarboxim, Butoxycarboxim, Carbaryl, Carbofuran, Carbosulfan, Cloethocarb, Dimetilan, Ethiofencarb, Fenobucarb, Fenothiocarb, Formetanate, Furathiocarb, Isoprocarb, Metam-sodium, Methiocarb, Methomyl, Metolcarb, Oxamyl, Pirimicarb, Promecarb, Propoxur, Thiodicarb, Thiofanox, Trimethacarb, XMC, Xylylcarb, Triazamate
      Organophosphate,
      zum Beispiel Acephate, Azamethiphos, Azinphos (-methyl, -ethyl), Bromophos-ethyl, Bromfenvinfos (-methyl), Butathiofos, Cadusafos, Carbophenothion, Chlorethoxyfos, Chlorfenvinphos, Chlormephos, Chlorpyrifos (-methyl/-ethyl), Coumaphos, Cyanofenphos, Cyanophos, Chlorfenvinphos, Demeton-S-methyl, Demeton-S-methylsulphon, Dialifos, Diazinon, Dichlofenthion, Dichlorvos/DDVP, Dicrotophos, Dimethoate, Dimethylvinphos, Dioxabenzofos, Disulfoton, EPN, Ethion, Ethoprophos, Etrimfos, Famphur, Fenanüphos, Fenitrothion, Fensulfothion, Fenthion, Flupyrazofos, Fonofos, Formothion, Fosmethilan, Fosthiazate, Heptenophos, Iodofenphos, lprobenfos, Isazofos, Isofenphos, Isopropyl O-salicylate, Isoxathion, Malathion, Mecarbam, Methacrifos, Methamidophos, Methidathion, Mevinphos, Monocrotophos, Naled, Omethoate, Oxydemeton-methyl, Parathion (-methyl/ -ethyl), Phenthoate, Phorate, Phosalone, Phosmet, Phosphamidon, Phosphocarb, Phoxim, Pirimiphos (-methyl/-ethyl), Profenofos, Propaphos, Propetamphos, Prothiofos, Prothoate,
      Pyraclofos, Pyridaphenthion, Pyridathion, Quinalphos, Sebufos, Sulfotep, Sulprofos, Tebupirimfos, Temephos, Terbufos, Tetrachlorvinphos, Thiometon, Triazophos, Triclorfon, Vamidothion
   Natrium-Kanal-Modulatoren / Spannungsabhängige Natrium-Kanal-Blocker
      Pyrethroide,
      zum Beispiel Acrinathrin, Allethrin (d-cis-trans, d-trans), Beta-Cyfluthrin, Bifenthrin, Bioallethrin. Bioallethrin-S-cyclopentyl-isomer, Bioethanomethrin, Biopermethrin, Bioresmethrin, Chlovaporthrin, Cis-Cypermethrin, Cis-Resmethrin, Cis-Permethrin, Clocythrin, Cycloprothrin, Cyfluthrin, Cyhalothrin, Cypermethrin (alpha-, beta-, theta-, zeta-), Cyphenothrin, Deltamethrin, Empenthrin (1R-isomer), Esfenvalerate, Etofenprox, Fenfluthrin, Fenpropathrin, Fenpyrithrin, Fenvalerate, Flubrocythrinate, Flucythrinate, Flufenprox, Flumethrin, Fluvalinate, Fubfenprox, Gamma-Cyhalothrin, Imiprothrin, Kadethrin, Lambda- Cyhalothrin, Metofluthrin, Permethrin (cis-, trans-), Phenothrin (1R-trans isomer), Prallethrin, Profluthrin, Protrifenbute, Pyresmethrin, Resmethrin, RU 15525, Silafluofen, Tau-Fluvalinate, Tefluthrin, Terallethrin, Tetramethrin (-1R- isomer), Tralomethrin, Transfluthrin, ZXI 8901, Pyrethrins (pyrethrum)
      DDT
      Oxadiazine,
      zum Beispiel Indoxacarb

      Semicarbazon,
      zum Beispiel Metaflumizon (BAS3201)
   Acetylcholin-Rezeptor-Agonisten/-Antagonisten
      Chloronicotinyle,
      zum Beispiel Acetamiprid, Clothianidin, Dinotefuran, Imidacloprid, Nitenpyram, Nithiazine, Thiacloprid, Thiamethoxam, AKD-1022, Imidaclotiz
      Nicotine, Bensultap, Cartap
   Acetylcholin-Rezeptor-Modulatoren
      Spinosyne,
      zum Beispiel Spinosad und Spinetoram
   GABA-gesteuerte Chlorid-Kanal-Antagonisten
      Organochlorine,
      zum Beispiel Camphechlor, Chlordane, Endosulfan, Gamma-HCH, HCH, Heptachlor, Lindane, Methoxychlor
      Fiprole,
      zum Beispiel Acetoprole, Ethiprole, Fipronil, Pyrafluprole, Pyriprole, Vaniliprole
   Chlorid-Kanal-Aktivatoren
      Mectine,
      zum Beispiel Abamectin, Emamectin, Emamectin-benzoate, Ivermectin, Lepimectin, Milbemycin, Latidectin, Selamectin, Doramectin, Eprinomectin, Moxidectin
   Latrophilinrezeptor-Agonisten
      Depsipeptide, wie zum Beispiel zykl. Depsipeptide,
      zum Beispiel Emodepsid
   Juvenilhormon-Mimetika,
      zum Beispiel Diofenolan, Epofenonane, Fenoxycarb, Hydroprene, Kinoprene, Methoprene, Pyriproxifen, Triprene
   Ecdysonagonisten/disruptoren
      Diacylhydrazine,
      zum Beispiel Chromafenozide, Halofenozide, Methoxyfenozide, Tebufenozide
   Inhibitoren der Chitinbiosynthese
      Benzoylharnstoffe,
      zum Beispiel Bistrifluron, Chlofluazuron, Diflubenzuron, Fluazuron, Flucycloxuron, Flufenöxuron, Hexaflumuron, Lufenuron, Novaluron, Noviflumuron, Penfluron, Teflubenzuron, Triflumuron
      Buprofezin
      Cyromazine
   Inhibitoren der oxidativen Phosphorylierung, ATP-Disruptoren
      Diafenthiuron
      Organozinnverbindungen,
      zum Beispiel Azocyclotin, Cyhexatin, Fenbutatin-oxide
   Entkoppler der oxidativen Phoshorylierung durch Unterbrechung des H-Protongradienten
      Pyrrole,
      zum Beispiel Chlorfenapyr
      Dinitrophenole,
      zum Beispiel Binapacyrl, Dinobuton, Dinocap, DNOC
   Seite-I-Elektronentransportinhibitoren
      METI's,
      zum Beispiel Fenazaquin, Fenpyroximate, Pyrimidifen, Pyridaben, Tebufenpyrad, Tolfenpyrad
      Hydramethylnon
      Dicofol
   Seite-II-Elektronentransportinhibitoren
      Rotenone
   Seite-III-Elektronentransportinhibitoren
      Acequinocyl, Fluacrypyrim
   Mikrobielle Disruptoren der Insektendarmmembran
      Bacillus thuringiensis-Stämme
   Inhibitoren der Fettsynthese
      Tetronsäuren,
      zum Beispiel Spirodiclofen, Spiromesifen,
      Tetramsäuren,

      zum Beispiel Spirotetramat, cis-3-(2,5-dimethylphenyl)-4-hydroxy-8-methoxy-1-azaspiro[4.5]dec-3-en-2-on
      Carboxamide,
      zum Beispiel Flonicamid
      Oktopaminerge Agonisten,
      zum Beispiel Amitraz
   Inhibitoren der Magnesium-stimulierten ATPase,
      Propargite
      Nereistoxin-Analoge,
      zum Beispiel Thiocyclam hydrogen oxalate, Thiosultap-sodium
   Agonisten des Ryanodin-Rezeptors,
      Benzoesäuredicarboxamide,
      zum Beispiel Flubendiamide
      Anthranilamide,
      zum Beispiel Rynaxypyr (3-bromo-N-{4-chloro-2-methyl-6-[(methylamino)carbonyl]phenyl}-1-(3-chloropyridin-2-yl)-1H-pyrazole-5-carboxamide)
   Biologika, Hormone oder Pheromone
      Azadirachtin, Bacillus spec., Beauveria spec., Codlemone, Metarrhizium spec., Paecilomyces spec., Thuringiensin, Verticillium spec.
   Wirkstoffe mit unbekannten oder nicht spezifischen Wirkmechanismen
      Begasungsmittel,
      zum Beispiel Aluminium phosphide, Methyl bromide, Sulfuryl fluoride
      Fraßhemmer,
      zum Beispiel Cryolite, Flonicamid, Pymetrozine
      Milbenwachstumsinhibitoren,
      zum Beispiel Clofentezine, Etoxazole, Hexythiazox
      Amidoflumet, Benclothiaz, Benzoximate, Bifenazate, Bromopropylate, Buprofezin, Chinomethionat, Chlordimeform, Chlorobenzilate, Chloropicrin, Clothiazoben, Cycloprene, Cyflumetofen, Dicyclanil, Fenoxacrim, Fentrifanil, Flubenzimine, Flufenerim, Flutenzin, Gossyplure, Hydramethylnone, Japonilure, Metoxadiazone, Petroleum, Piperonyl butoxide, Potassium oleate, Pyridalyl, Sulfluramid, Tetradifon, Tetrasul, Triarathene,Verbutin

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigt Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Emährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizidtolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführte Pflanzen können besonders vorteilhaft erfindungsgemäß mit den erfindungsgemäßen Avermectinderivaten bzw. Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor. Sie sind insbesondere gegen tierische Parasiten (Ekto- und Endoparasiten) wie beispielsweise Schildzecken, Lederzecken, Räudemilben, Laufmilben, Fliegen (stechend und leckend), parasitierende Fliegenlarven, Läuse, Haarlinge, Federlinge und Flöhe wirksam.

Zu diesen tierischen Parasiten gehören:
Aus der Ordnung der Anoplurida z.B. Haematopinus spp., Linognathus spp., Pediculus spp., Phtirus spp., oder Solenopotes spp.. Insbesondere Linognathus setosus, Linognathus vituli, Linognathus ovillus, Linognathus oviformis, Linognathus pedalis, Linognathus stenopsis, Haematopinus asini macrocephalus, Haematopinus eurysternus, Haematopinus suis, Pediculus humanus capitis, Pediculus humanus corporis, Phylloera vastatrix, Phthirus pubis oder Solenopotes capillatus.

Aus der Ordnung der Mallophagida und den Unterordnungen Amblycerina sowie Ischnocerina z.B. Trimenopon spp., Menopon spp., Trinoton spp., Bovicola spp., Wemeckiella spp., Lepikentron spp., Damalina spp., Trichodectes spp. oder Felicola spp.. Insbesondere Bovicola bovis, Bovicola ovis, Bovicola limbata, Damalina bovis, Trichodectes canis, Felicola subrostratus, Bovicola caprae, Lepikentron ovis oder Wemeckiella equi.

Aus der Ordnung der Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp., Rhinoestrus spp., oder Tipula spp.. Insbesondere Aedes aegypti, Aedes albopictus, Aedes taeniorhynchus, Anopheles gambiae, Anopheles maculipennis, Calliphora erythrocephala, Chrysozona pluvialis, Culex quinquefasciatus, Culex pipiens, Culex tarsalis, Fannia canicularis, Sarcophaga carnaria, Stomoxys calcitrans, Tipula paludosa, Lucilia cuprina, Lucilia sericata, Simulium reptans, Phlebotomus papatasi, Phlebotomus longipalpis, Odagmia ornata, Wilhelmia equina, Boophthora erythrocephala, Tabanus bromius, Tabanus spodopterus, Tabanus atratus, Tabanus sudeticus, Hybomitra ciurea, Chrysops caecutiens, Chrysops relictus, Haematopota pluvialis, Haematopota italica, Musca autumnalis, Musca domestica, Haematobia irritans irritans, Haematobia irritans exigua, Haematobia stimulans, Hydrotaea irritans, Hydrotaea albipuncta, Chrysomya chloropyga, Chrysomya bezziana, Oestrus ovis, Hypoderma bovis, Hypoderma lineatum, Przhevalskiana silenus, Dermatobia hominis, Melophagus ovinus, Lipoptena capreoli, Lipoptena cervi, Hippobosca variegata, Hippobosca equina, Gasterophilus intestinalis, Gasterophilus haemorroidalis, Gasterophilus inermis, Gasterophilus nasalis, Gasterophilus nigricornis, Gasterophilus pecorum, Braula coeca.

Aus der Ordnung der Siphonapterida z.B. Pulex spp., Ctenocephalides spp., Tunga spp. Xenopsylla spp. oder Ceratophyllus spp.. Insbesondere Ctenocephalides canis, Ctenocephalides felis, Pulex irritans, Tunga penetrans oder Xenopsylla cheopis.

Aus der Ordnung der Heteropterida z.B. Cimex spp., Triatoma spp., Rhodnius spp. oder Panstrongylus spp..

Aus der Ordnung der Blattarida z.B. Blatta orientalis, Periplaneta americana, Blattela germanica, Supella spp.. oder Supella longipalpa.

Aus der Unterklasse der Acari (Acarina) und den Ordnungen der Meta- sowie Mesostigmata z.B. Argas spp., Ornithodorus spp., Otobius spp., Ixodes spp., Amblyomma spp., Rhipicephalus (Boophilus) spp., Dermacentor spp., Haemophysalis spp., Hyalomma spp., Rhipicephalus spp., Dermanyssus spp., Ornithonyssus spp., Pneumonyssus spp., Raillietia spp., Stemostoma spp., Varroa spp. oder Acarapis spp.. Insbesondere Argas persicus, Argas reflexus, Ornithodorus moubata, Otobius megnini, Rhipicephalus (Boophilus) microplus, Rhipicephalus (Boophilus) decoloratus, Rhipicephalus (Boophilus) annulatus, Rhipicephalus (Boophilus) calceratus, Hyalomma anatolicum, Hyalomma aegypticum, Hyalomma marginatum, Hyalomma transiens, Rhipicephalus evertsi, Ixodes ricinus, Ixodes hexagonus, Ixodes canisuga, Ixodes pilosus, Ixodes rubicundus, Ixodes scapularis, Ixodes holocyclus, Haemaphysalis concinna, Haemaphysalis punctata, Haemaphysalis cinnabarina, Haemaphysalis otophila, Haemaphysalis leachi, Haemaphysalis longicorni, Dermacentor marginatus, Dermacentor reticulatus, Dermacentor pictus, Dermacentor albipictus, Dermacentor andersoni, Dermacentor variabilis, Hyalomma mauritanicum, Rhipicephalus sanguineus, Rhipicephalus bursa, Rhipicephalus appendiculatus, Rhipicephalus capensis, Rhipicephalus turanicus, Rhipicephalus zambeziensis, Amblyomma americanum, Amblyomma variegatum, Amblyomma maculatum, Amblyomma hebraeum, Amblyomma cajennense, Dermanyssus gallinae, Ornithonyssus bursa, Ornithonyssus sylviarum oder Varroa jacobsoni.

Aus der Ordnung der Actinedida (Prostigmata) und Acaridida (Astigmata). z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp. oder Laminosioptes spp.. Insbesondere Cheyletiella yasguri, Cheyletiella blakei, Demodex canis, Demodex bovis, Demodex ovis, Demodex caprae, Demodex equi, Demodex caballi, Demodex suis, Neotrombicula autumnalis, Neotrombicula desaleri, Neoschöngastia xerothermobia, Trombicula akamushi, Otodectes cynotis, Notoedres cati, Sarcoptis canis, Sarcoptes bovis, Sarcoptes ovis, Sarcoptes rupicaprae (=S. caprae), Sarcoptes equi, Sarcoptes suis, Psoroptes ovis, Psoroptes cuniculi, Psoroptes equi, Chorioptes bovis, Psoergates ovis, Pneumonyssoidic Mange, Pneumonyssoides caninum oder Acarapis woodi.

Die erfindungsgemäßen Avermectinderivate eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pow-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfmdungsgemäßen Verbindungen eine hohe insektizide Wirkung gegen Insekten zeigen, die technische Materialien zerstören.
Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Emobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifügus, Mastotermes darwiniensis, Zootermopsis nevadensis, Coptotermes formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.
Die anwendungsfertigen Mittel können gegebenenfalls noch weitere Insektizide und gegebenenfalls noch ein oder mehrere Fungizide enthalten.
Hinsichtlich möglicher zusätzlicher Zumischpartner sei auf die oben genannten Insektizide und Fungizide verwiesen.
Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.
Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören: Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp..
Aus der Ordnung der Chilopoda z.B. Geophilus spp..
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forfcula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.
Die Anwendung im Bereich der Haushaltsinsektizide erfolgt allein oder in Kombination mit anderen geeigneten Wirkstoffen wie Phosphorsäureestern, Carbamaten, Pyrethroiden, Neo-nicotinoiden, Wachstumsregulatoren oder Wirkstoffen aus anderen bekannten Insektizidklassen.
Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die erfindungsgemäße Behandlung der Pflanzen und Pflanzenteile mit den Wirkstoffkombinationen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Saatgut, weiterhin durch ein- oder mehrschichtiges Umhüllen.

Insbesondere eignen sich die erfindungsgemäßen Mischungen zur Behandlung von Saatgut. Bevorzugt sind dabei die vorstehend als bevorzugt oder besonders bevorzugt genannten erfindungsgemäßen Kombinationen zu nennen. So entsteht ein großer Teil des durch Schädlinge verursachten Schadens an Kulturpflanzen bereits durch den Befall des Saatguts während der Lagerung und nach dem Einbringen des Saatguts in den Boden sowie während und unmittelbar nach der Keimung der Pflanzen. Diese Phase ist besonders kritisch, da die Wurzeln und Sprosse der wachsenden Pflanze besonders empfindlich sind und bereits ein geringer Schaden zum Absterben der ganzen Pflanze führen kann. Es besteht daher ein insbesondere großes Interesse daran, das Saatgut und die keimende Pflanze durch den Einsatz geeigneter Mittel zu schützen.

Die Bekämpfung von Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen. Es ist weiterhin erstrebenswert, die Menge des eingesetzten Wirkstoffs dahingehend zu optimieren, dass das Saatgut und die keimende Pflanze vor dem Befall durch Schädlinge bestmöglich geschützt werden, ohne jedoch die Pflanze selbst durch den eingesetzten Wirkstoff zu schädigen. Insbesondere sollten Verfahren zur Behandlung von Saatgut auch die intrinsischen insektiziden Eigenschaften transgener Pflanzen einbeziehen, um einen optimalen Schutz des Saatguts und auch der keimenden Pflanze bei einem minimalen Aufwand an Pflanzenschutzmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einem erfindungsgemäßen Mittel behandelt wird. Die Erfindung bezieht sich ebenfalls auf die Verwendung der erfindungsgemäßen Mittel zur Behandlung von Saatgut zum Schutz des Saatguts und der daraus entstehenden Pflanze vor Schädlingen. Weiterhin bezieht sich die Erfindung auf Saatgut, welches zum Schutz vor Schädlingen mit einem erfindungsgemäßen Mittel behandelt wurde.

Einer der Vorteile der vorliegenden Erfindung ist es, dass aufgrund der besonderen systemischen Eigenschaften der erfindungsgemäßen Mittel die Behandlung des Saatguts mit diesen Mitteln nicht nur das Saatgut selbst, sondern auch die daraus hervorgehenden Pflanzen nach dem Auflaufen vor Schädlingen schützt. Auf diese Weise kann die unmittelbare Behandlung der Kultur zum Zeitpunkt der Aussaat oder kurz danach entfallen.

Ein weiterer Vorteil besteht in der synergistischen Erhöhung der insektiziden Wirksamkeit der erfindungsgemäßen Mittel gegenüber dem insektiziden Einzelwirkstoff, die über die zu erwartende Wirksamkeit der beiden einzeln angewendeten Wirkstoffe hinausgeht. Vorteilhaft ist auch die synergistische Erhöhung der fungiziden Wirksamkeit der erfindungsgemäßen Mittel gegenüber dem fungiziden Einzelwirkstoff, die über die zu erwartende Wirksamkeit des einzeln angewendeten Wirkstoffs hinausgeht. Damit wird eine Optimierung der Menge der eingesetzten Wirkstoffe ermöglicht.

Ebenso ist es als vorteilhaft anzusehen, dass die erfmdungsgemäßen Mischungen insbesondere auch bei transgenem Saatgut eingesetzt werden können, wobei die aus diesem Saatgut hervorgehenden Pflanzen zur Expression eines gegen Schädlinge gerichteten Proteins befähigt sind. Durch die Behandlung solchen Saatguts mit den erfindungsgemäßen Mitteln können bestimmte Schädlinge bereits durch die Expression des z.B. insektiziden Proteins kontrolliert werden, und zusätzlich durch die erfindungsgemäßen Mittel vor Schwaden bewahrt werden.

Die erfindungsgemäßen Mittel eignen sich zum Schutz von Saatgut jeglicher Pflanzensorte wie bereits vorstehend genannt, die in der Landwirtschaft, im Gewächshaus, in Forsten oder im Gartenbau eingesetzt wird. Insbesondere handelt es sich dabei um Saatgut von Mais, Erdnuss, Canola, Raps, Mohn, Soja, Baumwolle, Rübe (z.B. Zuckerrübe und Futterrübe), Reis, Hirse, Weizen, Gerste, Hafer, Roggen, Sonnenblume, Tabak, Kartoffeln oder Gemüse (z.B. Tomaten, Kohlgewächs). Die erfindungsgemäßen Mittel eignen sich ebenfalls zur Behandlung des Saatguts von Obstpflanzen und Gemüse wie vorstehend bereits genannt. Besondere Bedeutung kommt der Behandlung des Saatguts von Mais, Soja, Baumwolle, Weizen und Canola oder Raps zu.

Wie vorstehend erwähnt, kommt auch der Behandlung von transgenem Saatgut mit einem erfindungsgemäßen Mittel eine besondere Bedeutung zu. Dabei handelt es sich um das Saatgut von Pflanzen, die in der Regel zumindest ein heterologes Gen enthalten, das die Expression eines Polypeptids mit insbesondere insektiziden Eigenschaften steuert. Die heterologen Gene in transgenem Saatgut können dabei aus Mikroorganismen wie *Bacillus, Rhizobium, Pseudomonas, Serratia, Trichoderma, Clavibacter, Glomus* oder *Gliocladium* stammen. Die vorliegende Erfindung eignet sich besonders für die Behandlung von transgenem Saatgut, das zumindest ein heterologes Gen enthält, das aus *Bacillus sp.* stanunt und dessen Genprodukt Wirksamkeit gegen Maiszünsler und/oder Maiswurzel-Bohrer zeigt. Besonders bevorzugt handelt es sich dabei um ein heterologes Gen, das aus *Bacillus thuringiensis* stammt.

Im Rahmen der vorliegenden Erfindung wird das erfindungsgemäße Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

### Herstellungsbeispiele

### Avermectin B₁ Monosaccharid (III-1) (-C₂₂R¹-A-C₂₃R²- : -HC=CH-)

Eine Lösung von 13.52 g Abamectin (II-1) in 170 ml 2-Propanol und 1.7 ml konz. Schwefelsäure wird 20 Stunden bei Raumtemperatur gerührt. Nach Zugabe von gesättigter Natriumhydrogencarbonat-Lösung wird mit Dichlormethan extrahiert, die organische Phase über Natriumsulfat getrocknet und das Lösungsmittel im Vakuum entfernt. Der Rückstand wird über Kieselgel (Kieselgel 60 - Merck, Korngröße: 0.04 bis 0.063 mm) mit Cyclohexan/Essigester (5:2) gereinigt. Man erhält 6.83 g Avermectin B₁ Monosaccharid (III-1) als farblosen Feststoff.

### 5-O-tert-Butyldimethylsilyl-avermectin B₁ Monosaccharid (IV-1) (-C₂₂R¹-A-C₂₃R²- : -HC=CH-)

Zu einer Lösung von 6.8 g Avermectin B₁ Monosaccharid (III-1) in 31 ml *N,N-*Dimethylformamid werden 4.1 g Imidazol und 2.2 g *tert*-Butyldimethylsilylchlorid gegeben und der Ansatz 2 Stunden bei Raumtemperatur gerührt. Nach Zugabe von Cyclohexan und Essigester (1:1) wird mit Wasser und gesättigter Natriumchlorid-Lösung gewaschen, die organische Phase über Natriumsulfat getrocknet und das Lösungsmittel im Vakuum entfernt. Der Rückstand wird über Kieselgel (Kieselgel 60 - Merck, Korngröße: 0.04 bis 0.063 mm) mit Cyclohexan/Essigester (4:1) gereinigt. Man erhält 4.4 g 5-O-tert-Butyldimethylsilyl-avermectin B₁ Monosaccharid (IV-1) als farblosen Feststoff.

### 5-O-tert-Butyldimethylsilyl-4'-O-(cyclopropylearbonyl)-avermectin B₁ Monosaccharid (VI-1) (-C₂₂R¹-A-C₂₃R²- : -HC=CH-)

Man löst 150 mg 5-O-tert-Butyldimethylsilyl-avermectin B₁ Monosaccharid (IV-1) in 15 ml Dichlormethan. Unter Argon werden 102 mg N-(3-Dimethylaminopropyl)-N'-ethylcarbodiimid, 65 mg 4-Dimethylaminopyridin (DMAP), 46 mg Cyclopropan-carbonsäure und etwas Molsieb zugegeben und der Ansatz 2 Stunden bei Raumtemperatur gerührt. Nach Zugabe von gesättigter Natriumhydrogencarbonat-Lösung wird mit Dichlormethan extrahiert, die organische Phase über Natriumsulfat getrocknet und das Lösungsmittel im Vakuum entfernt. Der Rückstand wird über Kieselgel (Kieselgel 60 - Merck, Korngröße: 0.04 bis 0.063 mm) mit Cyclohexan/ Essigester (4:1) filtriert. Man erhält 150 mg 5-O-tert-Butyldimethylsilyl-4'-O-(cyclopropylcarbonyl)-avennectin B₁ Monosaccharid (VI-1).
LC-MS: 933.6 (M+Na, 80%) C₅₁H₇₈O₁₂Si (911.251)
Retentionszeit: 20.5 min (22 min)

### Methode A:

### Beispiel 1: 4'-O-(Cyclopropylcarbonyl)avermectin B₁ Monosaccharid (-C₂₂R¹-A-C₂₃R²- : -HC=CH-)

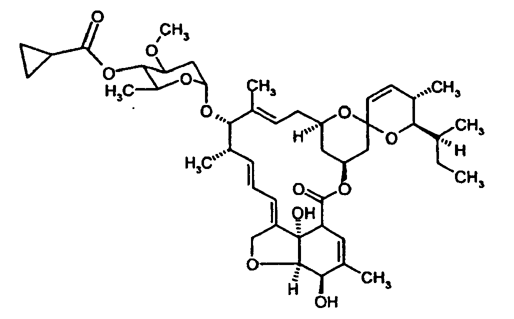

Zu einer Lösung von 150 mg 5-O-tert-Butyldimethylsilyl-4'-O-(cyclopropyl-carbonyl)-avermectin B₁ Monosaccharid (VI-1) in absolutem Methanol werden 6 mg 4-Toluolsulfonsäure gegeben und der Ansatz 4 Stunden bei Raumtemperatur gerührt. Nach Zugabe von gesättigter Natriumhydrogencarbonat-Lösung wird mit Essigester extrahiert, die organische Phase über Natriumsulfat getrocknet und das Lösungsmittel im Vakuum entfernt. Der Rückstand wird durch präparative HPLC (Waters 2996 Photodiode Array Detector, 245nm, x Terra Prep MS C18 Column 5µm 19x100mm, Flow 20ml/min Wasser/Acetonitril) gereinigt. Man erhält 47 mg 4'-O-(Cyclopropylcarbonyl)-avermectin B, Monosaccharid.
LC-MS: 797.6 (M+H, 60%) C₄₅H₆₄O₁₂, MW: 796.989
Retentionszeit: 15.70 min (22 min)

¹H-NMR (600 MHz, CDCl₃): δ (ppm) = 0.85-0.96 (m, 11H), 1.05 (m, sym, 2H), 1.13 (d, J = 6.3, 3H), 1.17 (d, J = 7.0, 3H), 1.45-1.71 (m, 10H), 1.78 (m, sym 1H), 1.88 (s, 3H), 2.02 (ddd, J = 12.1, 4.8, 1.6, 1H), 2.21-2-38 (m, 5H), 2.54 (m, sym., 1H), 3.30 (q, J = 2.2, 1H), 3.45 (s, 3H), 3.49 (dd, J = 11.4, 1.5, 1H), 3.68 (m, sym, 1H), 3.88 (m, sym, 1H), 3.92-3.96 (m, 2H), 3.98 (d, J = 6.3, 1H), 4.06 (s, 1H), 4.30 (s, breit, 1H), 4.65-4.73 (m, 3H), 4.82 (d, J = 3.4, 1H), 4.99 (d, J = 8.8, 1H), 5.38-5.44 (m, 2H), 5.55 (dd, J=9.9, 2.6, 1H), 5.73-5.79 (m, 3H), 5.88 (m, sym, 1H).

¹³C-NMR (150 MHz, CDCl₃): δ (ppm) = 8.41, 8.54, 12.03, 12.93, 12.96, 15.11, 16.36, 17.38, 19.98, 20.20, 27.49, 30.55, 34.23, 34.85, 35.11, 36.61, 39.70, 40.42, 45.66, 57.32, 66.57, 67.68, 68.29, 68.32, 68.45, 74.89, 75.81, 76.14, 79.00, 80.35, 81.95, 94.94, 95.73, 118.00, 118.33, 120.35, 124.80, 127.67, 134.99, 136.34, 137.88, 137.96, 139.69, 173.76, 174.29.

Analog können die in der nachstehenden Tabelle (Tabelle 2) aufgeführten Avermectinderivate der Formel (I) (mit R⁵ = H) hergestellt werden.

**Tabelle 2**

| **Bsp. Nr.** | **-C₂₂R¹-A-C₂₃R²-** | **R³** | **R⁴** | **Physikalische Daten ^{a)}** |
|---|---|---|---|---|
| **2** | -HC=CH- | sec-Butyl | 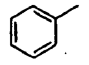 | 7.47 (t, J = 7.5, 2H), 7.59 (t, J = 7.4, 1H), 8.10 (d, J = 7.1, 2H); m/z=833.6 (M+H, 80); Rₜ=16.75 (22) |
| **3** | -HC=CH- | sec-Bu | 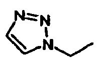 | 5.27 (d, J = 5.0, 2H), 7.77 (d, J = 7.9, 2H); m/z=838.6 (M+H, 70); Rₜ=13.86 (22) |
| **4** | -HC=CH- | sec-Bu | 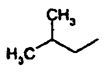 | 0.99 (dd, J = 6.6, 1.3, 6H), 2.10-2.21 (m, 1H), 2.23-2.36 (m, 7H); m/z=813.6 (M+H, 100); Rₜ=16.89 (22) |
| **5** | -HC=CH- | sec-Bu | 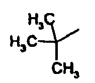 | 1.24 (s, 9H); m/z= 813.6 (M+H, 80); Rₜ=16.80 (22) |
| **6** | -HC=CH- | sec-Bu | 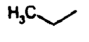 | 1.12-1.21 (m,9H), 2.21-2.43 (m, 7H); m/z= 785.6 (M+H, 100); Rₜ=15.61 (22) |
| **7** | -HC=CH- | sec-Bu | 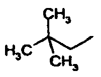 | 1.07 (s, 9H), 2.22-2.36 (m, 7H); m/z=827.6 (M+H, 70); Rₜ=17.46 (22) |
| **8** | -HC=CH- | sec-Bu | 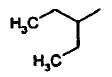 | 0.90-0.97 (m, 15H), 2.20-2.36 (m, 6H); m/z=827.6 (M+H, 50), 849.6 (M+Na, 20); Rₜ=17.45 (22) |
| **9** | -HC=CH- | sec-Bu | 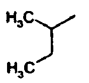 | 0.89-0.98 (m; 12H), 1.10-1.20 (m, 9H), 2.36-2.48 (m, 1H); m/z=813.6 (M+H, 80); Rₜ=16.84 (22) |
| **10** | -HC=CH- | sec-Bu | 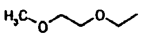 | 3.40 (s, 3H), 3.60-3.62 (m, 2H), 3.74-3.77 (m, 2H), 4.22 (d, J = 0.7, 2H); m/z=867 (M+Na, 40); Rₜ=14.89 (22) |
| **11** | -HC=CH- | sec-Bu | 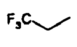 | 3.25 (q, J = 10.1, 2H); m/z=839.5 (M+H, 50); Rₜ=15.99 (22) |
| **12** | -HC=CH- | sec-Bu | 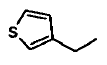 | 3.71 (s, 2H), 7.07 (dd, J = 5.0, 1.2, 1H), 7.19-7.20 (m, 1H), 7.26-7.30 (m, 1H); m/z=853.5 (M+H, 70), 870.5 (M+NH4, 100); Rₜ=16.26 (22) |
| **13** | -HC=CH- | sec-Bu | 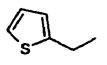 | 3.89 (s, 2H), 6.93.6.99 (m, 2H), 7.22 (dd, J = 5.0, 1.3, 1H); m/z=853 (M+H, 80); Rₜ=16.26 (22) |
| **14** | -HC=CH- | sec-Bu | 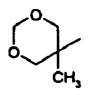 | 1.20 (s, 3H), 3.56-3.62 (m, 2H), 4.30-4.33 (m, 2H), 4.75-4.77 (m, 1H), 4.89 (d, J = 6.3, 1H); m/z=879.6 (M +Na, 50); Rₜ=15.39 (27) |
| **15** | -HC=CH- | sec-Bu | 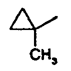 | 0.69-0.73 (m, 2H), 1.25-1.29 (m, 2H), 1.3 (s, 3H); m/z=811.6 (M+H, 40); Rₜ=16.51 (27) |
| **16** | -HC=CH- | sec-Bu | 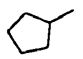 | 2.79 (q, J = 8, 1H); m/z=825.6 (M+H, 40); Rₜ=17.25 (27) |
| **17** | -H₂C-CH₂- | sec-Bu | 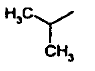 | 1.20 (d, J = 7.0, 3H), 1.21 (d, J = 7.0, 3H), 2.60 (q, J = 7.0, 1H); m/z=824.6 (M+Na, 20); Rₜ=18.07 (27) |
| **18** | -H₂C-CH₂- | sec-Bu | 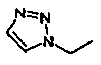 | 5.27 (d, J = 5.3, 2H), 7.77 (dd, J = 8.3, 1.1, 2H); m/z=840.6 (M+H, 70); Rₜ=15.82 (27) |
| **19** | -HC=CH- | sec-Bu | 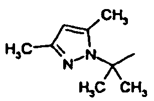 | 1.83, 1.87, 2.22, 2.25 (s, je 3H), 5.86 (s, 1H); m/z=893.7 (M+H, 100); Rₜ=16.70 (27) |
| **20** | -HC=CH- | sec-Bu | 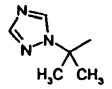 | 1.92 (s, 3H), 1.93 (s, 3H), 7.96 (s, 1H), 8.27 (s, 1H); m/z=866.6 (M+H, 100); Rₜ=14.76 (27) |
| **21** | -H₂C-CH₂- | sec-Bu | 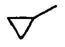 | 1.03-1.05 (m, 2H); m/z=801 (M+H, 70); Rₜ=17.59 (27) |
| **22** | -H₂C-CH₂- | sec-Bu | 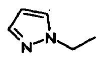 | 4.99 (d, J = 2.3, 2H), 6.34 (t, J = 2.1, 1H), 7.52 (d, J = 2.3, 1H), 7.56 (d, J = 1.6, 1H); m/z=840 (M+H, 10); Rₜ=16.41 (22) |
| **23** | -H₂C-CH₂- | sec-Bu | 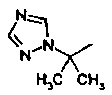 | 1.92 (s, 3H), 1.93 (s, 3H), 7.96 (s, 1H), 8.28 (s, 1H); m/z=868.7 (M+H, 100); Rₜ=16.56 (22) |
| **24** | -H₂C-CH₂- | sec-Bu | 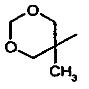 | 1.20 (s, 3H), 3.56-3.62 (m, 2H), 4.30-4.33 (m, 2H), 4.74-4.77 (m, 1H), 4.89 (d, J = 6.0, 1H); m/z=882.8 (M+Na, 80); Rₜ=17,11 (22) |
| **25** | -HC=CH- | sec-Bu | 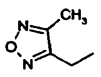 | 2.41 (s, 3H), 3.90 (d, J = 1.1, 2H); m/z=853.7 (M+H, 100); Rₜ=15.98 (27) |
| **26** | -HC=CH- | sec-Bu | 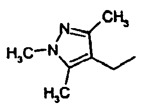 | 2.20 (s, 3H), 2.20 (s, 3H), 3.33 (s, 2H), 3.70 (s, 3H); m/z=879.9 (M+H, 100); Rₜ=14.48 (27) |
| **27** | -HC=CH- | sec-Bu | 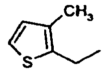 | 2.21 (d, J = 1.1, 3H), 3.78 (d, J = 1.1, 2H), 6.82 (dd, J = 5.1, 0.8, 1H), 7.11 (dd, J = 5.1, 0.9, 1H); m/z=867.8 (M+H, 60); Rₜ=16.90 (27) |
| **28** | -HC=CH- | sec-Bu | 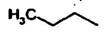 | 0.98 (t, J = 7.4, 3H), 2.21-2.37 (m, 7H); m/z=799.6 (M+H, 100); Rₜ=16.27 (22) |
| **29** | -HC=CH- | sec-Bu | 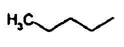 | 0.89-0.96 (m, 12H), 1.39 (sext., J = 7.6, 2H), 2.22-2.39 (m, 7H); m/z=813.6 (M+H, 70); Rₜ=16.89 (22) |
| **30** | -HC=CH- | sec-Bu | 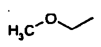 | 3.48 (s, 3H), 4.09 (s, 2H); m/z=3.48 (s, 3H), 4.09 (s, 2H); Rₜ=14.72 (22) |
| **31** | -HC=CH- | sec-Bu | 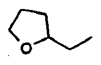 | 1.86-1.98 (m, 6H), 2.06-2.17 (m, 1H), 2.49-2.56 (m, 2H), 2.67 (ddd, J = 21.4, 10.8, 6.7, 1H), 3.75 (q, J = 7.6, 1H), 3.83-3.99 (m, 5H), 4.24-4.32 (m, 2H); m/z=841 (M+H, 10), 858.6 (M+NH4, 100); Rₜ=15.38 (22) |
| **32** | -HC=CH- | sec-Bu | 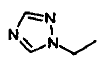 | 5.04 (s, 2H), 7.99 (s, 1H), 8.25 (s, 1H); m/z=838.5 (M+H, 100); Rₜ=13.46 (22) |
| **33** | -HC=CH- | sec-Bu | 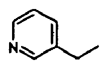 | 3.69 (s, 2H), 7.28 (dd, J = 4.9, 3.0, 1H), 7.69 (dt, J = 7.9, 2.2, 1H), 8.53 (dd, 4.8, 2.0, 1H), 8.45 (d, J = 1.8, 1H); m/z=848.6 (M+H, 100); Rₜ=12.50 (22) |
| **34** | -HC=CH- | sec-Bu | 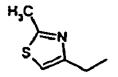 | 3.68 (s, 2H), 6.93-6.99 (m, 1H), 7.04-7.12 (m, 2H), 7.26-7.32 (m, 1H); m/z=865.5 (M+H, 90); Rₜ=16.53 (22) |
| **35** | -HC=CH- | sec-Bu | 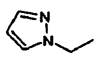 | 4.99 (s, 2H), 6.34 (t, J = 2.2, 1H), 7.52 (d, J = 2.3, 1H), 7.56 (d, J = 1.9); m/z=837 (M+H, 10); Rₜ=14.43 (22) |
| **36** | -HC=CH- | sec-Bu | 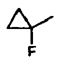 | 0.85-0.90 (m, 2H); m/z=815.6 (M+H, 50); Rₜ=16.05 (27) |
| **37** | -HC=CH- | sec-Bu | 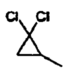 | 1.92 (dd, J = 9.7, 7.3, 1H), 2.13 (t, J = 7.3, 1H), 2.62 (dd, J = 9.6, 7.9, 1H); m/z=865.5 (M+H, 40); Rₜ=16.89 (27) |
| **38** | -HC=CH- | sec-Bu | 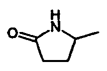 | 2.50 -2.58 (m, 2H), 4.27-4.33 (m, 2H); m/z=862.5 (M+Na, 5); Rₜ=13.32 (27) |
| **39** | -HC=CH- | sec-Bu | 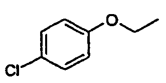 | 4.67-4.70 (m, 5H), 6.87 (d, J = 9.0, 2H), 7.25 (d, J = 8.7, 2H); m/z=897.6 (M+H, 40); Rₜ=17.13 (27) |
| **40** | -HC=CH- | sec-Bu | 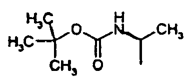 | 1.44 (s, 9H), 1.50 (s, 3H), 4.26-4.35 (m, 1H); m/z=922.7 (M+H, 20); Rₜ=16.36 (27) |
| **41** | -HC=CH- | sec-Bu | 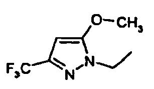 | 3.41 (s, 3H), 4.83 (s, 2H), 5.83 (s, 1H); m/z=957.6 (M+Na, 20); Rₜ=16.36 (27) |
| **42** | -HC=CH- | sec-Bu | 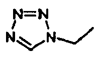 | 5.32 (d, J = 2.8, 2H), 8.86 (s, 1H); m/z=861.6 (M+Na, 10); Rₜ=14.28 (27) |
| **43** | -HC=CH- | sec-Bu | 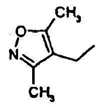 | 2.25 (s, 3H), 2.37 (s, 3H), 3.34 (s, 2H); m/z=864 (M-H, 100); Rₜ=864 (M-H, 100) |
| **44** | -HC=CH- | sec-Bu | 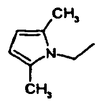 | 2.19 (s, 6H), 4.54 (s, 2H), 5.80 (s, 2H); m/z=846 (M-18, 100); Rₜ=16.80 (27) |
| **45** | -HC=CH- | sec-Bu | 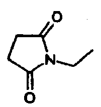 | 2.81 (s, 4H), 4.24 (d, 1H), 4.38 (d, 1H); m/z=868 (M+H, 40); Rₜ=14.27 (27) |
| **46** | -HC=CH- | sec-Bu | 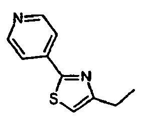 | 3.93-3.99 (m, 5H), 7.80 (dd, J = 4.6, 1.5, 2H), 8.69 (d, J = 6.0, 2H); m/z=931.8 (M+H, 100); Rₜ=14.39 (27) |
| **47** | -HC=CH- | sec-Bu | 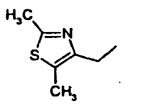 | 2.37 (s, 3H), 2.60 (s, 3H), 3.75 (s, 2H); m/z=882 (M+H, 40); Rₜ=15.64 (27) |
| **48** | -HC=CH- | sec-Bu | 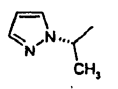 | 1.83 (d, J = 7.1, 3H), 5.18 (q, J = 7.3, 1H), 6.32 (t, J = 2.2, 1H), 7.54 (dd, J = 1.3, 0.5, 1H), 7.59 (d, J = 2.3, 1H); m/z=851 (M+H, 10); Rₜ=15.22 (27) |
| **49** | -HC=CH- | sec-Bu | 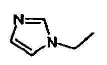 | 4.75 (s, 2H), 6.99 (t, J = 1.3, 1H), 7.11 (t, J = 1.0, 1H), 7.54 (s, 1H); m/z=837 (M+H, 100); Rₜ=10.32 (27) |
| **50** | -HC=CH- | sec-Bu | 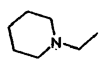 | 2.49-2.59 (m, 5H), 3.25 (d, J = 4.6, 2H); m/z=854.4 (M+H, 100); Rₜ=10.51 (27) |
| **51** | -HC=CH- | sec-Bu | 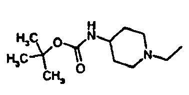 | 1.44 (s, 9H), 1.90-1.99 (m, 2H), 2.86-2.97 (m, 2H), 4.40-4.47 (m, 1H); m/z=969.5 (M+H, 100); Rₜ=11.15 (27) |
| **52** | -HC=CH- | sec-Bu | 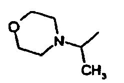 | 1.31-1.38 (m, 3H), 2.60-2.71 (m, 4H), 3.26-3.33 (m, 2H), 3.63.3.78 (m, 5H); m/z=870.4 (M+H, 100); Rₜ=11.66 (27) |
| **53** | -HC=CH- | sec-Bu | 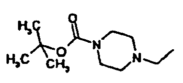 | 1.46 (s, 9H), 2.49-2.60 (m, 5H), 3.28-3.32 (m, 3H), 3.46-3.52 (m, 5H); m/z=955.6 (M+H, 100); Rₜ=14.18 (27) |
| **54** | -HC=CH- | sec-Bu | 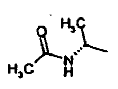 | 2.02 (s, 3H), 4.60-4.73 (m, 4H), 6.05 und 6.12 (d, d, zusamm. 1H); m/z=842 (M+H, 2); Rₜ=13.48 (27) |
| **55** | -HC=CH- | sec-Bu | 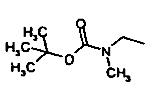 | 1.44 (s, 9H), 2.95 (d, J = 4.4, 3H), 4.03-4.04 (m, 2H); m/z=900 (M+H, 5), 917 (M+18, 30); Rₜ=16.61 (27) |
| **56** | -HC=CH- | sec-Bu | 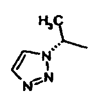 | 1.87-1_{.}92 (m, 6H), 5.50-5.62 (m, 2H), 7.76 (d, J = 1.1, 1H), 7.77 (d, J = 1.1, 1H); m/z=852 (M+H, 50); Rt=14.47 (27) |
| **57** | -HC=CH- | sec-Bu | 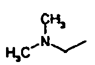 | 2.38 (s, 6H), 3.24 (d, J = 6.3, 2H); m/z=814.5 (M+H, 100); Rt=10.54 (27) |
| **58** | -HC=CH- | sec-Bu | 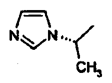 | 1.79 (d, J = 7.3, 3H), 4.92 (q, J = 7.3, 1H), 7.06 (s, 1H), 7.10 (s, 1H), 7.61 (s, 1H); m/z=851.5 (M+H, 100); Rt=11.01 (27) |
| **59** | -HC=CH- | sec-Bu | 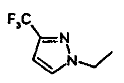 | 5.02 (s, 2H), 6.61 (d, J = 2.1, 1H), 7.59 (dd, J = 2.4, 0.9, 1H); m/z=905.4 (M+H, 60); Rₜ=16.25 (27) |
| **60** | -HC=CH- | sec-Bu | 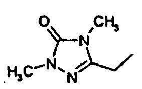 | 3.26 (s, 3H), 3.45 (s, 3H), 3.66 (s, 2H); m/z=864.5 (M - 18, 20), 883 (M + H, 5); Rₜ=13.55 (27) |
| **61** | -HC=CH- | sec-Bu | 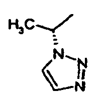 | 1.87-1.91 (m, 6H), 5.51-5.62 (m, 2H), 7.76-7.77 (m, 2H); m/z=852.4 (M+H, 100); Rₜ=14.43 (27) |
| **62** | -HC=CH- | sec-Bu | 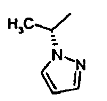 | 1.83 (d, J = 10.4, 3H), 5.18 (q, J = 7.3, 1H), 6.32 (dd, J = 2.3, 1.9, 1H), 7.54 (dd, J = 1.8, 0.5, 1H), 7.56 (dd, J = 2.3, 0.4, 1H); m/z=833.4 (M-18, 100), 852 (M+H, 5); Rₜ=15.17 (27) |
| **63** | -HC=CH- | sec-Bu | 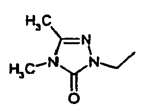 | 2.23 (s, 3H), 3.23 (s, 3H), 4.58 (d, J = 4.1, 2H); m/z=899 (M+18, 20); Rₜ=13.41 (27) |
| **64** | -HC=CH- | sec-Bu | 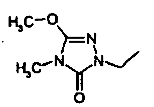 | 3.14 (s, 3H), 3.96 (s, 3H), 4.52 (s, 2H); m/z=915 (M+18, 15); Rₜ=13.98 (27) |
| **65** | -HC=CH- | sec-Bu | 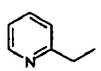 | m/z=830 (M-18, 100); Rₜ=13.91 (27) |
| **66** | -HC=CH- | sec-Bu | 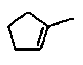 | 1.93-2.05 (m, 3H), 2.50-2.58 (m, 3H), 2.58-2.65 (m, 2H), 6.85 (d, J = 2H, 1H); m/z=919 (M+H, 20); Rₜ=16.76 (27) |
| **67** | -HC=CH- | sec-Bu | 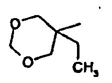 | 0.90-0.98 (m, 15H), 3.60-3.70 (m, 3H), 4.28-4.37 (m, 2H), 4.71-4.80 (m, 2H); m/z=870 (M+H, 30); Rₜ=15.86 (27) |
| **68** | -HC=CH- | sec-Bu | 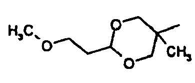 | m/z=915 (M+H, 20); Rₜ=16.51 (27) |
| **69** | -HC=CH- | sec-Bu | 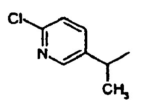 | m/z=897 (M+H, 50); Rₜ=16.52 (27) |
| **70** | -HC=CH- | sec-Bu | 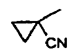 | m/z=821 (M+H, 60); Rₜ=15.70 (27) |
| **71** | -HC=CH- | sec-Bu | 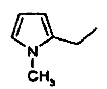 | 3.60 (s, 3H), 3.67 (s, 2H), 6.04-6.08 (m, 2H), 6.59 (t, J = 2.3, 1H); m/z=850 (M+H, 20); Rₜ=8.74 (12) |
| **72** | -HC=CH- | sec-Bu | 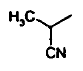 | 1.64 (dd, J = 7.4, 2.6, 3H), 3.61 (quint., J = 7.6, 1H); m/z=809 (M+H, 80); Rₜ=15.49 (27) |
| **73** | -HC=CH- | sec-Bu | 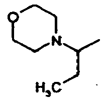 | m/z=884 (M+H, 100); Rₜ=9.79 (27) |
| **74** | -HC=CH- | sec-Bu | 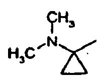 | 0.96-0.99 (m, 2H), 1.29-1.33 (m, 2H), 2.57 (s, 6H); m/z=840 (M+H, 100); Rₜ=11.68 (27) |
| **75** | -HC=CH- | sec-Bu | 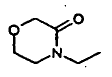 | m/z=853 (M-18, 30); Rₜ=13.60 (27) |
| **76** | -HC=CH- | sec-Bu | 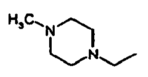 | 2.40 (s, 3H), 2.46-2.80 (m, 13H), 3.27-3.31(m, 3H); m/z=867 (M-H, 80); Rₜ=10.19 (27) |
| **77** | -HC=CH- | sec-Bu | 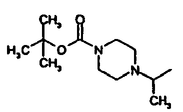 | m/z=969 (M+H, 100); Rₜ=14.63 und 15.05 (27) |
| **78** | -HC=CH- | sec-Bu | 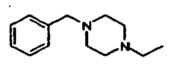 | 2.48-2.69 (m, 8H), 3.27 (d, J = 2.7, 2H), 3.52 (s, 2H), 7.24-7.32 (m, 5H); m/z=945 (M+H, 100); Rₜ=11.33 (27) |
| **79** | -HC=CH- | sec-Bu | 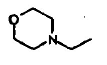 | 2.58-2.67 (m, 4H), 3.27 (d, J = 2.6, 2H), 3.77 (t, J = 4.6, 4H); m/z=856 (M+H, 100); Rₜ=10.83 (27) |
| **80** | -HC=CH- | sec-Bu | 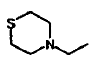 | m/z=872 (M+H, 100); Rₜ=11.96 (27) |
| **81** | -HC=CH- | sec-Bu | 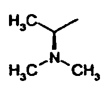 | 1.35 (d, J = 7.0, 3H), 2.38 (s, 6H), 3.66-3.78 (m, 2H); m/z=828 (M+H, 100); Rₜ=10.90 (27) |
| **82** | -HC=CH- | sec-Bu | 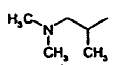 | 1.88 (s, 3H), 2.26 (s, 6H), 3.29-3.32 (m, 2H); m/z=842 (M+H, 100); Rₜ=10.52 (27) |
| **83** | -HC=CH- | sec-Bu | 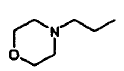 | m/z=870 (M+H, 100); Rₜ=10.88 (27) |
| **84** | -HC=CH- | sec-Bu | 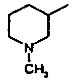 | m/z=854 (M+H, 100); Rₜ=10.79 (27) |
| **85** | -HC=CH- | sec-Bu | 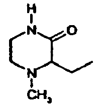 | 2.40 (s, 3H), 2.60-2.66 (m, 1H), 2.89-3.11 (m, 3H), 3.22-3.31 (m, 2H)3.48 (d, J = 8.5, 1H); m/z=883 (M+H, 5); Rₜ=10.13 (27) |
| **86** | -HC=CH- | sec-Bu | 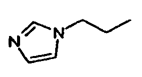 | 2.80-2.88 (m, 2H), 4.27-4.35 (m, 3H), 6.99 (s breit, 1H), 7.07 (s breit, 1H), 7.54 (s breit, 1H); m/z=852 (M+H, 100); Rₜ=10.31 (27) |
| **87** | -HC=CH- | sec-Bu | 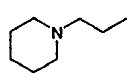 | 2.49-2.75 (m, 7H), 2.82-2.90 (m, 2H); m/z=868 (M+H, 100); Rₜ=11.03 (27) |
| **88** | -HC=CH- | sec-Bu | 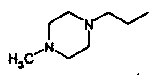 | 2.21-2.40 (m, 11H), 2.59-2.65 (m, 2H), 2.72-2.80 (m, 2H); m/z=883.5 (M+H, 100); Rₜ=10.22 (27) |
| **89** | -HC=CH- | sec-Bu | 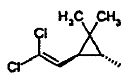 | 1.22 (s, 3H), 1.29 (s, 3H), 1.68 (d, J = 5.3, 1H), 2.22-2.38 (m, 6H), 5.65 (d, J = 8.1, 1H); m/z=919.5 (M+H, 80); Rₜ=18.49 (22) |
| **90** | -HC=CH- | sec-Bu | 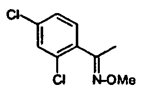 | 4.08 (s, 3H), 7.17 (d, J = 8.3, 1H), 7.32 (dd, J = 8.3, 2.0, 1H), 7.46 (d, J = 2.0, 1H); m/z=958 (M+H, 80); Rₜ=17.81 (27) |
| **91** | -HC=CH- | sec-Bu | 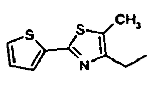 | 2.43 (s, 3H), 3.81 (s, 2H), 7.04 (dd, J = 5.1, 3.7, 1H), 7.33 (dd, J = 5.1, 1.1, 1H), 7.39 (dd, J = 3.7, 1.1, 1H); m/z=951 (M+H, 15); Rₜ=17.19 (27) |
| **92** | -HC=CH- | sec-Bu | 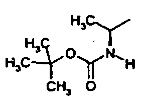 | 1.46 (s, 9H), 1.88 (s, 3H), 2.85 (s, 3H), 4.62-4.75 (m, 4H); m/z=931.5 (M+18, 20); Rₜ=17.18 (27) |
| **93** | -HC=CH- | sec-Bu | 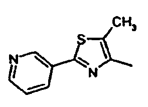 | 2.49 (s, 3H), 3.86 (s, 2H), 7.34 (dd, J = 8.0, 4.8, 1H), 8.16 (d, J = 8.0, 1H), 8.61 (dd, J = 4.8, 1.6, 1H), 9.06 (d, J = 1.6, 1H); m/z= 945.5 (M+H, 100); Rₜ= 15.48 (27) |
| **94** | -HC=CH- | sec-Bu | 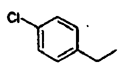 | 3.64 (s, 2H), 7.25-7.32 (m, 4H); m/z= 881 (M+H, 100); Rₜ= 17.21 (27) |
| **95** | -HC=CH- | sec-Bu | 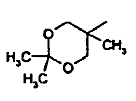 | 1.59 (s, 3H), 1.60 (s, 3H), 7.28-7.34 (m, 4H); m/z= 909 (M+H, 100) |
| **96** | -HC=CH- | sec-Bu | 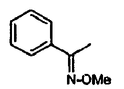 | 4.07 (s, 3H), 7.41-7.42 (m, 3H), 7.49-7.52 (m, 2H); m/z=890 (M+H, 30); Rₜ= 9,01 (12) |
| **97** | -HC=CH- | sec-Bu | 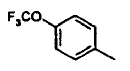 | 7.29 (t, J = 8.1, 2H), 8.15 (dd, J = 6.9, 2.0, 2H); m/z= 917.5 (M+H, 100); Rₜ= 17.87 (22) |

| | | | | |
|---|---|---|---|---|
| ^{a)} Bei LC-MS wird die Retentionszeit (Rₜ) sowie in Klammern die Gesamtlaufzeit des Chromatogramms in Minuten angegeben; die Prozentangabe der gefundenen Masse bezieht sich auf die relative Intensität, normiert auf 100. Zur Beschreibung der ¹H-NMR-Signale werden als Abkürzungen verwendet: s (Singulett), d (Dublett), t (Triplett), q (Quartett), sym (symmetrisch). | | | | |

### Methode B:

### 5-O-tert-Butyldimethylsilyl-4'-O-isobutyryl-avermectin B₁ Monosaccharid (VI-2) (-C₂₂R¹-A-C₂₃R²- : -HC=CH-)

Man löst 120 mg 5-O-tert-Butyldimethylsilyl-avermectin B1 Monosaccharid in ca. 15 ml Dichlormethan. Unter Argon werden 1.7 mg 4-Dimethylaminopyridin, 43 mg Triethylamin und 15 mg Isobutyrylchlorid zugegeben und der Ansatz 2 Stunden bei Raumtemperatur gerührt. Man gibt weitere 1.7 mg 4-Dimethylaminopyridin, 8 mg Triethylamin und 3 mg Isobutyrylchlorid zu und rührt 2 Stunden. Nach Zugabe von gesättigter Natriumhydrogencarbonat-Lösung wird mit Dichlormethan extrahiert, die organische Phase über Natriumsulfat getrocknet und das Lösungsmittel im Vakuum entfernt. Der Rückstand wird über Kieselgel mit Cyclohexan/Essigester (4:1) filtriert. Man erhält 139 mg 5-O-tert-Butyldimethylsilyl-4'-O-isobutyryl-avermectin B1 Monosaccharid (VI-2).
LC-MS: 935.6 (M+Na, 100%) C₅₁H₈₀O₁₂Si (913.267)
Retentionszeit: 11.75 min (12 min),

### Beispiele 98 4'-O-Isobutyryl-avermectin B1 Monosaccharid (-C₂₂R¹-A-C₂₃R²- : -HC=CH-)

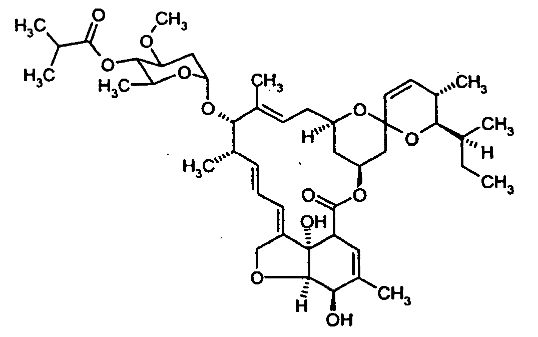

Zu einer Lösung von 139 mg 5-O-tert-Butyldimethylsilyl-4'-O-isobutyryl-avermectin B1 Monosaccharid (VI-2) in absolutem Methanol werden 6 mg 4-Toluolsulfonsäure gegeben und der Ansatz 2 Stunden bei Raumtemperatur gerührt. Nach Zugabe von gesättigter Natriumhydrogencarbonat-Lösung wird mit Essigester extrahiert, die organische Phase über Natriumsulfat getrocknet und das Lösungsmittel im Vakuum entfernt. Der Rückstand wird durch präparative HPLC gereinigt. Man erhält 34 mg 4'-O-Isobutyryl-avermectin B1 Monosaccharid (Beispiel 98).
LC-MS: 799.6 (M+H, 100%) C₄₅H₆₆O₁₂ (799.004)
Retentionszeit: 8.84 min (12 min)
¹H-NMR (400 MHz, CDCl₃) ausgewählte Signale: δ (ppm) = 1.21 (d, J = 7.0, 3H), 1.22 (d, J = 7.0, 3H), 2.58-2.63 (m, 1H).

Analog können die in der nachstehenden Tabelle (Tabelle 3) aufgeführten Verbindungen der Formel (I) mit R⁵ = H hergestellt werden.

**Tabelle 3**

| **Bsp.-Nr.** | **-C₂₂R¹-A-C₂₃R²-** | **R³** | **R⁴** | **Physikalische Daten ^{a)}** |
|---|---|---|---|---|
| **99** | -HC=CH- | sec-Bu | 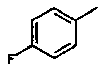 | δ (ppm) = 7.14 (t, J = 8.7, 2H), 8.11 (symmetr. m, 2H); m/z = 851.6 (M+H, 100); Rₜ= 16.88 (22) |
| **100** | -HC=CH- | sec-Bu | 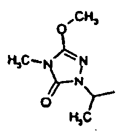 | δ (ppm) = 1.66 und 1.68 (d, J = 7.4, zus. 3H), 3.12 (s, 3H), 3.95 und 3.97 (s, zus. 3H), 4.91-4.99 (m, 2H); m/z = 929.4 (M+18,15%); Rₜ= 14.53, 14.72 (27) |

| | | | | |
|---|---|---|---|---|
| ^{a)} Bei LC-MS wird die Retentionszeit (Rₜ) sowie in Klammern die Gesamtlaufzeit des Chromatogramms in Minuten angegeben; die Prozentangabe der gefundenen Masse bezieht sich auf die relative Intensität, normiert auf 100. Zur Beschreibung der ¹H-NMR-Signale (ausgewählte Signale) werden als Abkürzungen verwendet: s (Singulett), d (Dublett), t (Triplett), q (Quartett), sym (symmetrisch). | | | | |

### Methode C:

### Beispiel 101 4'-O-(Piperazin-1-ylacetyl)-avermectin B1 Monosaccharid (-C₂₂R¹-A-C₂₃R²- : -HC=CH-)

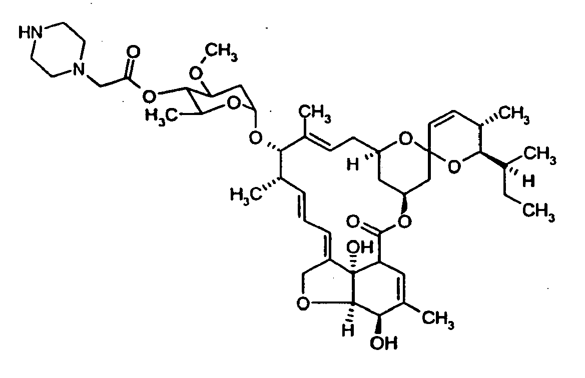

Man löst 163 mg 4'-O-{[4-(tert-Butoxycarbonyl)piperazin-1-yl]acetyl}-avermectin B1 Monosaccharid (Verbindung aus Bsp. 103) in 0.7 ml Essigester. Zu dieser Lösung werden 2.1 ml 3N Salzsäure in drei Portionen innerhalb von 3 Stunden gegeben und 2 Stunden nachgerührt. Nach Zugabe von gesättigter Natriumhydrogencarbonat-Lösung wird mit Essigester extrahiert, die organische Phase abgetrennt, über Natriumsulfat getrocknet und das Lösungsmittel im Vakuum entfernt. Der Rückstand (154 mg) wird durch präparative HPLC (Waters 2996 Photodiode Array Detector, 245nm, x Terra Prep MS C18 Column 5µm 19x100mm, Flow 20ml/min Wasser/Acetonitril) gereinigt. Man erhält 53 mg 4'-O-(Piperazin-1-ylacetyl)-avermectin B1 Monosaccharid.
LC-MS: 855.5 (M+H, 100%) C₄₇H₇₀N₂O₁₂ (855.072)
Retentionszeit: 10.57 min (27 min)
¹H-NMR (400 MHz, CDCl₃) ausgewählte Signale: δ (ppm) = 2.73-2.87 (m, 4H), 3.10-3.18 (m, 4H).

### Beispiel 53 4'-O-{[4-(tert-Butoxycarbonyl)piperazin-1-yl]acetyl}-avermectin B1 Monosaccharid (-C₂₂R¹-A-C₂₃R²- : -HC=CH-)

Zu einer Lösung von 221 mg 5-O-tert-Butyldimethylsilyl-4'-O-{[4-(tert-Butoxycarbonyl)piperazin-1-yl]acetyl}-avermectin B1 Monosaccharid (VI-3) in absolutem Methanol werden 48 mg 4-Toluolsulfonsäure in drei Portionen im Abstand von 3-4 Stunden gegeben und der Ansatz bei Raumtemperatur bis zum vollständigen Umsatz gerührt. Nach Zugabe von gesättigter Natriumhydrogencarbonat-Lösung wird mit Essigester extrahiert, die organische Phase abgetrennt, über Natriumsulfat getrocknet und das Lösungsmittel im Vakuum entfernt. Man erhält 163 mg 4'-O-{[4-(tert-Butoxycarbonyl)piperazin-1-yl]acetyl}-avermectin B1 Monosaccharid.
LC-MS: 955.6 (M+H, 100%) C₅₂H₇₈N₂O₁₄, MW: 955.188
Retentionszeit: 14.18 min (27 min)
¹H-NMR (400 MHz, CDCl₃) ausgewählte Signale: δ (ppm) = 1.46 (s, 9H), 2.49-2.60 (m, 5H), 3.28-3.32 (m, 3H), 3.46-3.52 (m, 5H).

### 5-O-tert-Butyldimethylsilyl-4'-O-{[4-(tert-butoxycarbonyl)piperazin-1-yl]acetyl}-avermectin B1 Monosaccharid (-C₂₂R¹-A-C₂₃R²- : -HC=CH-) (VI-3)

Man löst 200 mg 5-O-tert-Butyldimethylsilyl-avermectin B₁ Monosaccharid (IV-1) in 5 ml Dichlormethan. Unter Argon werden 136 mg N-(3-Dimethylaminopropyl)-N'-ethylcarbodiimid, 87 mg 4-Dimethylaminopyridin (DMAP), 199 mg 2-(1-tert-Butoxycarbonyl-piperazin-4-yl)essigsäure und etwas Molsieb zugegeben und der Ansatz 4 Stunden bei Raumtemperatur gerührt. Nach Zugabe von gesättigter Natriumhydrogencarbonat-Lösung wird die org. Phase über eine semipermeable Kartusche von der wässrigen Phase abgetrennt, anschließend über eine Natriumsulfat/Kieselgel-Kartusche filtriert und das Lösungsmittel im Vakuum entfernt. Man erhält 221 mg 5-O-tert-Butyldimethylsilyl-4'-O-{[4-(tert-butoxycarbonyl)piperazin-1-yl]acetyl}-avermectin B1 Monosaccharid (VI-3).
LC-MS: 1069.6 (M+H, 100%) C₅₈H₉₂N₂O₁₄Si (1069.45)
Retentionszeit: 20.18 min (27 min)

Analog können die in der nachstehenden Tabelle (Tabelle 4) aufgeführten Verbindungen der Formel (I) mit R⁵ = H hergestellt werden.

**Tabelle 4**

| **Bsp.-Nr.** | **-C₂₂R¹-A-C₂₃R²-** | **R³** | **R⁴** | **Physikalische Daten ^{a)}** |
|---|---|---|---|---|
| **103** | -H₂C-CH₂- | sec-Bu | 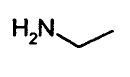 | δ (ppm) = 3.49 (d, J = 5.5, 2H), 4.1 (breit, 1-2H); m/z = 770 .5 (M - 18, 100), 788 (M + H, 10); Rₜ = 10.66 (27) |
| **104** | -HC=CH- | sec-Bu | 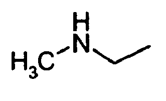 | δ (ppm) = 2.47 (s, 3H), 3.39-3.45 (m, 5H); m/z= 800.2 (M+H, 100); Rₜ= 10.43 (27) |
| **105** | -HC=CH- | sec-Bu | 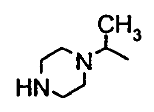 | m/z = 869 (M+H, 100); Rₜ = 9.67 (27) |

| | | | | |
|---|---|---|---|---|
| ^{a)} Bei LC-MS wird die Retentionszeit (Rₜ) sowie in Klammern die Gesamtlaufzeit des Chromatogramms in Minuten angegeben; die Prozentangabe der gefundenen Masse bezieht sich auf die relative Intensität, normiert auf 100. Zur Beschreibung der ¹H-NMR-Signale (ausgewählte Signale) werden als Abkürzungen verwendet: s (Singulett), d (Dublett), t (Triplett), q (Quartett), sym (symmetrisch). | | | | |

### Methode D:

### Beispiel 106 4'-O-(2-Morpholin-4-ylpropanoyl)-avermectin B1 Monosaccharid Benzoat (-C₂₂R¹-A-C₂₃R²- : -HC=CH-)

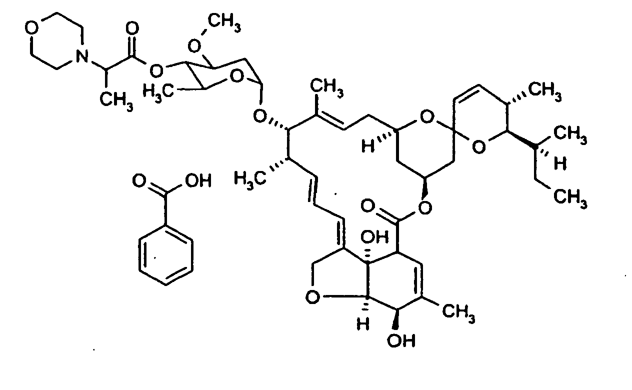

Man löst 4 mg Benzoesäure in 3 ml Dichlormethan. Nach Zugabe von 30 mg 4'-O-(2-Morpholin-4-ylpropanoyl)-avermectin B1 Monosaccharid (Verbindung aus Bsp. 52, hergestellt nach Methode A) wird kurz gerührt, das Lösungsmittel im Vakuum entfernt und der Rückstand im Hochvakuum getrocknet. Man erhält 33 mg 4'-O-(2-Morpholin-4-ylpropanoyl)-avermectin B1 Monosaccharid Benzoat.
LC-MS: 870 (M+H, 100) C₄₈H₇₁NO₁₃*C₇H₆O₂ (992.205)
Retentionszeit: 12.02 min (27 min)
¹H-NMR (400 MHz, CDCl₃) ausgewählte Signale: δ (ppm) = 2.61-2.72 (m, 4H), 3.27-3.33 (m, 2H), 3.64-3.79 (m, 5H), 7.45-7.51 (m, 2H), 7.58-7.62 (m, 1H), 8.10 (dd, J = 8.3, 1.3, 2H).

Analog kann die nachfolgend aufgeführte Verbindung der Formel (I, R⁵ = H) hergestellt werden:

### Beispiel 107 4'-O-(Piperazin-1-ylacetyl)-avermectin B1 Monosaccharid Dibenzoat

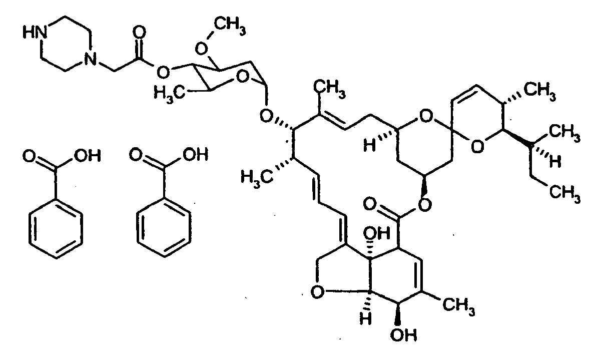

LC-MS: 855 (M+H, 100) C₄₇H₇₀N₂O₁₂*2 C₇H₆O₂ (1099.32)
Retentionszeit: 10.04 min (27 min)

### Herstellung der Ausgangsverbindungen der Formel (V)

### V-1 (2S)-2-(1H-Pyrazol-1-yl)propansäure

a) Zu einer Lösung aus 82 mg Pyrazol in 5 ml Dichlormethan werden 200 mg N-Ethyl-diisopropylamin ("Hünig's Base") gegeben und 300 mg (2*R*)-2-{[(Trifluoromethyl)-sulfonyl]oxy}propansäure-ethylester in 2 ml Dichlormethan zugetropft. Man rührt weitere 16 Stunden bei Raumtemperatur. Nach Zugabe von gesättigter Natrium-hydrogencarbonat-Lösung wird mit Essigester extrahiert, die organische Phase über Natriumsulfat getrocknet und das Lösungsmittel im Vakuum entfernt. Man erhält 250 mg *(2S)*-2-(1H-Pyrazol-1-yl)propansäureethylester in 96% Reinheit (LCMS).
   LC-MS: 169.1 (M+H, 100%).C₈H₁₂N₂O₂ (168.195)
   Retentionszeit: 3.13 min (12 min)
   ¹H-NMR (400 MHz, CD₃CN): δ (ppm) = 1.19 (t, J = 7.1, 3H), 1.70 (d, J = 7.3, 3H), 4.13 (dq, J = 7.1, 2.1, 2H), 5.10 (q, J = 7.3, 1H), 6.28 (t, J = 2.3, 1H), 7.46 (d, J = 1.4, 1H), 7.62 (dd, J = 2.3, 0.4, 1H).
b) Zu einer Lösung von 250 mg *(2S)*-2-(1H-Pyrazol-1-yl)propansäureethylester in 3 ml Tetrahydrofuran werden 62 mg Lithiumhydroxid und 1 ml Wasser gegeben. Man rührt 4 Stunden bei Raumtemperatur. Nach Zugabe von 1N wässriger Salzsäure wird mit Essigester extrahiert, die organische Phase über Natriumsulfat getrocknet und das Lösungsmittel im Vakuum entfernt. Man erhält 195 mg der *(2S)*-2-(1H-Pyrazol-1-yl)propansäure (V-1) in 93% Reinheit (LC-MS).
   LC-MS: 141.1 (M+H, 100%) C₆H₈N₂O₂ (140.141)
   Retentionszeit: 1.12 min (12 min)
   ¹H-NMR (400 MHz, DMSO-d₆): δ (ppm) = 1.64 (d, J = 7.3, 3H), 5.12 (q, J = 7.3, 1H), 6.26 (t, J = 2.2, 1H), 7.44 (dd, J = 1.2, 0.5, 1H), 7.79 (dd, J = 1.8, 0.5, 1H), 12.9 (s, breit, 1H).

Analog können die in der nachstehenden Tabelle (Tabelle 5) aufgeführten Verbindungen der Formel (V) mit LG=OH) hergestellt werden.

**Tabelle 5**

| **Bsp.-Nr.** | **R⁴** | **Physikalische Daten ^{a)}** |
|---|---|---|
| **V-2** | 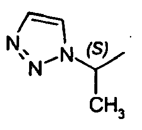 | LC-MS: m/z=142.1 (M+H, 100%) Rₜ: 0.85 min (12 min) C₅H₇N₃O₂ (141.129) |
| **V-3** | 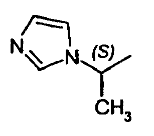 | LC-MS: m/z=141.1 (M+H, 100%) Rₜ: 0.41 min (12 min) C₆H₈N₂O₂ (140.141) |
| **V-4** | 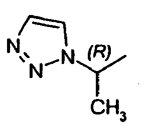 | LC-MS: m/z = 142.1 (M+H, 100%) Rₜ: 0.50 min (12 min) C₅H₇N₃O₂ (141.129) |
| **V-5** | 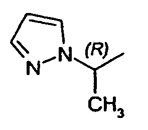 | LC-MS: m/z = 141.1 (M+H, 100%) Rₜ: 0.96 min (12 min) C₆H₈N₂O₂ (140.141) |

| | | |
|---|---|---|
| ^{a)} Bei LC-MS wird die Retentionszeit (Rₜ) sowie in Klammern die Gesamtlaufzeit des Chromatogramms in Minuten angegeben; die Prozentangabe der gefundenen Masse bezieht sich auf die relative Intensität, normiert auf 100. | | |

### V-6 (3,4-Dimethyl-5-oxo-4,5-dihydro-1H-1,2,4-triazol-1-yl)essigsäure

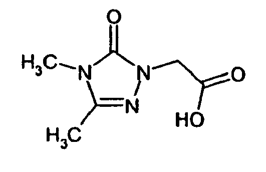

Man löst 593 mg (3,4-Dimethyl-5-oxo-4,5-dihydro-1H-1,2,4-triazol-1-yl)essigsäuremethylester (Va-6) in 5 ml Tetrahydrofuran und gibt 134 mg Lithiumhydroxid in 2 ml Wasser zu. Der Ansatz wird 16 Stunden bei Raumtemperatur gerührt, mit 1N Salzsäure angesäuert und mit Essigester extrahiert. Die organische Phase wird über Natriumsulfat getrocknet und das Lösungsmittel im Vakuum entfernt. Man erhält 167 mg (3,4-Dimethyl-5-oxo-4,5-dihydro-1H-1,2,4-triazol-1-yl)essigsäure.
LC-MS: 172 (M+H, 100%) C₆H₉N₃O₃ (171.155)
Retentionszeit: 0.45 min (12 min)

### Va-6 (3,4-Dimethyl-5-oxo-4,5-dihydro-1H-1,2,4-triazol-1-yl)essigsäuremethylester

Zu einer Lösung von 500 mg 4,5-Dimethyl-2,4-dihydro-3H-1,2,4-triazol-3-on in 15 ml Acetonitril werden 1222 mg Kaliumcarbonat gegeben und der Ansatz auf 50°C erwärmt. Nach Zugabe von 879 mg Bromessigsäuremethylester wird 16 Stunden auf Rückfluss erhitzt. Der Ansatz wird im Vakuum eingeengt, der Rückstand in Dichlormethan aufgenommen, mit verdünnter Salzsäure (pH 2) gewaschen, über Natriumsulfat getrocknet und das Lösungsmittel im Vakuum entfernt. Man erhält 593 mg (3,4-Dimethyl-5-oxo-4,5-dihydro-1H-1,2,4-triazol-1-yl)essigsäuremethylester (Va-6) in 93% Reinheit (LCMS).
LC-MS: 186 (M+H, 100%) C₇H₁₁N₃O₃ (185.182)
Retentionszeit: 0.58 min (12 min)

Analog können die in der nachstehenden Tabelle (Tabelle 6) aufgeführten Verbindungen der Fromel (V) mit LG = OH hergestellt werden.

**Tabelle 6**

| **Bsp.-Nr.** | **R⁴** | **Physikalische Daten ^{a)}** |
|---|---|---|
| **V-7** | 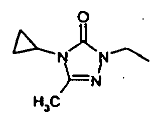 | LC-MS: m/z= 198 (M+H) Rₜ: 0.81 min (12 min) C₈H₁₁N₃O₃ (197.193) |
| **V-8** | 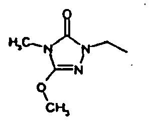 | LC-MS: m/z= 188 (M+H) Rₜ: 0.71 min (12 min) C₆H₉N₃O₄ (187.154) |

| | | |
|---|---|---|
| ^{a)} Bei LC-MS wird die Retentionszeit (Rₜ) sowie in Klammern die Gesamtlaufzeit des Chromatogramms in Minuten angegeben; die Prozentangabe der gefundenen Masse bezieht sich auf die relative Intensität, normiert auf 100. | | |

### V-9 2-(2-Metboxyethyl)-5-methyl-1,3-dioxan-5-carbonsäure

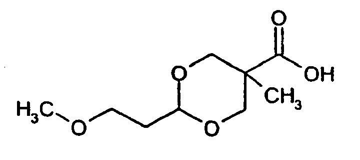

Man löst 478 mg 2-(2-Methoxyethyl)-5-methyl-1,3-dioxan-5-carbonsäuremethylester (Va-9) in 8 ml Tetrahydrofuran und gibt 92 mg Lithiumhydroxid in 2 ml Wasser gelöst hinzu. Der Ansatz wird 16 Stunden bei Raumtemperatur gerührt, 92 mg Lithiumhydroxid in 2 ml Wasser gelöst zugegeben und 24 Stunden gerührt. Mit 1N Salzsäure wird sauer gestellt, mit Essigester extrahiert und das Lösungsmittel der organischen Phase im Vakuum entfernt. Der Rückstand wird mit Wasser versetzt, mit 1N Natronlauge basisch gestellt und mit Essigester extrahiert. Die wässrige Phase wird mit 1N Salzsäure sauer gestellt, mit Essigester extrahiert, die organische Phase abgetrennt, über Natriumsulfat getrocknet und das Lösungsmittel im Vakuum entfernt. Man erhält 261 mg 2-(2-Methoxyethyl)-5-methyl-1,3-dioxan-5-carbonsäure als Gemisch von zwei isomeren Formen in ca. 78% Reinheit (LCMS).
LC-MS: 205 (M+H, 100%) C₉H₁₆O₅ (204.22)
Retentionszeit: 2.27 min und 2.70 min (12 min)

### Va-9 2-(2-Methoxyethyl)-5-methyl-1,3-dioxan-5-carbonsäuremethylester

Zu einer Lösung von 400 mg 3-Hydroxy-2-(hydroxymethyl)-2-methylpropansäuremethylester und 362 mg 1,1,3-Trimethoxypropan in Toluol werden 51 mg 4-Toluolsulfonsäure gegeben und der Ansatz 4 Stunden bei 80°C gerührt. Nach Zugabe von gesättigter Natriumhydrogencarbonat-Lösung wird die organische Phase abgetrennt, über Natriumsulfat getrocknet und das Lösungsmittel im Vakuum entfernt. Der Rückstand wird über Kieselgel (Eluent Cyclohexan/Essigester 4:1) filtriert. Man erhält 478 mg 2-(2-Methoxyethyl)-5-methyl-1,3-dioxan-5-carbonsäuremethylester als Gemisch von zwei isomeren Formen in ca. 38% Reinheit (LCMS).
LC-MS: 219 (M+H, 100%) C₁₀H₁₈O₅ (218.247)
Retentionszeit: 3.39 min und 4.16 min (12 min)

### V-10 4-(Ethoxycarbonyl)-3-(trifluoromethyl)-1H-pyrazol-1-yl-essigsäure

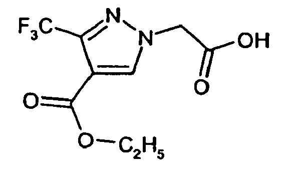

Eine Lösung von 742 mg 1-(2-tert-Butoxy-2-oxoethyl)-3-(trifluoromethyl)-1H-pyrazol-4-carbonsäureethylester (Va-10) in 0.35 ml Dichlormethan wird mit 0.35 ml Trifluoressigsäure versetzt und 90 min bei Raumtemperatur gerührt. Nach Zugabe von 0.2 ml Dichlormethan und 0.2 ml Trifluoressigsäure wird 5 Stunden gerührt und das Lösungsmittel im Vakuum entfernt. Der ölige Rückstand erstarrt beim Stehen zu einem Feststoff. Man erhält 780 mg 4-(Ethoxycarbonyl)-3-(trifluoromethyl)-1H-pyrazol-1-yl-essigsäure in ca. 50% Reinheit (LCMS).
LC-MS: 267 (M +H, 100%) C₉H₉F₃N₂O₄ (266.174)
Retentionszeit: 2.01 min (6 min)
¹H-NMR (400 MHz, D₆-DMSO): δ (ppm) = 1.28 (t, J = 7.1, 3H), 4.26 (q, J = 7.1, 2H), 5.10 (s, 2H), 8.53 (d, J = 0.9, 1H).

### Va-101-(2-tert-Butoxy-2-oxoethyl)-3-(trifluoromethyl)-1H-pyrazol-4-carbonsäureethylester

Zu einer Lösung von 50 mg 3-(Trifluoromethyl)-1H-pyrazol-4-carbonsäureethylester in 10 ml Dichlormethan werden 404 mg Hünigbase gegeben und 469 mg Bromessigsäure-tert.-butylester in 2 ml Dichlormethan gelöst zugetropft. Man rührt 16 Stunden bei Raumtemperatur. Nach Zugabe von gesättigter Natriumhydrogencarbonat-Lösung wird mit Essigester extrahiert, die organische Phase abgetrennt, über Natriumsulfat getrocknet und das Lösungsmittel im Vakuum entfernt. Man erhält 742 mg 1-(2-tert-Butoxy-2-oxoethyl)-3-(trifluoromethyl)-1H-pyrazol-4-carbonsäureethylester (Va-10) in ca. 78% Reinheit (LCMS).
LC-MS: 267 (M - tert.-Butyl, 100%) C₁₃H₁₇F₃N₂O₄ (322.281)
Retentionszeit: 3.27 min (6 min)

### Biologische Vergleichsbeipiele

### I) Verbindungen der Formel (I); -C₂₂R¹-A-C₂₃R²- steht für eine -HC=CH- Gruppierung

### Beispiel A

### Lucilia cuprina-Test (LUCICU)

### Beschreibung für Beispiele A und B

| | |
|---|---|
| Lösungsmittel: | Dimethylsulfoxid |

Zur Herstellung einer zweckmäßigen Wirkstoffzubereitung vermischt man 1 Gewichtsteil Wirkstoff mit der angegebenen Menge Wasser und verdünnt das Konzentrat mit Wasser auf die gewünschte Konzentration.

Gefäße, die Pferdefleisch enthalten, das mit der Wirkstoffzubereitung der gewünschten Konzentration behandelt wurde, werden mit *Lucilia cuprina* Larven besetzt.

Nach der gewünschten Zeit wird die Abtötung in % bestimmt. Dabei bedeutet 100 %, dass alle Larven abgetötet wurden; 0 % bedeutet, dass keine Larven abgetötet wurden.

Bei diesem Test zeigen z.B. die folgenden Verbindungen der Herstellungsbeispiele gute Wirksamkeit: siehe Tabelle

### Lucilia cuprina-Test (LUCICU)

| **Beispiel-Nr.** | **Wirkstoffkonzentration in ppm** | **Abtötungsgrad in % nach 2 Tagen** |
|---|---|---|
| 4'-O-Acetyl-4'-O-de(2,6-dideoxy-3-O-methyl-α-L-arabino-hexopyranosyl)-5-O-demethyl-avermectin A1a ^{a)} | 0.8 | 30 |
| **1** | 0.8 | 100 |
| **4** | 0.8 | 100 |
| **5** | 0.8 | 100 |
| **6** | 0.8 | 98 |
| **7** | 0.8 | 98 |
| **8** | 0.8 | 100 |
| **9** | 0.8 | 100 |
| **11** | 0.8 | 100 |
| **12** | 0.8 | 100 |
| **14** | 0.8 | 100 |
| **15** | 0.8 | 100 |
| **16** | 0.8 | 100 |
| **20** | 0.8 | 100 |
| **25** | 0.8 | 95 |
| **26** | 0.8 | 100 |
| **27** | 0.8 | 100 |
| **48** | 0.8 | 100 |
| **50** | 0.8 | 100 |
| **52** | 0.8 | 100 |
| **54** | 0.8 | 80 |
| **55** | 0.8 | 90 |
| **57** | 0.8 | 100 |
| **60** | 0.8 | 90 |
| **98** | 0.8 | 95 |
| **100** | 0.8 | 100 |

| | | |
|---|---|---|
| ^{a)} bekannt aus US 4,201,861 und JP 54-06197 | | |

### Beispiel B

### Lucilia cuprina-Test (LUCICU)

| **Beispiel Nr.** | **Wirkstoffkonzentration in ppm** | **Abtötungsgrad in % nach 2 Tagen** |
|---|---|---|
| 4'-O-Acetyl-4'-O-de(2,6-dideoxy-3-O-methyl-a-L-arabino-hexopyranosyl)-5-O-demethyl-avermectin A1a^{a)} | 0.16 | 0 |
| **18** | 0.16 | 100 |
| **19** | 0.16 | 100 |

| | | |
|---|---|---|
| ^{a)} bekannt aus US 4,201,861 und JP 54-06197 | | |

### Beispiel C

### Musca domestica-Test (MUSCDO)

| | |
|---|---|
| Lösungsmittel: | Dimethylsulfoxid |

Zur Herstellung einer zweckmäßigen Wirkstoffzubereitung vermischt man 1 Gewichtsteil Wirkstoff mit der angegebenen Menge Wasser und verdünnt das Konzentrat mit Wasser auf die gewünschte Konzentration.

Gefäße, die einen Schwamm enthalten, der mit der Wirkstoffzubereitung der gewünschten Konzentration behandelt wurde, werden mit *Musca domestica* Adulten besetzt.

Nach der gewünschten Zeit wird die Abtötung in % bestimmt. Dabei bedeutet 100 %, dass alle Fliegen abgetötet wurden; 0 % bedeutet, dass keine Fliegen abgetötet wurden.

Bei diesem Test zeigen z.B. die folgenden Verbindungen der Herstellungsbeispiele gute Wirksamkeit: siehe Tabelle

### Musca domestica-Test (MUSCDO)

| **Beispiel Nr.** | **Wirkstoffkonzentration in ppm** | **Abtötungsgrad in % nach 2 Tagen** |
|---|---|---|
| 4'-O-Acetyl-4'-O-de(2,6-dideoxy-3-O-methyl-α-L-arabino-hexopyranosyl)-5-O-demethyl-avermectin A1a^{a)} | 100 | 60 |
| **48** | 100 | 100 |
| **50** | 100 | 90 |
| **57** | 100 | 90 |
| **60** | 100 | 100 |
| **55** | 100 | 90 |
| **89** | 100 | 100 |
| **90** | 100 | 100 |
| **91** | 100 | 90 |
| **92** | 100 | 90 |
| **93** | 100 | 100 |
| **94** | 100 | 80 |
| **95** | 100 | 80 |
| 4'-O-Acetyl-4'-O-de(2,6-dideoxy-3-O-methyl-α-L-arabino-hexopyranosyl)-5-O-demethyl-avermectin A1a^{a)} | 0.8 | 0 |
| **13** | 0.8 | 50 |

| | | |
|---|---|---|
| ^{a)} bekannt aus US 4,201,861 und JP 54-06197 | | |

### Beispiel D

### Phaedon-Test (PHAECO Spritzbehandlung)

| | | |
|---|---|---|
| Lösungsmittel: | 78 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele gute Wirksamkeit: siehe Tabelle

### Phaedon-Test (PHAECO Spritzbehandlung)

| **Beispiel Nr.** | **Wirkstoffkonzentration in ppm** | **Abtötungsgrad in % nach 7 Tagen** |
|---|---|---|
| 4'-O-Acetyl-4'-O-de(2,6-dideoxy-3-O-methyl-α-L-arabino-hexopyranosyl)-5-O-demethyl-avermectin A1a^{a)} | 4 | 0 |
| **1** | 4 | 90 |
| **3** | 4 | 80 |
| **20** | 4 | 80 |

| | | |
|---|---|---|
| ^{a)} bekannt aus US 4,201,861 und JP 54-06197 | | |

### Beispiel E

### Bemisia tabaci -Test, normal sensibler Stamm, (BEMITA)

| | | |
|---|---|---|
| Lösungsmittel: | 7 | Gewichtsteile Dimethylformamid |
| Emulgator: | 10 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Baumwollpflanzen (*Gossypium hirsutum*), die von Eiern, Larven und Puparien der Weißen Fliege (*Bemisia tabaci*) befallen sind, werden durch Sprühen mit der Wirkstoffzubereitung in der gewünschten Konzentration behandelt.

Nach der gewünschten Zeit wird die Abtötung in % bestimmt. Dabei bedeutet 100 %, dass alle Tiere abgetötet wurden; 0 % bedeutet, dass keine Tiere abgetötet wurden.

Bei diesem Test zeigen z. B. die folgenden Verbindungen der Herstellungsbeispiele gute Wirksamkeit: siehe Tabelle

### Bemisia tabaci -Test, normal sensibler Stamm, (BEMITA)

| **Beispiel Nr.** | **Wirkstoffkonzentration in g/ha** | **Abtötungsgrad in % nach 7 Tagen** |
|---|---|---|
| 4'-O-Acetyl-4'-O-de(2,6-dideoxy-3-O-methyl-α-L-arabino-hexopyranosyl)-5-O-demethyl-avermectin A1a^{a)} | 0.8 | 0 |
| **14** | 0.8 | 80 |

| | | |
|---|---|---|
| ^{a)} bekannt aus US 4,201,861 und JP 54-06197 | | |

### Beispiel F

### Tetranychus-Test, OP-resistent (TETRUR Spritzbehandlung)

| | | |
|---|---|---|
| Lösungsmittel: | 78 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele gute Wirksamkeit: siehe Tabelle

### Tetranychus-Test, OP-resistent (TETRUR Spritzbehandlung)

| **Beispiel Nr.** | **Wirkstoffkonzentration in g/ha** | **Abtötungsgrad in % nach 5 Tagen** |
|---|---|---|
| 4'-O-Acetyl-4'-O-de(2,6-dideoxy-3-O-methyl-α-L-arabino-hexopyrano-syl)-5-O-demethylavermectin A1a^{a)} | 0.00128 | 0 |
| **2** | 0.00128 | 80 |

| | | |
|---|---|---|
| ^{a)} bekannt aus US 4,201,861 und JP 54-06197 | | |

### II) Verbindungen der Formel (I); -C₂₂R¹-A-C₂₃R²- steht für eine -H₂C-CH₂- Gruppierung

### Beispiel G

### Phaedon-Test (PHAECO Spritzbehandlung)

| | | |
|---|---|---|
| Lösungsmittel: | 78 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis*) werden mit einer Wirkstoffzubereitung der gewünschten Konzentration gespritzt und nach dem Abtrocknen mit Larven des Meerrettichblattkäfers *(Phaedon cochleariae*) besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele gute Wirksamkeit: siehe Tabelle

### Phaedon-Test (PHAECO Spritzbehandlung)

| **Beispiel Nr.** | **Wirkstoffkonzentration in g/ha** | **Abtötungsgrad in % nach 7 Tagen** |
|---|---|---|
| 4'-O-Acetyl-4'-O-de(2,6-dideoxy-3-O-methyl-α-L-arabino-hexopyrano-syl)-5-O-demethyl-25-de(1-methyl-propyl)-22,23-dihydro-25-(1-methylethyl)-avermectin A1a^{a)} | 4 | 0 |
| **17** | 4 | 100 |
| **18** | 4 | 100 |
| **21** | 4 | 83 |
| **22** | 4 | 100 |
| **23** | 4 | 100 |
| **24** | 4 | 83 |
| **28** | 4 | 100 |
| **29** | 4 | 100 |
| **31** | 4 | 100 |
| **32** | 4 | 100 |
| **33** | 4 | 100 |
| **34** | 4 | 100 |
| **35** | 4 | 100 |
| **36** | 4 | 100 |
| **37** | 4 | 100 |
| **40** | 4 | 100 |
| **41** | 4 | 83 |
| **42** | 4 | 83 |
| **43** | 4 | 100 |
| **44** | 4 | 100 |
| **45** | 4 | 100 |
| **48** | 4 | 100 |
| **50** | 4 | 83 |
| **51** | 4 | 100 |
| **52** | 4 | 100 |
| **53** | 4 | 100 |
| **54** | 4 | 100 |
| **56** | 4 | 100 |
| **57** | 4 | 100 |
| **58** | 4 | 100 |
| **59** | 4 | 100 |
| **60** | 4 | 100 |
| **61** | 4 | 100 |
| **62** | 4 | 100 |
| **63** | 4 | 100 |
| **64** | 4 | 100 |
| **65** | 4 | 100 |
| **66** | 4 | 100 |
| **67** | 4 | 100 |
| **68** | 4 | 100 |
| **69** | 4 | 100 |
| **70** | 4 | 100 |
| **71** | 4 | 100 |
| **72** | 4 | 100 |
| **73** | 4 | 100 |
| **74** | 4 | 100 |
| **75** | 4 | 100 |
| **76** | 4 | 100 |
| **77** | 4 | 100 |
| **78** | 4 | 100 |
| **79** | 4 | 100 |
| **80** | 4 | 100 |
| **81** | 4 | 100 |
| **82** | 4 | 100 |
| **83** | 4 | 100 |
| **84** | 4 | 100 |
| **85** | 4 | 83 |
| **87** | 4 | 100 |
| **88** | 4 | 100 |
| **96** | 4 | 83 |
| **101** | 4 | 100 |
| **103** | 4 | 100 |
| **104** | 4 | 100 |
| **105** | 4 | 100 |
| **106** | 4 | 100 |
| **107** | 4 | 100 |

| | | |
|---|---|---|
| ^{a)} bekannt aus EP 0 235 085 A1 | | |

### Beispiel H

### Tetranychus-Test, OP-resistent (TETRUR Spritzbehandlung)

| | | |
|---|---|---|
| Lösungsmittel: | 78 | Gewichtsteile Aceton |
| | | |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele gute Wirksamkeit: siehe Tabelle

### Tetranychus-Test, OP-resistent (TETRUR Spritzbehandlung)

| **Beispiel** | **Wirkstoffkonzentration in g/ha** | **Abtötungsgrad in % nach 5 Tagen** |
|---|---|---|
| 4'-O-Acetyl-4'-O-de(2,6-dideoxy-3-O-methyl-α-L-arabino-hexopyrano-syl)-5-O-demethyl-25-de(1-methyl-propyl)-22,23-dihydro-25-(1-me-thylethyl)-avermectin A1a^{a)} | 0.032 | 0 |
| **17** | 0.032 | 70 |
| **21** | 0.032 | 70 |
| **23** | 0.032 | 70 |

| | | |
|---|---|---|
| ^{a)} bekannt aus EP 0 235 085 A1 | | |

### Beispiel I

### Spodoptera frugiperda-Test (SPODFR Spritzbehandlung)

| | | |
|---|---|---|
| Lösungsmittel: | 78,0 | Gewichtsteile Aceton |
| | | |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach der gewünschten Zeit wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele gute Wirksamkeit: siehe Tabelle

| **Beispiel** | **Wirkstoffkonzentration in g/ha** | **Abtötungsgrad in % nach 5 Tagen** |
|---|---|---|
| 4'-O-Aectyl-4'-O-de(2,6-dideoxy-3-O-methyl-α-L-arabino-hexopyrano-syl)-5-O-demethyl-25-de(1-methyl-propyl)-22,23-dihydro-25-(1-me-thylethyl)-avermectin A1a^{a)} | 4 | 0 |
| **28** | 4 | 100 |
| **29** | 4 | 100 |
| **30** | 4 | 100 |
| **32** | 4 | 100 |
| **33** | 4 | 100 |
| **34** | 4 | 100 |
| **35** | 4 | 100 |
| **36** | 4 | 83 |
| **38** | 4 | 100 |
| **39** | 4 | 100 |
| **40** | 4 | 100 |
| **42** | 4 | 100 |
| **45** | 4 | 100 |
| **46** | 4 | 100 |
| **47** | 4 | 100 |
| **48** | 4 | 100 |
| **49** | 4 | 100 |
| **50** | 4 | 100 |
| **52** | 4 | 100 |
| **59** | 4 | 100 |
| **66** | 4 | 100 |
| **67** | 4 | 100 |
| **68** | 4 | 100 |
| **69** | 4 | 100 |
| **70** | 4 | 100 |
| **71** | 4 | 100 |
| **72** | 4 | 100 |
| **74** | 4 | 100 |
| **75** | 4 | 100 |
| **76** | 4 | 100 |
| **78** | 4 | 100 |
| **80** | 4 | 100 |
| **81** | 4 | 100 |
| **82** | 4 | 100 |
| **83** | 4 | 100 |
| **84** | 4 | 100 |
| **85** | 4 | 100 |
| **86** | 4 | 100 |
| **87** | 4 | 100 |
| **88** | 4 | 100 |
| **99** | 4 | 100 |
| **103** | 4 | 100 |
| **105** | 4 | 100 |
| **106** | 4 | 100 |
| **107** | 4 | 100 |

| **Beispiel** | **Wirkstoffkonzentration in g/ha** | **Abtötungsgrad in % nach 5 Tagen** |
|---|---|---|
| 4'-O-(4-chloro-benzoyl)-4'-O-de(2,6-dideoxy-3-O-methyl-α-L-arabino-hexopyranosyl)-5-O-demethyl-avermectin A1a ^{b)} | 4 | 0 |
| **97** | 4 | 33 |

| | | |
|---|---|---|
| ^{a)} vgl. EP 0 235 085 A1, ^{b)} bekannt aus US 4,201,861 und JP 54-06197 | | |

### Beispiel J

### Ctenocephalides felis; oral (CTECFE)

| | |
|---|---|
| Lösungsmittel: | Dimethylsulfoxid |

Zwecks Herstellung einer zweckmäßigen Wirkstoffzubereitung vermischt man 1 Gewichtsteil Wirkstoff mit der angegebenen Mengen Lösungsmittel. Ein Teil des Konzentrats wird mit citiriertem Rinderblut verdünnt und die gewünschte Konzentration hergestellt.

20 nüchterne adulte Flöhe (*Ctenocephalides felis*) werden in eine Kammer eingesetzt, die oben und unten mit Gaze verschlossen ist. Auf die Kammer wird ein Metallzylinder gestellt, dessen Unterseite mit Parafilm verschlossen ist. Der Zylinder enthält die Blut-Wirkstoffzubereitung, die von den Flöhen durch die Parafilmmembran aufgenommen werden kann. Während das Blut auf 37° C erwärmt wird, ist im Bereich der Flohkammern Raumtemperatur.

Nach der gewünschten Zeit wird die Abtötung in % bestimmt. Dabei bedeutet 100 %, dass alle Flöhe abgetötet wurden; 0 % bedeutet, dass kein Floh abgetötet wurde.

Bei diesem Test zeigen z.B. die folgenden Verbindungen der Herstellungsbeispiele eine gute Wirkung: siehe Tabelle

| **Beispiel** | **Wirkstoffkonzentration in g/ha** | **Abtötungsgrad in % nach 2 Tagen** |
|---|---|---|
| 4'-O-Acetyl-4'-O-de(2,6-dideoxy-3-O-methyl-α-L-arabino-hexopyrano-syl)-5-O-demethyl-25-de(1-methyl-propyl)-22,23-dihydro-25-(1-methylethyl)-avermectin A1a ^{a)} | 4 | 20 |
| **50** | 4 | 50 |
| **52** | 4 | 50 |
| **57** | 4 | 40 |
| **60** | 4 | 50 |

## Patentansprüche

1. Avermectinderivate der Formel (I) 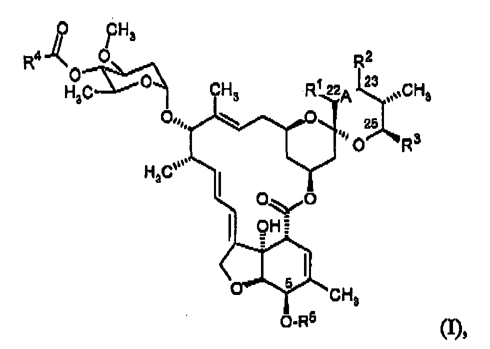 in welcher
die Gruppierung -C₂₂R¹-A-C₂₃R²- für -HC=CH-, -H₂C-CH(OH)- oder -H₂C-CH₂- steht,
R³ für sec-Butyl, iso-Propyl oder Cyclohexyl,
R⁵ für Wasserstoff, Methyl oder C₁₋₄-Alkylcarbonyl, und
R⁴ für gegebenenfalls substituiertes C₂₋₆₋Alkyl, C₂₋₆₋Alkenyl, C₂₋₆₋Alkinyl, C₁₋₆₋Alkoxy- C₁₋₄₋alkyl, C₁₋₄-Alkoxy-C₁₋₄-alkoxy-C₁₋₄-alkyl, Cycloalkyl, Cycloalkenyl, Cycloalkyl- C₁₋₄₋alkyl, Aryl, wobei 4-Chlorphenyl ausgenommen ist, Aryl-C₁₋₄₋alkyl, Hetaryl, Hetaryl-C₁₋₄₋alkyl, Heterocyclyl oder Heterocyclyl-C₁₋₄₋alkyl, oder für einen Rest steht, ausgewählt aus den Resten (G⁷) bis (G¹⁴) 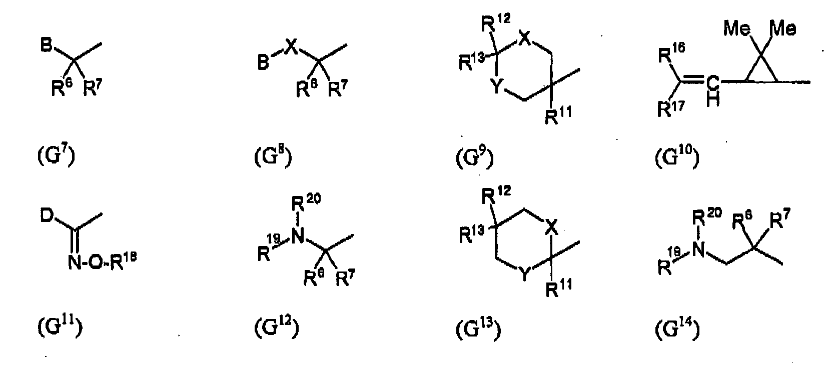
worin
B für gegebenenfalls mit R⁸, R⁹ und R¹⁰ substituiertes Aryl, Cycloalkyl, Heterocyclyl, Hetaryl oder NR¹⁹R²⁰ steht,
D für gegebenenfalls mit R⁸, R⁹ und R¹⁰ substituiertes Aryl, Cycloalkyl, Heterocyclyl, Hetaryl oder NR¹⁹R²⁰ steht,
R⁶ für Wasserstoff, Halogen, insbesondere Fluor, Cyan, gegebenenfalls substituiertes Alkyl, Alkenyl, Alkinyl, Cycloalkyl oder Heterocyclyl steht,
R⁷ für Wasserstoff, Halogen, insbesondere Fluor, Cyan, gegebenenfalls substituiertes Alkyl, Alkenyl, Alkinyl steht oder
R⁰ und R⁷ gemeinsam mit dem Atom, an das sie gebunden sind, für einen 3-, 4-, 5-, 6- oder 7- gliedrigen Ring stehen, der gegebenenfalls substituiert ist und/oder gegebenenfalls durch Sauerstoff, Schwefel, Stickstoff, Sulfinyl oder Sulfonyl unterbrochen ist, oder
R⁶ und R⁷ gemeinsam mit dem Atom, an das sie gebunden sind, für eine gegebenenfalls substituierte *exo*-Methylenbindung stehen,
R⁸ für Wasserstoff, gegebenenfalls substituiertes C₁₋₄-Alkyl, C₃₋₆-Cycloalkyl, C₁₋₄- Halogenalkyl, C₁₋₄-Alkoxy, C₁₋₄-Alkoxy-C₁₋₄-alkoxy, C₁₋₄-Halogenalkoxy, C₁₋₄- Alkylthio, C₁₋₄-Halogenalkylthio, C₁₋₄-Alkylsulfmyl, C₁₋₄-Halogenalkylsulflnyl, C₁₋₄- Alkylsulfonyl, C₁₋₄-Halogenakylsulonyl, Hetaryl, wie Pyridyl oder Thienyl, Halogen, Nitro, Cyan, Amino, C₁₋₄-Alkylamino, Di-(C₁₋₄-alkyl)-amino, steht, oder für einen Rest ausgewählt unter CO-OH, COO⁽⁻⁾, COO-C₁₋₆-alkyl, NH-CHO, NH-CO-C₁₋₄-alkoxy, N(C ₁₋₄-alkyl)-CO-C₁₋₄-alkoxy, P(O)(OH)₂, P(O)O⁽⁻⁾₂, CO-NH₂, CS-NH₂, C(=NH)-NH₂, C(=N-OH)-NH₂, CO-NH-C₁₋₄-alkyl, CO-N-(C₁₋₄-alkyl)₂, CO-NH-C₁₋₄- alkoxy, CO-NH-CO-C₁₋₄-alkyl, CO-NH-CO-C₁₋₄-halogenalkyl, CO-NH-CO-C₃₋₇- cycloalkyl, CO-NH-CO-C₁₋₄-alkoxy, CO-NH-CO-(aryl-C₁₋₂-alkyoxy), SO₂-OH, SO₂- O⁽⁻⁾, SO₂-NH₂, SO₂-NH-C₁₋₄-alkyl, SO₂-N-(C₁₋₄-alkyl)₂, CO-NH-SO₂-NH-C₁₋₄-alkyl, CO-NH-SO₂-N[di(C₁-₄-alkyl), CO-O-C₁₋₆-alkyl, steht,
R⁹ für Wasserstoff oder gegebenenfalls substituiertes C₁₋₄-Alkyl, C₁₋₄-Halogenalkyl, C₁₋₄- Alkoxy, C₁₋₄-Halogenalkoxy, C₁₋₄-Alkylthio, C₁₋₄-Alkylsulfinyl, C₁₋₄-Alkylsulfonyl, Halogen, Nitro, Cyan, Formyl, C₁₋₄-Alkylcarbonyl, Amino, C₁₋₄-Alkylamino, Di-(C₁₋₄-
R¹⁰ alkyl)-amino, gegebenenfalls substituiertes Aryl, gegebenenfalls substituiertes Hetaryl oder gegebenenfalls substituiertes Heterocyclyl steht, für Wasserstoff oder gegebenenfalls substituiertes C₁₋₄-Alkyl, C₂₋₄-Alkenyl, C₂₋₄- Alkinyl, C₁₋₄-Halogenalkyl, C₁₋₄-Alkylcarbonyl, C₁₋₄-Alkoxycarbonyl steht,
R¹¹ für Wasserstoff, Cyan oder gegebenenfalls substituiertes C₁₋₆-Alkyl steht,
R¹² und R¹³ unabhängig voneinander für Wasserstoff, Hydroxy, oder gegebenenfalls substituiertes C₁₋₄-Alkyl, C₂₋₄-Alkenyl, C₂₋₄-Alkinyl, C₁₋₆-Alkylcarbonyl, C₁₋₆- Alkylamino, Di-(C₁₋₆-alkyl)-amino, C₁₋₆-Alkylamino-C₁₋₄-alkyl, Di-(C₁₋₆-akyl)-amino- C₁₋₄-alkyl, C₁₋₆-Alkoxy-C₁₋₄-alkyl, Amino-C₁₋₄-alkyl, C₃₋₆-Cycloalkyl, Aryl, Aryl-C₁₋₄- alkyl, Hetaryl-C₁₋₄-alkyl, Heterocyclyl, Heterocyclyl-C₁₋₄-alkyl oder Hetaryl steht,
oder
R¹² und R¹³ gemeinsam mit dem Atom, an das sie gebunden sind, für einen gegebenenfalls substituierten 3-, 4-, 5-, 6- oder 7-gliedrigon Ring stehen, der gegebenenfalls durch Sauerstoff, Schwefel, Stickstoff, Sulfinyl oder Sulfonyl unterbrochen sein kann,
oder
R¹² und R¹³ gemeinsam mit dem Atom, an das sie gebunden sind, für 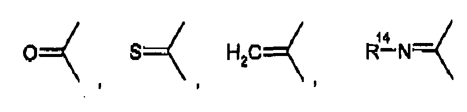 stehen,
X für Sauerstoff, Schwefel, Sulfinyl, Sulfonyl oder N-R¹⁴ steht, worin R¹⁴ für Wasserstoff oder gegebenenfalls substituiertes C₁₋₄-Alkyl steht,
Y für Sauerstoff, Schwefel, Sulfinyl, Sulfonyl oder N-R¹⁵ steht, worin R¹⁵ für Wasserstoff, gegebenenfalls substituiertes C₁₋₄-Alkyl steht,
R¹⁶ für Methyl, Chlor, Brom oder Trifluormethyl steht,
R¹⁷ für Methyl, Chlor oder Brom steht,
R¹⁸ für Wasserstoff oder gegebenenfalls substituiertes C₁₋₄-Alkyl, Aryl-C₁₋₄-alkyl oder Hetaryl-C₁₋₄-alkyl steht
R¹⁹ und R²⁰ unabhängig voneinander für Wasserstoff oder gegebenenfalls substituiertes C₁₋₄- Alkyl, C₁₋₄-Halogenalkyl, C₁₋₄-Alkoxycarbonyl, C₁₋₄-Alkylcarbonyl, C₁₋₆-Alkoxy-C₁₋₄- alkyl, C₁₋₆-Alkylamino-C₁₋₄-alkyl, Di-(C₁₋₆-alkyl)-amino-C₁₋₄-alkyl, C₁₋₄- Alkoxycarbamoyl, C₂₋₆-Alkenyl, C₂₋₆-Alkinyl, Aryl-C₁₋₄-alkyl, Hetaryl-C₁₋₄-alkyl steht,
oder
R¹⁹ und R²⁰ gemeinsam mit dem Stickstoffatom, an das sie gebunden sind, für eine cyclische Aminogruppe stehen, oder für einen 3-, 4-, 5-, 6- oder 7-gliedrigen Ring stehen, der gegebenenfalls durch Sauerstoff, Schwefel, Stickstoff, Sulfinyl oder Sulfonyl unterbrochen ist und/oder der gegebenenfalls mit mindestens einem, insbesondere 1, 2, 3 oder 4, Rest wie in R⁸, R⁹ und R¹⁰ definiert, substituiert ist,
und
wobei
Aryl für einen ein-, zwei- oder mehrkernigen aromatischen Rest steht, und
Hetaryl für einen 5-7 gliedrigen Ring mit 1-3 gleichen oder verschiedenen Heteroatomen ausgewählt aus Sauerstoff, Schwefel und Stickstoff steht, und
Heterocyclyl, für einen gesättigten oder teilweise ungesättigten 3-7 gliedrigen monocyclischen Ring mit 1-3 gleichen oder verschiedenen Heteroatomen ausgewählt aus Sauerstoff, Schwefel und Stickstoff steht oder für ein gesättigtes oder teilweise ungesättigte bicyclisches Ringsystem bestehend aus 8-14 Atomen steht, welche entweder in einem Ring oder in beiden Ringen unabhängig voneinander 1-5 gleiche oder verschiedene Heteroatome ausgewählt aus Sauerstoff, Schwefel oder Stickstoff enthalten.

2. Verfahren zur Herstellung von Avermectinderivaten der Formel (I), **dadurch gekennzeichnet, dass** Verbindungen der Formel (II) 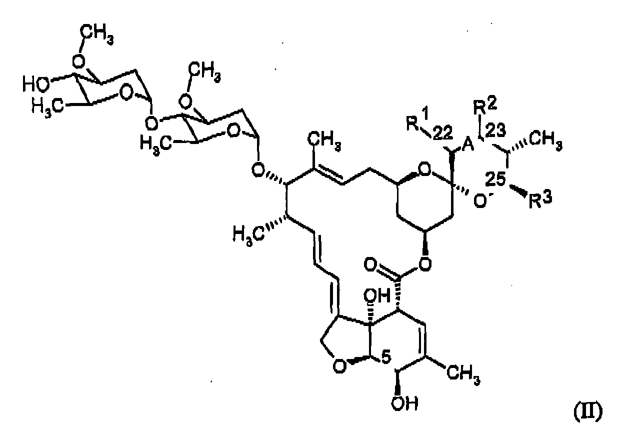 in welcher die Gruppierung -C₂₂R¹-A-C₂₃R²- und R³ die in Anspruch 1 genannten Bedeutungen haben,
in einem ersten Reaktionsschritt in Gegenwart eines Verdünnungsmittels und in Gegenwart eines sauren Reaktionshilfsmittels zu Verbindungen der Formel (III) 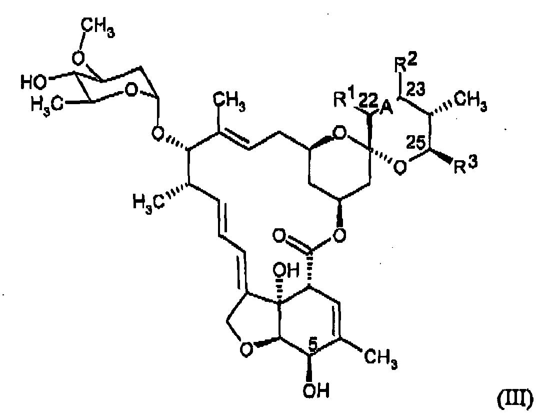 umgesetzt werden, und diese
in einem zweiten Reaktionsschritt in Gegenwart eines Vordumungsmittels und gegebenenfalls in Gegenwart eines basischen Reaktionshilfsmittels mit geeigneten Schutzgruppen in makrocyclische Lactone der Formel (IV) 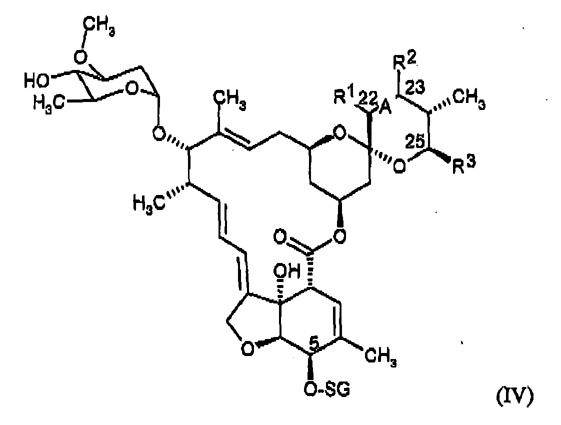 überführt werden in welcher SG für einen geeigneten Schutzgruppenrest steht, und diese dann in einem dritten Reaktionsschritt gegebenenfalls in Gegenwart eines Verdünnungsmittels und gegebenenfalls in Gegenwart eines basischen Reaktionshilfsmittels mit Verbindungen der Formel (V)
R⁴-C(=O)-LG (V)
in welcher
R⁴ eine in Anspruch 1 genannte Bedeutung hat, und
LG für eine gegebenenfalls *in-situ* erzeugte nucleofuge Abgangsgruppe (Leaving Group) steht,
zu Verbindungen der Formel (VI) 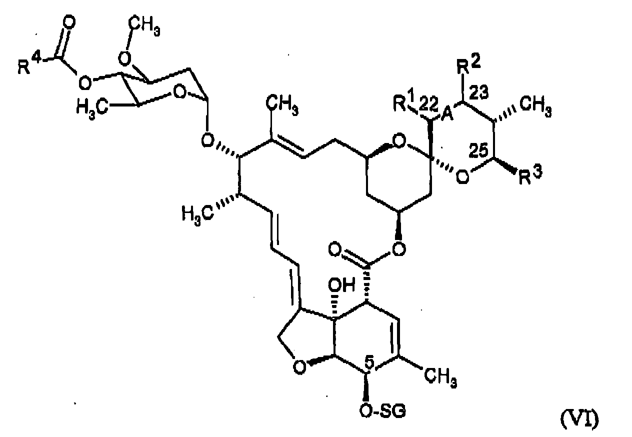 umgesetzt werden und diese
in einem vierten Reaktionsschritt unter den Reaktionsbedingungen einer Schutzgruppendeblockierung gegebenenfalls in Gegenwart eines Verdünnungsmittels und gegebenenfalls in Gegenwart eines geeigneten sauren oder basischen Reaktionshilfsmittels umsetzt.

3. Zusammensetzung umfassend mindestens ein Avermectinderivat der Formel (I) gemäß Anspruch 1 zur Bekämpfung tierischer Schädlinge.

4. Verwendung von Avermectinderivaten der Formel (I) gemäß Anspruch 1 zur Bekämpfung tierischer Schädlinge.

5. Verfahren zur Bekämpfung tierischer Schädlinge, **dadurch gekennzeichnet, dass** Avermectinderivate der Formel (I) gemäß Anspruch 1 auf tierische Schädlinge und/oder deren Lebensraum ausgebracht worden.

6. Verwendung von Avermectinderivaten der Formel (I) gemäß Anspruch 1 zur Herstellung von Zusammensetzungen zur Bekämpfung tierischer Parasiten.

7. Verwendung nach Anspruch 6, wobei die tierischen Parasiten parasitäre Arthropoden sind.

8. Verwendung von Avermectinderivaten der Formel (I) gemäß Anspruch 1 zur Behandlung von Saatgut.

9. Verwendung von Avermectinderivaten der Formel (I) gemäß Anspruch 1 zur Behandlung transgener Pflanzen.

10. Verwendung von Avermectinderivaten der Formel (I) gemäß Anspruch 1 zur Behandlung des Saatguts transgener Pflanzon.

11. Avermectinderivate der Formel (VI) gemäß Anspruch 1 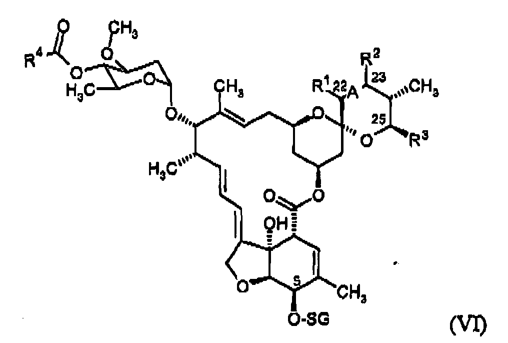 **dadurch gekennzeichnet, dass** ein Schutzgruppenrest SG vorhanden ist.

## Claims

1. Avermectin derivates of the formula (I) 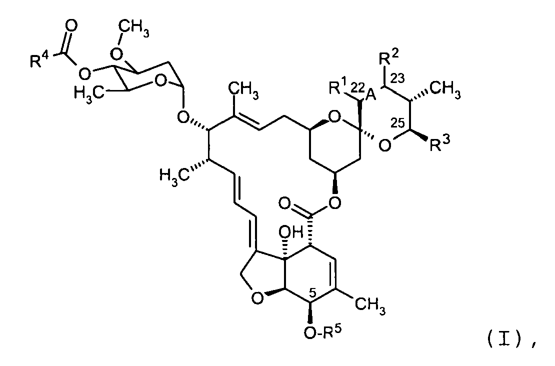 in which
the grouping -C₂₂R¹-A-C₂₃R²- represents -HC=CH-, -H₂C-CH(OH)- or -H₂C-CH₂-,
R³ represents sec-butyl, isopropyl or cyclohexyl,
R⁵ represents hydrogen, methyl or C₁₋₄-alkylcarbonyl, and
R⁴ represents optionally substituted C₂₋₆-alkyl, C₂₋₆- alkenyl, C₂₋₆-alkynyl, C₁₋₆-alkoxy-C₁₋₄-alkyl, C₁₋₄- alkoxy-C₁₋₄-alkoxy-C₁₋₄-alkyl, cycloalkyl , cycloalkenyl, cycloalkyl-C₁₋₄-alkyl, aryl, except for 4-chlorophenyl, aryl-C₁₋₄-alkyl, hetaryl, hetaryl-C₁₋₄-alkyl, heterocyclyl or heterocyclyl- C₁₋₄-alkyl, or represents a radical selected from the radicals (G⁷) to (G¹⁴) 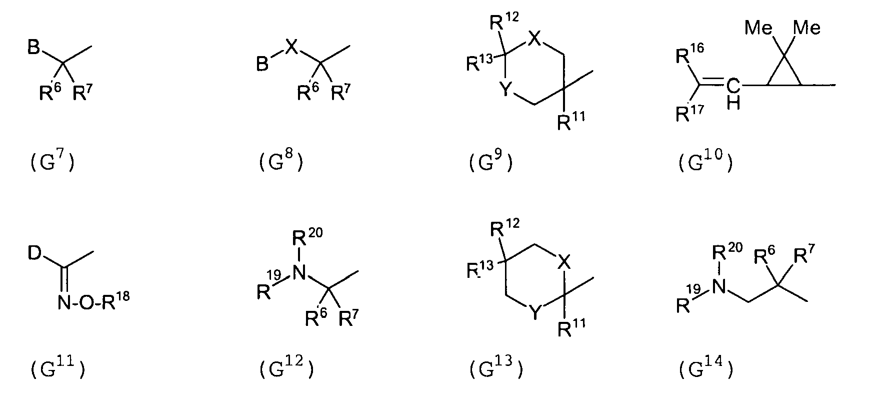
in which
B represents optionally R⁸-, R⁹- and R¹⁰-substituted aryl, cycloalkyl, heterocyclyl, hetaryl or NR¹⁹R²⁰,
D represents optionally R⁸-, R⁹- and R¹⁰-substituted aryl, cycloalkyl, heterocyclyl, hetaryl or NR¹⁹R²⁰,
R⁶ represents hydrogen, halogen, in particular fluorine, cyano, optionally substituted alkyl, alkenyl, alkynyl, cycloalkyl or heterocyclyl,
R⁷ represents hydrogen, halogen, in particular fluorine, cyano, optionally substituted alkyl, alkenyl, alkynyl or
R⁶ and R⁷ together with the atom to which they are attached represent a 3-, 4-, 5-, 6- or 7-membered ring which is optionally substituted and/or optionally interrupted by oxygen, sulphur, nitrogen, sulphinyl or sulphonyl, or
R⁶ and R⁷ together with the atom to which they are attached represent an optionally substituted exo-
R⁸ methylene bond, represents hydrogen, optionally substituted C₁₋₄- alkyl, C₃₋₆-cycloalkyl, C₁₋₄-haloalkyl, C₁₋₄-alkoxy, C₁₋₄-alkoxy-C₁₋₄-alkoxy, C₁₋₄-haloalkoxy, C₁₋₄- alkylthio, C₁₋₄-haloalkylthio, C₁₋₄-alkylsulphinyl, C₁₋₄-haloalkylsulphinyl, C₁₋₄-alkylsulphonyl, C₁₋₄- haloalkylsulphonyl, hetaryl, such as pyridyl or thienyl, halogen, nitro, cyano, amino, C₁₋₄- alkylamino, di-(C₁₋₄-alkyl)-amino, or represents a radical selected from the group consisting of CO- OH, COO⁽⁻⁾, COO-C₁₋₆-alkyl, NH-CHO, NH-CO-C₁₋₄-alkoxy, N(C₁₋₄-alkyl)-CO-C₁₋₄-alkoxy, P(O)(OH)₂, P(O)O⁽⁻⁾₂, CO- NH₂, CS-NH₂, C(=NH)-NH₂, C(=N-OH)-NH₂, CO-NH-C₁₋₄- alkyl, CO-N-(C₁₋₄-alkyl)₂, CO-NH-C₁₋₄-alkoxy, CO-NH- CO-C₁₋₄-alkyl, CO-NH-CO-C₁₋₄-haloalkyl, CO-NH-CO-C₃₋₇- cycloalkyl, CO-NH-CO-C₁₋₄-alkoxy, CO-NH-CO-(aryl-C₁₋₂- alkyloxy), SO₂-OH, SO₂-O⁽⁻⁾, SO₂-NH₂, SO₂-NH-C₁₋₄- alkyl, SO₂-N-(C₁₋₄-alkyl)₂, CO-NH-SO₂-NH-C₁₋₄-alkyl, CO-NH-SO₂-N[di(C₁₋₄-alkyl), CO-O-C₁₋₆-alkyl,
R⁹ represents hydrogen or optionally substituted C₁₋₄- alkyl, C₁₋₄-haloalkyl, C₁₋₄-alkoxy, C₁₋₄-haloalkoxy, C₁₋₄-alkylthio, C₁₋₄-alkylsulphinyl, C₁₋₄- alkylsulphonyl, halogen, nitro, cyano, formyl, C₁₋₄- alkylcarbonyl, amino, C₁₋₄-alkylamino, di-(C₁₋₄- alkyl)-amino, optionally substituted aryl, optionally substituted hetaryl or optionally substituted heterocyclyl,
R¹⁰ represents hydrogen or optionally substituted C₁₋₄- alkyl, C₂₋₄-alkenyl, C₂₋₄-alkynyl, C₁₋₄-haloalkyl, C₁₋₄- alkylcarbonyl, C₁₋₄-alkoxycarbonyl,
R¹¹ represents hydrogen, cyano or optionally substituted C₁₋₆-alkyl,
R¹² and R¹³ independently of one another represents hydrogen, hydroxyl, or optionally substituted C₁₋₄- alkyl, C₂₋₄-alkenyl, C₂₋₄-alkynyl, C₁₋₆-alkylcarbonyl, C₁₋₆-alkylamino, di-(C₁₋₆-alkyl)-amino, C₁₋₆- alkylamino-C₁₋₄-alkyl, di-(C₁₋₆-alkyl)-amino-C₁₋₄- alkyl, C₁₋₆-alkoxy-C₁₋₄-alkyl, amino-C₁₋₄-alkyl, C₃₋₆- cycloalkyl, aryl, aryl-C₁₋₄-alkyl, hetaryl-C₁₋₄- alkyl, heterocyclyl, heterocyclyl-C₁₋₄-alkyl or hetaryl,
or
R¹² and R¹³ together with the atom to which they are attached represent an optionally substituted 3-, 4-, 5-, 6- or 7-membered ring which may optionally be interrupted by oxygen, sulphur, nitrogen, sulphinyl or sulphonyl,
or
R¹² and R¹³ together with the atom to which they are attached represent 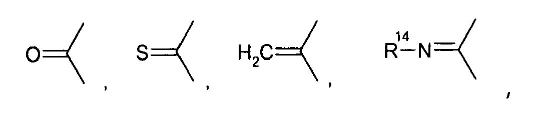
X represents oxygen, sulphur, sulphinyl, sulphonyl or N-R¹⁴, where R¹⁴ represents hydrogen or optionally substituted C₁₋₄-alkyl,
Y represents oxygen, sulphur, sulphinyl, sulphonyl or N-R¹⁵, where R¹⁵ represents hydrogen, optionally substituted C₁₋₄-alkyl,
R¹⁶ represents methyl, chlorine, bromine or trifluoromethyl,
R¹⁷ represents methyl, chlorine or bromine,
R¹⁸ represents hydrogen or optionally substituted C₁₋₄- alkyl, aryl-C₁₋₄-alkyl or hetaryl-C₁₋₄-alkyl,
R¹⁹ and R²⁰ independently of one another represent hydrogen or optionally substituted C₁₋₄-alkyl, C₁₋₄- haloalkyl, C₁₋₄-alkoxycarbonyl, C₁₋₄-alkylcarbonyl, C₁₋₆-alkoxy-C₁₋₄-alkyl, C₁₋₆-alkylamino-C₁₋₄-alkyl, di-(C₁₋₆-alkyl)-amino-C₁₋₄-alkyl, C₁₋₄-alkoxy- carbamoyl, C₂₋₆-alkenyl, C₂₋₆-alkynyl, aryl-C₁₋₄- alkyl, hetaryl-C₁₋₄-alkyl,
or
R¹⁹ and R²⁰ together with the nitrogen atom to which they are attached represent a cyclic amino group or represent a 3-, 4-, 5-, 6- or 7-membered ring which is optionally interrupted by oxygen, sulphur, nitrogen, sulphinyl or sulphonyl and/or which is optionally substituted by at least one, in particular 1, 2, 3 or 4, radical as defined in R⁸_{,} R⁹ and R¹⁰,
and
where
aryl represents a mono-, di- or polycyclic aromatic radical, and
hetaryl represents a 5-7-membered ring having 1-3 identical or different heteroatoms selected from the group consisting of oxygen, sulphur and nitrogen, and
heterocyclyl represents a saturated or partially unsaturated 3-7-membered monocyclic ring having 1-3 identical or different heteroatoms selected from the group consisting of oxygen, sulphur and nitrogen or represents a saturated or partially unsaturated bicyclic ring system consisting of 8-14 atoms and comprising either in one ring or in both rings independently of one another, 1-5 identical or different heteroatoms selected from the group consisting of oxygen, sulphur and nitrogen.

2. Process for preparing avermectin derivatives of the formula (I), **characterized in that** compounds of the formula (II) 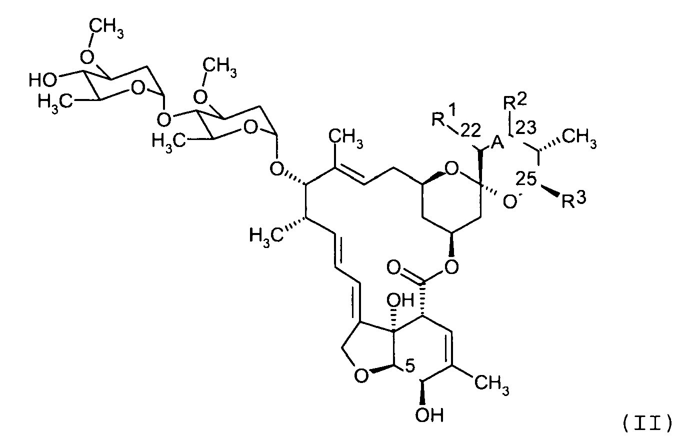 in which the grouping -C₂₂R¹-A-C₂₃R² and R³ have the meanings mentioned in Claim 1
are, in a first reaction step, converted in the presence of a diluent and in the presence of an acidic reaction auxiliary into compounds of the formula (III) 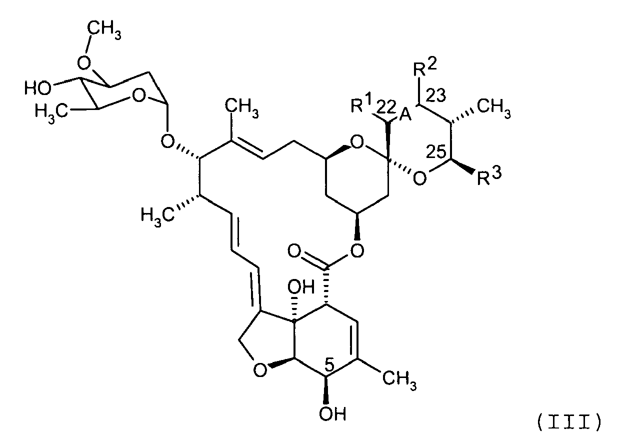 and these
are, in a second reaction step, converted in the presence of a diluent and, if appropriate, in the presence of a basic reaction auxiliary with suitable protective groups into macrocyclic lactones of the formula (IV) 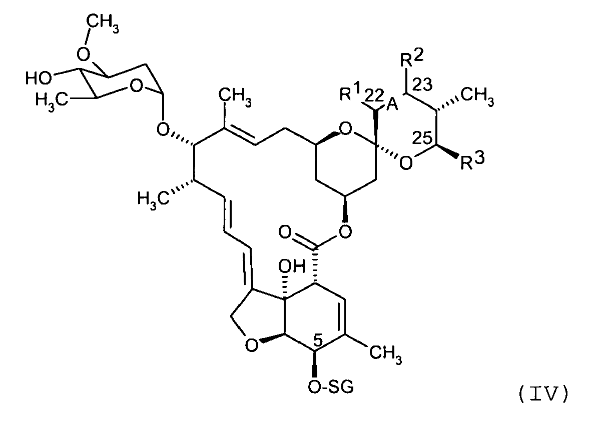 in which SG represents a suitable protective group radical, and these are then in a third reaction step, if appropriate in the presence of a diluent and if appropriate in the presence of a basic reaction auxiliary, reacted with compounds of the formula (V)
R⁴-C(=O)-LG (V)
in which
R⁴ has one of the meanings mentioned in Claim 1, and
LG represents a nucleofugic leaving group which, if appropriate, may be generated in situ,
to give compounds of the formula (VI) 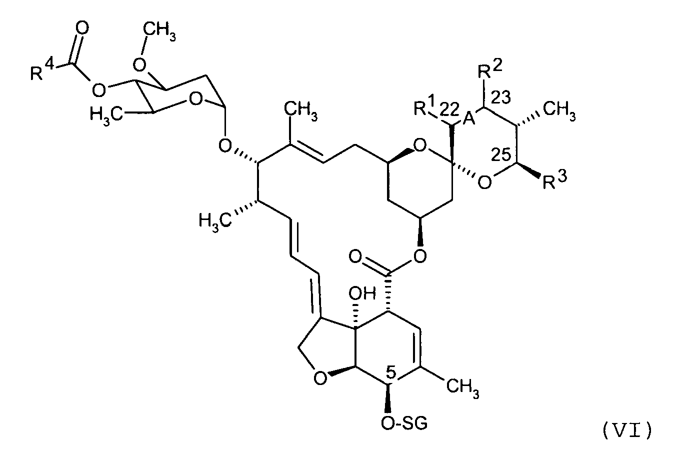 and these are,
in a fourth reaction step, reacted under the reaction conditions of a protective group deblocking, if appropriate in the presence of a diluent and if appropriate in the presence of a suitable acidic or basic reaction auxiliary.

3. Composition, comprising at least one avermectin derivative of the formula (I) according to Claim 1 for controlling animal pests.

4. Use of avermectin derivatives of the formula (I) according to Claim 1 for controlling animal pests.

5. Method for controlling animal pests, **characterized in that** avermectin derivatives of the formula (I) according to Claim 1 are applied to animal pests and/or their habitat.

6. Use of avermectin derivatives of the formula (I) according to Claim 1 for preparing compositions for controlling animal parasites.

7. Use according to Claim 6 where the animal parasites are parasitic arthropods.

8. Use of avermectin derivatives of the formula (I) according to Claim 1 for treating seed.

9. Use of avermectin derivatives of the formula (I) according to Claim 1 for treating transgenic plants.

10. Use of avermectin derivatives of the formula (I) according to Claim 1 for treating seed of transgenic plants.

11. Avermectin derivatives of the formula (VI) according to Claim 1 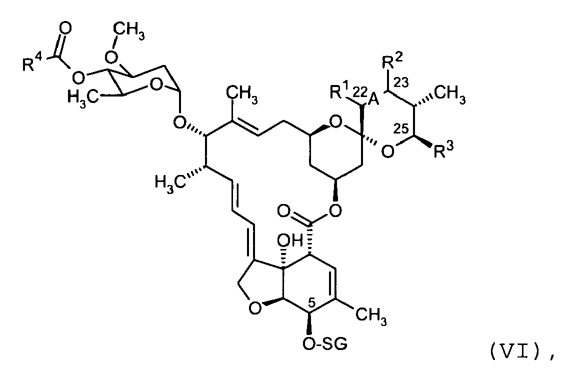 **characterized in that** a protective group radical SG is present.

## Revendications

1. Dérivés de l'avermectine de la formule (1) 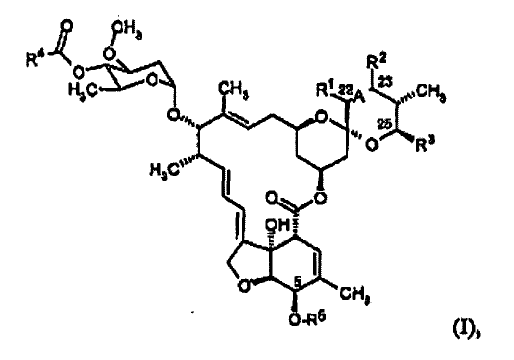 dans laquelle
le groupe -C₂₂R¹-A-C₂₃R²- représente -H₂C-CH(OH)- ou -H₂C-CH₂-,
R³ représente un sec-butyle, un isopropyle ou un cyclohexyle,
R⁵ représente un hydrogène, un méthyle ou alkylcarbonyle en C₁ à C₄, et
R⁴ représente un alkyle en C₂ à C₆, un alcényle en C₂ à C₆, un (alcoxyle en C₁ à C₆)-(alkyle en C₁ à C₄), un (alcoxyle en C₁ à C₄)-(alcoxyle en C₁ à C₄)-(alkyle en C₁ à C₄), un cycloalkyle, un cycloalcényle, un (cycloalkyle)-alkyle en C₁ à C₄, un aryle, substitués le cas échéant, sachant que le 4-chlorophényle est excepté, un aryle-(alkyle en C₁ à C₄), un hétéroaryle, un hétéroaryle-(alkyle en C₁ à C₄), un hétérocyclyle ou un hétérocyclyle-(alkyle en C₁ à C₄), ou un radical, choisi parmi les radicaux (G⁷) à (G¹⁴) 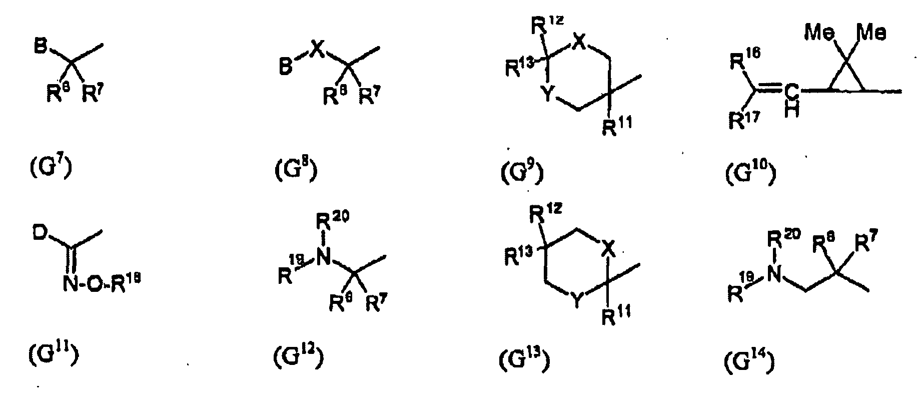
dans lesquels
B représente un aryle, un cycloalkyle, un hétérocyclyle, un hétéroaryle ou NR¹⁹R²⁰, substitués le cas échéant avec R⁸, R⁹ et R¹⁰
D représente un aryle, un cycloalkyle, un hétérocyclyle, un hétéroaryle ou NR¹⁹R²⁰, substitués le cas échéant avec R⁸, R⁹ et R¹⁰,
R⁶ représente un hydrogène, un halogène, en particulier un fluor, un cyano, ou un alkyle, un alcényle, un alcynyle, un cycloalkyle ou un hétérocyclyle substitués le cas échéant,
R⁷ représente un hydrogène, un halogène, en particulier un fluor, un cyano, ou un alkyle, un alcényle, un alcynyle substitués le cas échéant ou
R⁶ et R⁷ représentent conjointement avec l'atome auquel ils sont liés un cycle à 3, 4, 5, 6 ou 7 membres, qui est substitué le cas échéant et/ou interrompu le cas échéant par un oxygène, un soufre, un azote, un sulfinyle ou un sulfonyle, ou
R⁶ et R⁷ représentent conjointement avec l'atome auquel ils sont liés une liaison exo- méthylène substituée le cas échéant,
R⁸ représente un hydrogène, un alkyle en C₁ à C₄, un cycloalkyle en C₂ à C₆, un halogénoalkyle en C₁ à C₄, un alkoxyle en C₁ à C₄, un (alkoxyle en C₁ à C₄)-(alkoxyle en C₁ à C₄), un halogénoalkoxyle en C₁ à C₄, un alkylthio en C₁ à C₄, un halogénoalkylthio en C₁ à C₄, un alkylsulfinyle en C₁ à C₄, un halogénoalkylsulfinyl en C₁ à C₄, un alkylsulfonyle en C₁ à C₄, un halogénoalkylsulfonyle en C₁ à C₄, un hétéroaryle tel un pyridyle ou un thiényle, un halogène, un nitryle, un cyano, un amino, alkylamino en C₁ à C₄, un di-(alkyle en C₁ à C₄)- amino, substitués le cas échéant, ou un radical choisi parmi CO-OH, COO(-), COO-(alkyle en C₁ à C₆), NH-CHO, NH-CO-(alcoxyle en C₁ à C₄), N(alkyle en C₁ à C₄)-CO-(alkoxyle en C₁ à C₄), P(O)(OH)₂, P(O)O⁽⁻⁾₂, CO-NH₂, CS-NH₂, C(=NH)-NH₂, C(=N-OH)-NH₂, CO-NH- (alkyle en C₁ à C₄), CO-N-(alkyl en C₁ à C₄)₂, CO-NH-(alcoxyle en C₁ à C₄), CO-NH-CO- (alkyle en C₁ à C₄), CO-NH-CO-(halogénoalkyle en C₁ à C₄), CO-NH-CO-(cycloalkyle en C₃ à C₇), CO-NH-CO-(alkoxyle en C₁ à C₄), CO-NH-CO-(aryle-alkoxyle en C₁ à C₂), SO₂-OH SO₂-O(-), SO₂-NH₂, SO₂-NH-(alkyle en C₁ à C₄), SO₂-N-(alkyle en C₁ à C₄)₂, CO-NH-SO2- NH-(alkyle en C₁ à C₄), CO-NH-SO₂-N[di(alkyle en C₁ à C₄)], CO-O-(alkyle en C₁ à C₆),
R⁹ représente un hydrogène ou un alkyle en C₁ à C₄, un halogénoalkyle en C₁ à C₄, un alcoxyle en C₁ à C₄, un halogénoalkoxyle en C₁ à C₄, un alkylthio en C₁ à C₄, un alkylsulfinyle en C₁ à C₄, un alkylsulfonyle en C₁ à C₄, substitués le cas échéant, un halogène, un nitryle, un cyano, un formyle, un alkylcarbonyle en C₁ à C₄, un amino, un (alkyl en C₁ à C₄)-amino, un di-(alkyle en C₁ à C₄)-amino, un aryle substitué le cas échéant, un hétéroaryle substitué le cas échéant ou un hétérocyclyle substitué le cas échéant,
R¹⁰ représente un hydrogène ou un alkyle en C₁ à C₄, un alcényle en C₂ à C₄, un alcynyle en C₂ à C₄, un halogénoalkyle en C₁ à C₄, un alkylcarbonyle en C₁ à C₄, un (alkoxyle en C₁ à C₄)-carbonyle, substitués le cas échéant,
R¹¹ représente un hydrogène, un cyano ou un alkyle en C₁ à C₆, substitué le cas échéant,
R¹² et R¹³ représentent indépendamment l'un de l'autre un hydrogène, un hydroxyle ou un alkyle en C₁ à C₄, un alcényle en C₂ à C₄, un (alkyl en C₁ à C₆)-carbonyle, un alkylamino en C₁ à C₆, un di-(alkyle en C₁ à C₆)-amino, un (alkyle en C₁ à C₆)-amino-(alkyle en Ci à C₄), cycloalkyle en C₃ à C₆, un aryle, un aryle-(alkyle en C₁ à C₄), un hétéroaryle-(alkyle en C₁ à C₄), un hétérocyclyle, un hétérocyclyle-(alkyle en C₁ à C₄) ou un hétéroaryle, substitués le cas échéant,
ou
R¹² et R¹³ représentent conjointement avec l'atome auquel ils sont liés, un cycle à 3, 4, 5, 6 ou 7 membres, substitué le cas échéant, qui peut être interrompu le cas échéant par un oxygène, un soufre, un azote, un sulfinyle ou un sulfonyle, ou
R¹² et R¹³ représentent conjointement avec l'atome auquel ils sont liés, un groupe 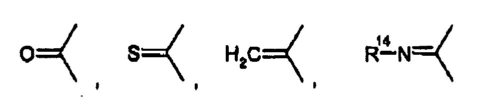
X représente un oxygène, un soufre, un sulfinyle, un sulfonyle ou N-R¹⁴, où R¹⁴ représente un hydrogène ou un alkyle en C₁ à C₄ substitué le cas échéant,
Y représente un oxygène, un soufre, un sulfinyle, un sulfonyle ou N-R¹⁵, où R¹⁵ représente un hydrogène, un alkyle en C₁ à C₄ substitué le cas échéant,
R¹⁶ représente un méthyle, un chlore, un brome ou un trifluorométhyle,
R¹⁷ représente un méthyle, un chlore, ou un brome,
R¹⁸ représente un hydrogène ou un alkyle en C₁ à C₄ substitué le cas échéant, un aryle-(alkyle en C₁ à C₄) ou un hétéroaryle-(alkyle en C₁ à C₄)
R¹⁹ et R²⁰ représentent indépendamment l'un de l'autre un hydrogène ou un alkyle en C₁ à C₄, un halogénoalkyle en C₁ à C₄, un alcoxycarbonyle en C₁ à C₄, un alkylcarbonyle en C₁ à C₄, un (alcoxyle en C₁ à C₆)-(alkyle en C₁ à C₄), un (alkyle en C₁ à C₆)-amino-(alkyle en C₁ à C₄), un di-(alkyle en C₁ à C₆)-amino-(alkyle en C₁ à C₄), un (alcoxyle en C₁ à C₄)- carbamoyl, un alcényle en C₂ à C₆, un alcynyle en C₂ à C₆, un aryle-(alkyl en C₁ à C₄), un hétéroaryle-(alkyl en C₁ à C₄), substitués le cas échéant,
ou
R¹⁹ et R²⁰ représentent conjointement avec l'atome d'azote auquel ils sont liés un groupe amino cyclique ou un cycle à 3, 4, 5, 6 ou 7 membres, qui est interrompu le cas échéant par un oxygène, un soufre, un azote, un sulfinyle ou un sulfonyle et/ou qui est substitué le cas échéant par au moins un, en particulier par 1, 2, 3 ou 4 radicaux comme définis en R⁸, R⁹ et R¹⁰,
et
dans lesquels
aryle signifie un radical aromatique à un, deux ou plusieurs noyaux, et
hétéroaryle signifie un cycle à 5 à 7 membres avec 1 à 3 hétéroatomes identiques ou différents, choisis parmi l'oxygène, le soufre et l'azote, et
hétérocyclyle signifie un cycle monocyclique à 3 à 7 membres saturé ou partiellement insaturé avec 1 à 3 hétéroatomes identiques ou différents, choisis parmi l'oxygène, le soufre et l'azote ou un système de cycles saturé ou partiellement insaturé bicyclique constitué de 8 à 14 atomes, lesquels contiennent soit dans un cycle soit dans les deux cycles, indépendamment l'un de l'autre, 1 à 5 hétéroatomes identiques ou différents, choisis parmi l'oxygène, le soufre et l'azote.

2. Procédé de préparation de dérivés de l'avermectine de la formule (1), **caractérisé en ce que** des composés de la formule (II) 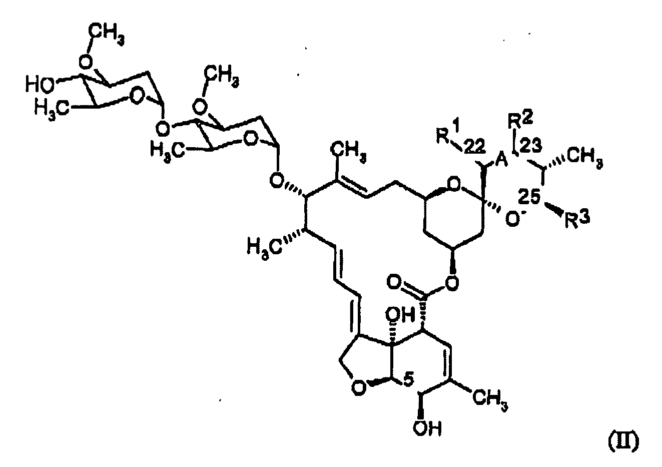 dans laquelle les groupes -C₂₂R¹-A-C₂₃R²- et R³ ont les significations énoncées dans la revendication 1,
sont transformés dans une première étape de réaction, en présence d'un diluant et en présence d'un auxiliaire de réaction acide, pour donner des composés de la formule (III) 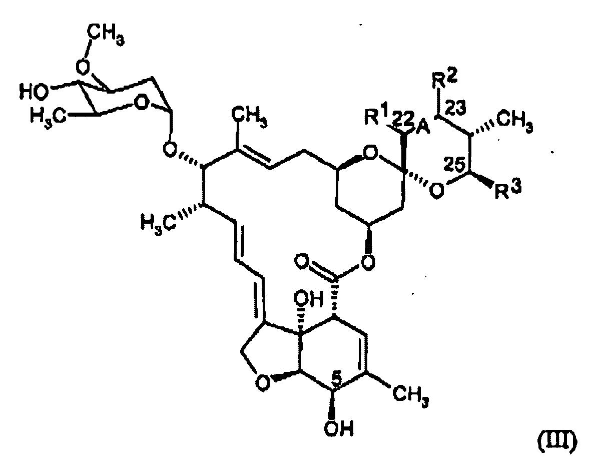 et **en ce que** ceux-ci
sont transformés dans une deuxième étape de réaction en présence d'un diluant et le cas échéant en présence d'un auxiliaire de réaction basique avec des groupes de protection appropriés en des lactones macrocycliques de la formule (IV) 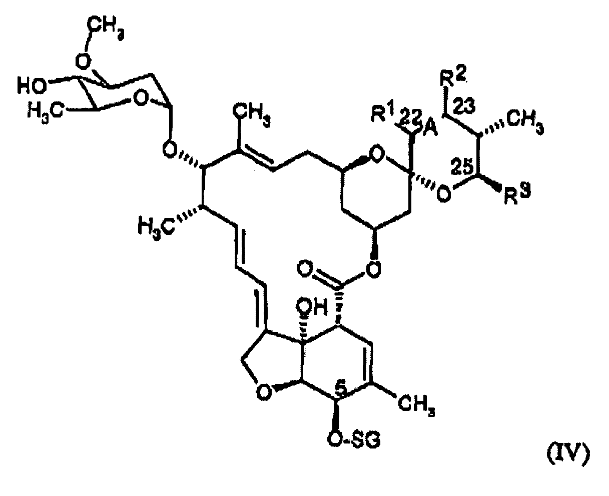 dans laquelle SG signifie un radical de protection approprié, et celles-ci sont ensuite transformées dans une troisième étape de réaction, le cas échéant en présence d'un diluant et le cas échéant en présence d'un auxiliaire de réaction basique, moyennant des composés de la formule (V)
R⁴-C(=O)-LG (V)
dans laquelle
R⁴ a une signification énoncée dans la revendication 1, et
LG signifie un groupe partant (Leaving Group) nucléofuge engendré le cas échéant *in-situ*,
pour donner des composés de la formule (VI) 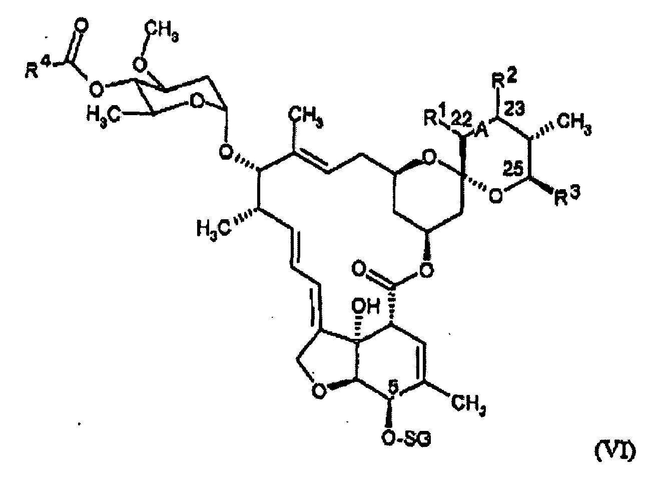 et **en ce que** ceux-ci
sont transformés dans une quatrième étape de réaction sous les conditions de réaction d'un déblocage des groupes de protection, le cas échéant en présence d'un diluant et le cas échéant en présence d'un auxiliaire de réaction approprié acide ou basique.

3. Composition comprenant au moins un dérivé de l'avermectine de la formule (I) selon la revendication 1 pour la lutte contre les organismes nuisibles animaux.

4. Utilisation des dérivés de l'avermectine de la formule (1) selon la revendication 1 pour la lutte contre les organismes nuisibles animaux.

5. Procédé de lutte contre les organismes nuisibles animaux, **caractérisé en ce que** des dérivés de l'avermectine de la formule (I) selon la revendication 1 sont appliqués sur des organismes nuisibles animaux et/ou sur leur espace vital.

6. Utilisation de dérivés de l'avermectine de la formule (I) selon la revendication 1 pour la préparation de compositions pour la lutte contre les parasites animaux.

7. Utilisation selon la revendication 6, les parasites animaux étant des arthropodes parasites.

8. Utilisation de dérivés de l'avermectine de la formule (I) selon la revendication 1 pour traiter des semences.

9. Utilisation de dérivés de l'avermectine de la formule (I) selon la revendication 1 pour traiter des plantes transgéniques.

10. Utilisation de dérivés de l'avermectine de la formule (I) selon la revendication 1 pour traiter les semences de plantes transgéniques.

11. Dérivés de l'avermectine de la formule (VI) selon la revendication 1, 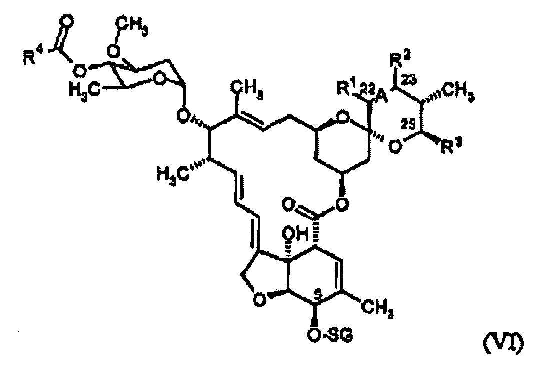 **caractérisés en ce qu'**il existe un radical de protection SG.
